# EUROPEAN PATENT APPLICATION

(11) **EP 4 201 928 A1**
(43) Date of publication of application: **28.06.2023**
(21) Application number: 21306869.5
(22) Date of filing: 21.12.2021
(51) Int. Cl.: C07D 277/46, C07D 401/12, A61K 31/426, A61K 31/427, A61K 31/497, A61K 31/5377, A61P 35/00

(54) **BENZENE SULFONAMIDE THIAZOLE COMPOUNDS AND THEIR USE FOR THE TREATMENT OF CANCERS**

(71) Applicant: Institut National de la Santé et de la Recherche Médicale (INSERM), 75013 Paris (FR); Université Côte d'Azur, 06103 Nice Cedex 2 (FR)
(72) Inventor: ROCCHI, Stéphane, 67000 Strasbourg (FR); CHELBI, Mehdi, 67000 Strasbourg (FR); RONCO, Cyril, 67000 Strasbourg (FR); BENHIDA, Rachid, 67000 Strasbourg (FR)
(74) Representative: Plasseraud IP

(57) **Abstract**

The present inventors have shown that specific benzene sulfonamide thiazole compounds have the ability to induce an early endoplasmic reticulum stress. These compounds also lead to cancerous cells growth inhibition and death.

## Description

### Field of the Invention

The present invention relates to new benzene sulfonamide thiazole compounds and their use for the treatment of cancers.

### Background of the Invention

Multiple disorders such as cancer result from uncontrolled metabolism and proteins synthesis, overwhelming the protein-folding capacity of the endoplasmic reticulum (ER), thus leading to pathological accumulation of misfolded proteins, known as ER stress (Song et al. Trends in Immunology, 2019). As a consequence, Unfolded Protein Response (UPR) complex is hyperactivated to deal with the high flux of proteins processed through the ER and to maintain ER homeostasis. Alterations in ER homeostasis cause accumulation of misfolded/ unfolded proteins in the ER, which pathways to orchestrate adaptive cellular responses activates signalling. One of the key proteins involved in UPR is HSPA5/GRP78/BiP.

GRP78 regulates the balance between cancer cell viability and apoptosis by sustaining ER protein folding capacity and by maintaining ER stress sensors and ER-associated pro-apoptotic machineries in their inactive state.

When misfolded proteins accumulate in the ER, GRP78 binds to them, thereby releasing the UPR sensors and leading to the activation of the UPR pathways. Conversely, when GRP78 is depleted or inactivated, the UPR can be spontaneously triggered, with diverse physiological consequences (Lee, A. Glucose-regulated proteins in cancer: molecular mechanisms and therapeutic potential. Nat Rev Cancer 14, 263-276 (2014). https://doi.org/10.1038/nrc3701).

Hence, in addition to fulfilling the function of a protein chaperone, HSPA5 is also considered to be a master regulator of the UPR. Therefore, HSPA5 positively correlates with poor prognosis especially in cancer. Indeed, HSPA5 positively correlates with an increase of tumor progression, tumor size and poor outcome for patients with melanoma (Michaël Cerezoa and Stéphane Rocchi, New anti-cancer molecules targeting HSPA5/BIP to induce endoplasmic reticulum stress, autophagy and apoptosis, Autophagy. 2017; 13(1): 216-217).

The identification of new candidate molecules acting on ER stress by targeting HSPA5 is thus a promising therapeutic strategy for cancers treatment, widely endorsed in the scientific community.

WO2014072486 describes a first series of benzene sulfonamide thiazole compounds invented by the instant inventors, which are active in the treatment of cancer, especially on melanoma models in mechanism mediated by HSPA5 selective binding. Among these compounds, the lead compound HA15 displays antimelanoma effects by targeting the ER stress axis to induce specific cancer cell death by concomitant induction of autophagy and apoptosis. These results highlight the key role of this specific pathway in melanoma malignancy and cancer, strengthening the idea that ER stress inducers could be useful in the future treatment of a various spectrum of cancers (Michaël Cerezoa and Stéphane Rocchi, New anti-cancer molecules targeting HSPA5/BIP to induce endoplasmic reticulum stress, autophagy and apoptosis, Autophagy. 2017; 13(1): 216-217).

The inventors optimized the series described in WO2014/072486 and generated novel hydrophobic derivatives also responsible for the induction of ER stress and showing a substantially higher potency in models of melanoma (WO2017/017004). The phenyl group of these compounds is substituted with (C₆-C₁₂) alkyl group. The inventors showed the benefit of introducing apolar substituent on this ring. A clear correlation between the size of the apolar group and the activity is highlighted. Indeed, the very active compound are substituted in *para* position with an octyne, hexyl and octyl chain.

The inventors have now improved these compounds in terms of pharmacological properties (efficacy and drug like properties). These novel derivatives highly decrease ER stress markers in a mechanism mediated by GRP78 (BiP) across several in vitro and in vivo models of cancers, supporting their ability for treating cancers, especially the ones over-expressing HSPA5.

### Summary of the Invention

The invention is defined by the claims.

In a **first aspect,** the present invention pertains to a benzene sulfonamide thiazole compound of formula (I): 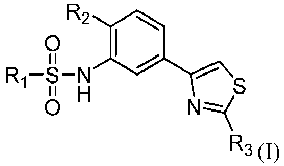 wherein
R₁ is a 5-(dimethylamino)naphthalene group or a 4-pentylphenyl group
R₂ is selected from H, halogen, OR₆
R₃ is selected from C₁-C₈ alkyl, NR₄R₅
R₄ is selected from H, alkyl optionally substituted with a nitrogen-containing heterocycle
R₅ is selected from H, alkyl optionally substituted with a nitrogen-containing heterocycle, COR₇
R₄ and R₅ can alternatively form together a nitrogen-containing heterocycle optionally substituted
R₆ is selected from C₁-C₈ alkyl, C₁-C₈ alkyl-O-C₁-C₈ alkyl,
R₇ is selected from
   - C₁-C₈ alkyl optionally substituted with
      ∘ a nitrogen-containing heterocycle optionally substituted
      ∘ O-alkyl, NHCO-alkyl, NHCOO-alkyl, COO-alkyl, NH-alkyl, NH-alkyl-OH, NHP(O)(O-alkyl)₂, NH-alkyl-SO₃H, NH-alkyl-N(alkyl)₂
      ∘ Alkenyl.

The present inventors have shown that specific benzene sulfonamide thiazole compounds have the ability to induce an early endoplasmic reticulum stress. These compounds also lead to cancerous cells growth inhibition and death.

Hence, in a **second aspect,** the present invention relates to a benzene sulfonamide thiazole compound of formula (I) for use in the treatment of cancer.

### Detailed Description

The inventors demonstrate the efficacy of new benzene sulfonamide thiazole compounds for decreasing the well-known endoplasmic reticulum (ER) stress marker CHOP, on human melanoma cell lines (A375), human cell line derived from gastric cancer (SH-10-TC, KatoIII) and esophageal squamous cell carcinoma (Kyse70), all of which have an over-expression of HSPA5.

CHOP is a DNA damage-inducible transcript 3, also known as C/EBP homologous protein, and is a pro-apoptotic transcription factor that is encoded by the DDIT3 gene CHOP is considered a key player of ER stress and is an initiating factor of ER stress-related cell death. They also show that these compounds lead to cancerous cells growth inhibition and death.

Accordingly, in a first aspect, the present invention pertains to a benzene sulfonamide thiazole of formula (I): 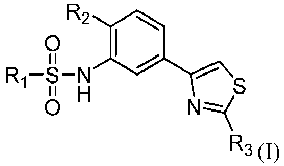 wherein
R₁ is a 5-(dimethylamino)naphthalene group or a 4-pentylphenyl group
R₂ is selected from H, halogen, OR₆
R₃ is selected from C₁-C₈ alkyl, NR₄R₅
R₄ is selected from H, alkyl optionally substituted with a nitrogen-containing heterocycle
R₅ is selected from H, alkyl optionally substituted with a nitrogen-containing heterocycle, COR₇
R₄ and R₅ can alternatively form together a nitrogen-containing heterocycle optionally substituted
R₆ is selected from C₁-C₈ alkyl, C₁-C₈ alkyl-O-C₁-C₈ alkyl
R₇ is selected from
   - C₁-C₈ alkyl optionally substituted with
      ∘ a nitrogen-containing heterocycle optionally substituted
      ∘ O-alkyl, NHCO-alkyl, NHCOO-alkyl, COO-alkyl, NH-alkyl, NH-alkyl-OH, NHP(O)(O-alkyl)₂, NH-alkyl-SO₃H, NH-alkyl-N(alkyl)₂
      ∘ Alkenyl.

### Compounds of formula (I)

In the above general formula (I), unless specified otherwise:
- Alkyl denotes a straight-chain or branched group containing 1, 2, 3, 4, 5,6, 7 or 8 carbon atoms. Examples of suitable alkyl radicals are methyl, ethyl, n-propyl, isopropyl, n-butyl, isobutyl, sec-butyl, tert-butyl, n-pentyl, isopentyl, n-hexyl, etc. A C₁-C₈ alkyl corresponds to an alkyl group containing 1 to 8 carbon atoms.
- Alkenyl denotes a straight-chain or branched group containing 2, 3, 4, 5 or 6 carbon atoms, and one or more double bonds. Examples of suitable alkenyl radicals are, ethenyl, n-propenyl, isopropenyl, n-butenyl, isobutenyl, sec-butenyl, tert-butenyl, n-pentenyl, isopentenyl, n-hexenyl, etc.,
- Heterocycle comprises 3 to 8 atoms and at least one heteroatom different from a carbon atom. Examples of suitable heterocycle comprise piperidine, piperazine, methylpiperazine, morpholine, four-membered rings β-lactam, five-membered rings 1,2,3-triazoles, imidazoles, benzoimidazole, pyrazoles, oxadiazoles, oxazoles, isoxazoles and thiazoles, six-membered rings quinolines, quinazolines, pyrimidines, pyrimidinones

Such heterocycle can be substituted with for examples C₁-C₈ alkyl or C₁-C₈ alkyl-OH, CONH₂.
- Halogen atom is selected from the group consisting of fluorine, chlorine, bromine and iodine.

In the above general formula (I), a preferred group of compounds comprise compounds of formula (II) wherein R₁ is a 5-(dimethylamino)naphthalene group: 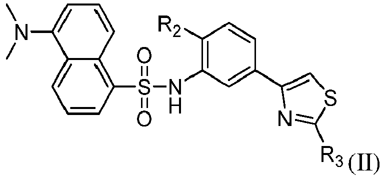 wherein R₂ and R₃ are as defined above.

Another preferred group of compounds comprise compounds of formula (III) wherein R₁ is a 4-pentylphenyl group: 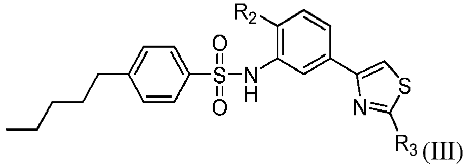 wherein R₂ and R₃ are as defined above.

Others preferred groups of compounds comprise compounds of formula (IV)

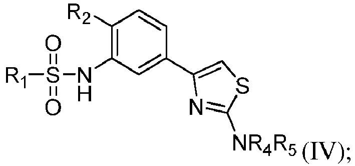

### Compounds of formula (V)

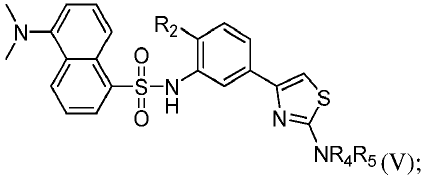

### Compounds of formula (VI)

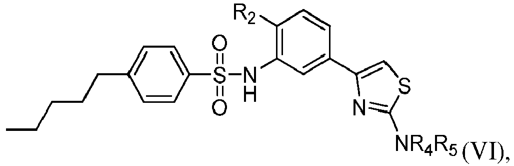

wherein R₁, R₂ R₄ and R₅ are as defined above.

Preferred compounds according to the invention are the following:

| **ID** | **Structure** | **Name** |
|---|---|---|
| PB600 | 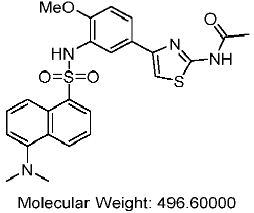 | *N-(4-(3-((5-(dimethylamino)naphthalene)-1-sulfonamido)-4-methoxyphenyl)thiazol-2-yl)acetamide* |
| PB601 | 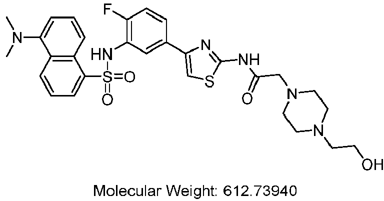 | *N-(4-(3-((5-(dimethylamino)naphthalene)-1-sulfonamido)-4-fluorophenyl)thiazol-2-yl)-2-(4-(2-hydroxyethyl)piperazin-1-yl)acetamide* |
| PB602 | 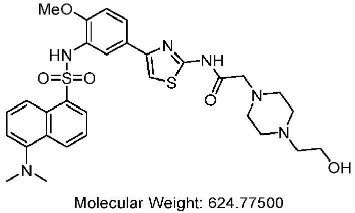 | *N-(4-(3-((5-(dimethylamino)naphthalene)-1-sulfonamido)-4-methoxyphenyl)thiazol-2-yl)-2-(4-(2-hydroxyethyl)piperazin-1-yl)acetamide* |
| PB603 | 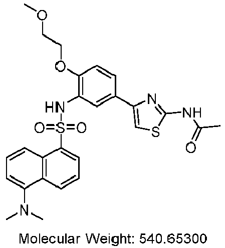 | *N-(4-(3-((5-(dimethylamino)naphthalene)-1-sulfonamido)-4-(2-methoxyethoxy)phenyl)thiazol-2-yl)acetamide* |
| PB604 | 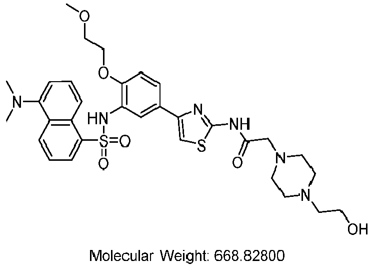 | *N-(4-(3-((5-(dimethylamino)naphthalene)-1-sulfonamido)-4-(2-methoxyethoxy)phenyl)thiazol-2-yl)-2-(4-(2-hydroxyethyl)piperazin-1-yl)acetamide* |
| PB605 | 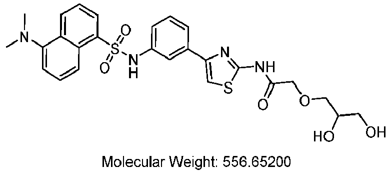 | *2-(2,3-dihydroxypropoxy)-N-(4-(3-((5-(dimethylamino)naphthalene)-1-sulfonamido)phenyl)thiazol-2-yl)acetamide* |
| PB624 | 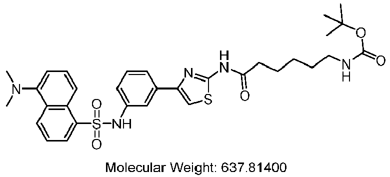 | *Tert-butyl(6-(((4-(3-(((5-(dimethylamino)naphthalene)-1-sulfonamido)phenyl)thiazol-2-yl)amino)-6-oxohexyl)carbamate.* |
| PB625 | 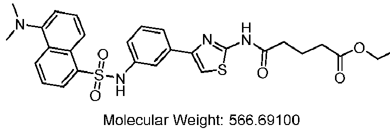 | *Ethyl 5-((4-(3-((5-(dimethylamino)naphthalene)-1-sulfonamido)phenyl)thiazol-2-yl)amino)-5-oxopentanoate* |
| PB628 | 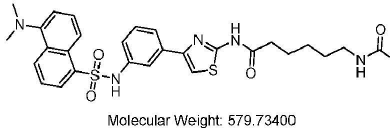 | *6-acetamido-N-(4-(3*-*((5-(dimethylamino)naphthalene)-1-sulfonamido)phenyl)thiazol-2-yl)hexanamide* |
| PB631 | 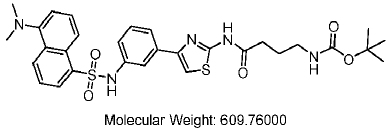 | *Tert-butyl (4-((4-(3-((5-(dimethylamino)naphthalene)-1-sulfonamido)phenyl)thiazol-2-yl)amino)-4-oxobutyl)carbamate.* |
| PB633 | 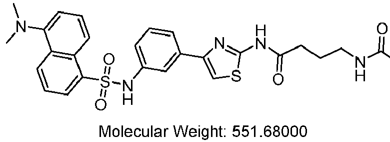 | *4-((4-(3-((5-(dimethylamino(naptalene)-1-sulfonamido)phenyl)thiazol-2-yl)butanamide* |
| PB635 | 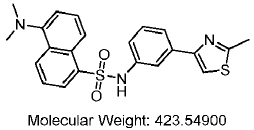 | *5-(dimethylamino)-N-(3-(2-methylthiazol-4-yl)phenyl)naphthalene-1-sulfonamide.* |
| PB638 | 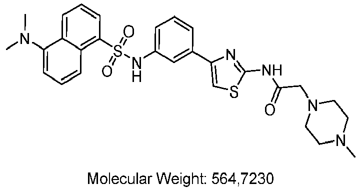 | *N-(4-(3-((5-(dimethylamino)naphthalene)-1-sulfonamido)phenyl)thiazol-2*-*yl)-2-(4-methylpiperazin-1-yl)acetamide* |
| PB639 | 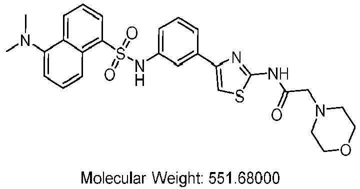 | *N-(4-(3-((5-(dimethylamino)naphthalene)-1-sulfonamido)phenyl)thiazol-2*-*yl)-2-morpholinoacetamide.* |
| PB640 | 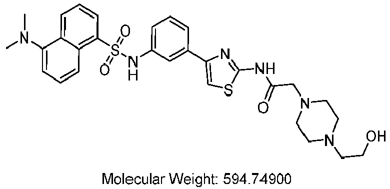 | *N-(4-(3-((5-(dimethylamino)naphthalene)-1-sulfonamido)phenyl)thiazol-2-yl)-2-(4-(2-hydroxyethyl)piperazin-1-yl)acetamide* |
| PB641 | 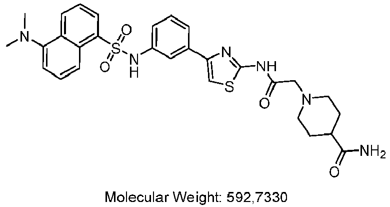 | *1-(2-((4-(3-((5-(dimethylamino)naphthalene)-1-sulfonamido)phenyl)thiazol-2-yl)amino)-2-oxoethyl)piperidine-4-carboxamide* |
| PB643 | 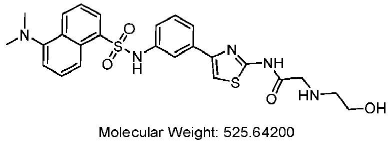 | *N-(4-(3-((5-(dimethylamino)naphthalene)-1-sulfonamido)phenyl)thiazol-2-yl)-2-((2-hydroxyethyl)amino)acetamide.* |
| PB644 | 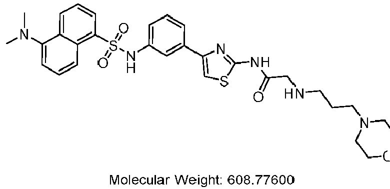 | *N-(4-(3-((5-(dimethylamino)naphthalene)-1-sulfonamido)phenyl)thiazol-2-yl)-2-((3-morpholinopropyl)amino)acetamide.* |
| PB649 | 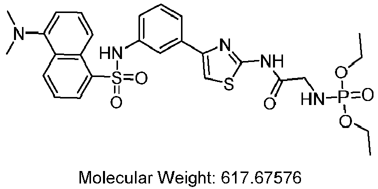 | *Diethyl-(2-((4-(3-(((5-(dimethylamino)naphthalene)-1-sulfonamido)phenyl)thiazol-2-yl)amino)-2-oxoethyl)phosphoramidate* |
| PB651 | 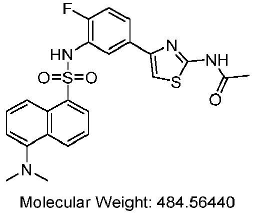 | *N-(4-(3-((5-(dimethylamino)naphthalene)-1-sulfonamido)-4-fluorophenyl)thiazol-2-yl)acetamide* |
| PB654 | 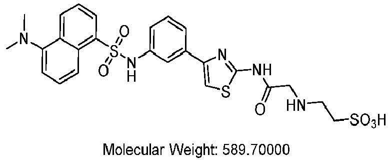 | *2-((2-((4-(3-((*(5-*(dimethylamino)naphthalene)-1-sulfonamido)phenyl)thiazol-2-yl)amino)-2-oxoethyl)amino)ethane-1-sulfonic.* |
| PB688 | 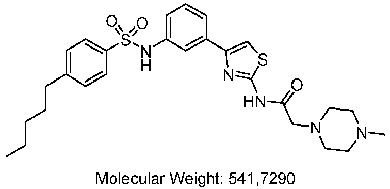 | *2-(4-methylpiperazin-1-yl)-N-(4-(3-((4-pentylphenyl)sulfonamido)phenyl)thiazol-2 yl)acetamide.* |
| PB689 | 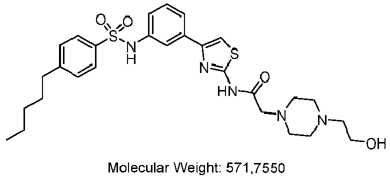 | *2-(4-(2-hydroxyethyl)piperazin-1-yl)-N-(4-(3-((4-pentylphenyl)sulfonamido)phenyl)thiazol-2-yl)acetamide.* |
| PB690 | 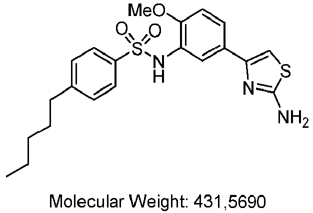 | *N-(5-(2-aminothiazol-4-yl)-2-methoxyphenyl)-4-pentylbenzenesulfonamide* |
| PB691 | 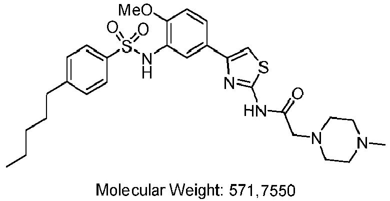 | *N-(4-(4-methoxy-3-((4-pentylphenyl)sulfonamido)phenyl)thiazol-2-yl)-2-(4-methylpiperazin-1-yl)acetamide* |
| PB692 | 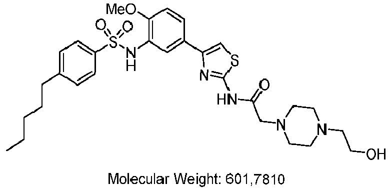 | *2-(4-(2-hydroxyethyl)piperazin-1-yl)-N-(4-(4-methoxy-3-((4-pentylphenyl)sulfonamido) phenyl)thiazol-2*-*yl)acetamide* |
| PB693 | 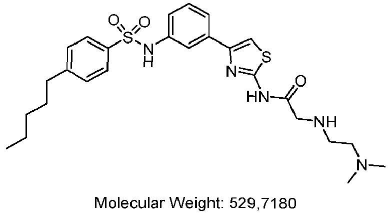 | *2-((2-(dimethylamino)ethyl)amino)-N-(4-(3-((4-pentylphenyl)sulfonamido)phenyl)thiazol-2-yl)acetamide.* |
| PB694 | 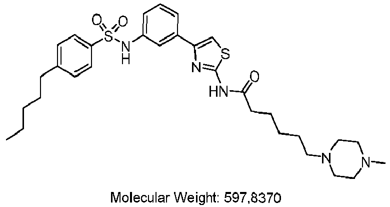 | *6-(4-methylpiperazin-1-yl)-N-(4-(3-((4-pentylphenyl)sulfonamido)phenyl)thiazol-2-yl)hexanamide.* |
| PB695 | 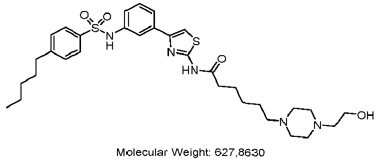 | *6-(4-(2-hydroxyethyl)piperazin-1-yl)-N-(4-(3-((4-pentylphenyl)sulfonamido)phenyl)thiazol-2-yl)hexanamide.* |
| PB696 | 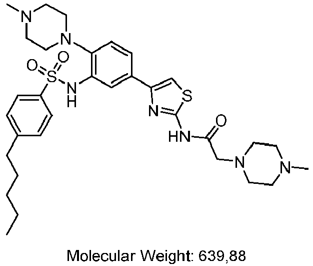 | *2-(4-methylpiperazin-1-yl)-N-(4-(4-(4-methylpiperazin-1-yl)-3-((4-pentylphenyl)sulfonamido)phenyl)thiazol-2-yl)acetamide* |
| PB697 | 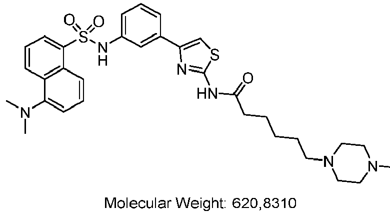 | *N-(4-(3-((5-(dimethylamino)naphthalene)-1-sulfonamido)phenyl)thiazol-2*-*yl)-6-(4-methylpiperazin-1-yl)hexanamide.* |
| PB698 | 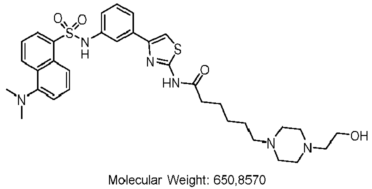 | *N-(4-(3-((5-(dimethylamino)naphthalene)-1-sulfonamido)phenyl)thiazol-2-yl)-6-(4-(2-hydroxyethyl)piperazin-1-yl)hexanamide* |
| PB700 | 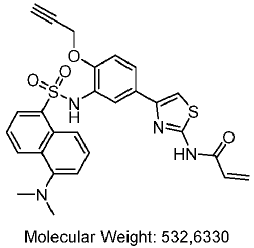 | *N-(4-(3-((5-(dimethylamino)naphthalene)-1-sulfonamido)-4-(prop-2-yn-1-yloxy)phenyl)thiazol-2-yl)acrylamide* |
| PB703 | 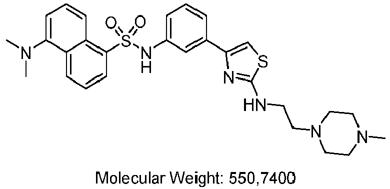 | *5-(dimethylamino)-N-(3-(2-((2-(4-methylpiperazin-1-yl)ethyl)amino)thiazol-4-yl)phenyl)naphthalene-1-sulfonamide* |
| PB704 | 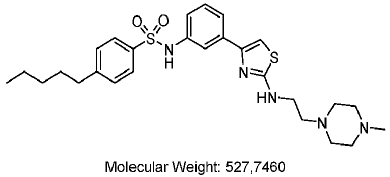 | *N-(3-(2-((2-(4-methylpiperazin-1-yl)ethyl)amino)thiazol-4-yl)phenyl)-4-pentylbenzenesulfonamide.* |
| PB705 | 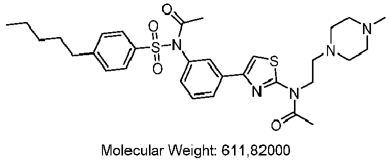 | *N-(2-(4-methylpiperazin-1-yl)ethyl)-N-(4-(3-(N-((4-pentylphenyl)sulfonyl)acetamido)phenyl)thi azol-2-yl)acetamide.* |
| PB706 | 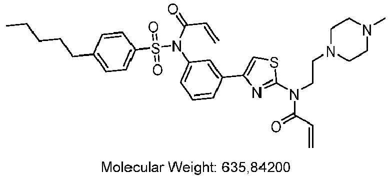 | *N-(2-(4-methylpiperazin-1-yl)ethyl)-N-(4-(3-(N-((4-pentylphenyl)sulfonyl)acrylamido)phenyl)th iazol-2-yl)acrylamide.* |
| PB707 | 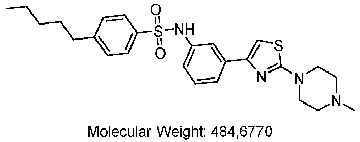 | *N-(3-(2-(4-methylpiperazin-1-yl)thiazol-4-yl)phenyl)-4-pentylbenzenesulfonamide.* |
| PB708 | 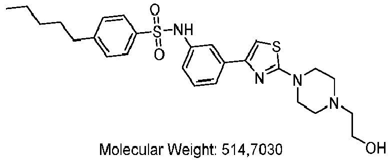 | *N-(3-(2-(4-(2-hydroxyethyl)piperazin-1-yl)thiazol-4-yl)phenyl)-4-pentylbenzenesulfonamide* |

Hence, in an embodiment, the benzene sulfonamide thiazole compound of formula (I) is chosen among:
- *N-(4-(3-((5-(dimethylamino)naphthalene)-1-sulfonamido)-4-methoxyphenyl)thiazol-2-yl)acetamide*
- *N-(4-(3-((5-(dimethylamino)naphthalene)-1-sulfonamido)-4-fluorophenyl)thiazol-2-yl)-2-(4-(2-hydroxyethyl)piperazin-1-yl)acetamide*
- *N-(4-(3-((5-(dimethylamino)naphthalene)-l-sulfonamido)-4-methoxyphenyl)thiazol-2-yl)-2-(4-(2-hydroxyethyl)piperazin-1-yl)acetamide*
- *N-(4-(3-((5-(dimethylamino)naphthalene)-1-sulfonamido)-4-(2-methoxyethoxy)phenyl)thiazol-2-yl)acetamide*
- *N-(4-(3-((5-(dimethylamino)naphthalene)-l-sulfonamido)-4-(2-methoxyethoxy)phenyl)thiazol-2-yl)-2-(4-(2-hydroxyethyl)piperazin-1-yl)acetamide*
- *2-(2,3-dihydroxypropoxy)-N-(4-(3-((5-(dimethylamino)naphthalene)-1-sulfonamido)phenyl)thiazol-2-yl)acetamide*
- *Tert-butyl(6-((4-(3-((5-(dimethylamino)naphthalene)-1-sulfonamido)phenyl)thiazol-2-yl)amino)-6-oxohexyl)carbamate*
- *Ethyl 5-((4-(3-((5-(dimethylamino)naphthalene)-1-sulfonamido)phenyl)thiazol-2-yl)amino)-5-oxopentanoate*
- *6-acetamido-N-(4-(3-((5-(dimethylamino)naphthalene)-1-sulfonamido)phenyl)thiazol-2-yl)hexanamide*
- *Tert-butyl (4-((4-(3-((5-(dimethylamino)naphthalene)-1-sulfonamido)phenyl)thiazol-2-yl)amino)-4-oxobutyl)carbamate*
- *4-((4-(3-((5-(dimethylamino(naptalene)-1-sulfonamido)phenyl)thiazol-2-yl)butanamide*
- *5-(dimethylamino)-N-(3-(2-methylthiazol-4-yl)phenyl)naphthalene-1-sulfonamide*
- *N-(4-(3-((5-(dimethylamino)naphthalene)-1-sulfonamido)phenyl)thiazol-2-yl)-2-(4-methylpiperazin-1-yl)acetamide*
- *N-(4-(3-((5-(dimethylamino)naphthalene)-1-sulfonamido)phenyl)thiazol-2-yl)-2-morpholinoacetamide.*
- *N-(4-(3-((5-(dimethylamino)naphthalene)-1-sulfonamido)phenyl)thiazol-2-yl)-2-(4-(2-hydroxyethyl)piperazin-1-yl)acetamide*
- *1-(2-((4-(3-((5-(dimethylamino)naphthalene)-1-sulfonamido)phenyl)thiazol-2-yl)amino)-2-oxoethyl)piperidine-4-carboxamide*
- *N-(4-(3-((5-(dimethylamino)naphthalene)-1-sulfonamido)phenyl)thiazol-2-yl)-2-((2-hydroxyethyl)amino)acetamide*
- *N-(4-(3-((5-(dimethylamino)naphthalene)-1-sulfonamido)phenyl)thiazol-2-yl)-2-((3-morpholinopropyl)amino)acetamide*
- *Diethyl-(2-((4-(3-((5-(dimethylamino)naphthalene)-1-sulfonamido)phenyl)thiazol-2-yl)amino)-2-oxoethyl)phosphoramidate*
- *N-(4-(3-((5-(dimethylamino)naphthalene)-1-sulfonamido)-4-fluorophenyl)thiazol-2-yl)acetamide*
- *2-((2-((4-(3-((5-(dimethylamino)naphthalene)-1-sulfonamido)phenyl)thiazol-2-yl)amino)-2-oxoethyl)amino)ethane-1-sulfonic*
- *2-(4-methylpiperazin-1-yl)-N-(4-(3-((4-pentylphenyl)sulfonamido)phenyl)thiazol-2 yl)acetamide*
- *2-(4-(2-hydroxyethyl)piperazin-1-yl)-N-(4-(3-((4-pentylphenyl)sulfonamido)phenyl)thiazol-2-yl)acetamide*
- *N-(5-(2-aminothiazol-4-yl)-2-methoxyphenyl)-4-pentylbenzenesulfonamide*
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide*
- *2-((2-(dimethylamino)ethyl)amino)-N-(4-(3-((4-pentylphenyl)sulfonamido)phenyl)thiazol-2-yl)acetamide*
- *6-(4-methylpiperazin-1-yl)-N-(4-(3-((4-pentylphenyl)sulfonamido)phenyl)thiazol-2-yl)hexanamide*
- *6-(4-(2-hydroxyethyl)piperazin-1-yl)-N-(4-(3-((4-pentylphenyl)sulfonamido)phenyl)thiazol-2-yl)hexanamide*
- *2-(4-methylpiperazin-1-yl)-N-(4-(4-(4-methylpiperazin-1-yl)-3-((4-pentylphenyl)sulfonamido)phenyl)thiazol-2-yl)acetamide*
- *N-(4-(3-((5-(dimethylamino)naphthalene)-1-sulfonamido)phenyl)thiazol-2-yl)-6-(4-methylpiperazin-1-yl)hexanamide*
- *N-(4-(3-((5-(dimethylamino)naphthalene)-1-sulfonamido)phenyl)thiazol-2-yl)-6-(4-(2-hydroxyethyl)piperazin-1-yl)hexanamide*
- *N-(4-(3-((5-(dimethylamino)naphthalene)-1-sulfonamido)-4-(prop-2-yn-1-yloxy)phenyl)thiazol-2-yl)acrylamide*
- *5-(dimethylamino)-N-(3-(2-((2-(4-methylpiperazin-1-yl)ethyl)amino)thiazol-4-yl)phenyl)naphthalene-1-sulfonamideN-(3-(2-((2-(4-methylpiperazin-1-yl)ethyl)amino)thiazol-4-yl)phenyl)-4-pentylbenzenesulfonamide*
- *N-(2-(4-methylpiperazin-1-yl)ethyl)-N-(4-(3-(N-((4-pentylphenyl)sulfonyl)acetamido)phenyl)thiazol-2-yl)acetamide*
- *N-(2-(4-methylpiperazin-1-yl)ethyl)-N-(4-(3-(N-((4-pentylphenyl)sulfonyl)acrylamido)phenyl)thiazol-2-yl)acrylamide*
- *N-(3-(2-(4-methylpiperazin-1-yl)thiazol-4-yl)phenyl)-4-pentylbenzenesulfonamide,*
- *N-(3-(2-(4-(2-hydroxyethyl)piperazin-1-yl)thiazol-4-yl)phenyl)-4-pentylbenzenesulfonamide*

In an embodiment, the benzene sulfonamide thiazole compound of formula (I) is chosen among
- *N-(4-(3-((5-(dimethylamino)naphthalene)-1-sulfonamido)-4-methoxyphenyl)thiazol-2-yl)acetamide*
- *N-(4-(3-((5-(dimethylamino)naphthalene)-1-sulfonamido)-4-fluorophenyl)thiazol-2-yl)-2-(4-(2-hydroxyethyl)piperazin-1-yl)acetamide*
- *N-(4-(3-((5-(dimethylamino)naphthalene)-1-sulfonamido)-4-methoxyphenyl)thiazol-2-yl)-2-(4-(2-hydroxyethyl)piperazin-1-yl)acetamide*
- *N-(4-(3-((5-(dimethylamino)naphthalene)-1-sulfonamido)-4-(2-methoxyethoxy)phenyl)thiazol-2-yl)acetamide*
- *N-(4-(3-((5-(dimethylamino)naphthalene)-1-sulfonamido)-4-(2-methoxyethoxy)phenyl)thiazol-2-yl)-2-(4-(2-hydroxyethyl)piperazin-1-yl)acetamide*
- *2-(2,3-dihydroxypropoxy)-N-(4-(3-((5-(dimethylamino)naphthalene)-1-sulfonamido)phenyl)thiazol-2-yl)acetamide*
- *Tert-butyl(6-((4-(3-((5-(dimethylamino)naphthalene)-1-sulfonamido)phenyl)thiazol-2-yl)amino)-6-oxohexyl)carbamate*
- *Ethyl 5-((4-(3-((5-(dimethylamino)naphthalene)-1-sulfonamido)phenyl)thiazol-2-yl)amino)-5-oxopentanoate*
- *6-acetamido-N-(4-(3-((5-(dimethylamino)naphthalene)-1-sulfonamido)phenyl)thiazol-2-yl)hexanamide*
- *Tert-butyl (4-((4-(3-((5-(dimethylamino)naphthalene)-1-sulfonamido)phenyl)thiazol-2-yl)amino)-4-oxobutyl)carbamate*
- *4-((4-(3-((5-(dimethylamino(naptalene)-1-sulfonamido)phenyl)thiazol-2-yl)butanamide*
- *5-(dimethylamino)-N-(3-(2-methylthiazol-4-yl)phenyl)naphthalene-1-sulfonamide*
- *N-(4-(3-((5-(dimethylamino)naphthalene)-1-sulfonamido)phenyl)thiazol-2-yl)-2-(4-methylpiperazin-1-yl)acetamide*
- *N-(4-(3-((5-(dimethylamino)naphthalene)-1-sulfonamido)phenyl)thiazol-2-yl)-2-morpholinoacetamide*
- *N-(4-(3-((5-(dimethylamino)naphthalene)-1-sulfonamido)phenyl)thiazol-2-yl)-2-(4-(2-hydroxyethyl)piperazin-1-yl)acetamide*
- *1-(2-((4-(3-((5-(dimethylamino)naphthalene)-1-sulfonamido)phenyl)thiazol-2-yl)amino)-2-oxoethyl)piperidine-4-carboxamide*
- *N-(4-(3-((5-(dimethylamino)naphthalene)-1-sulfonamido)phenyl)thiazol-2-yl)-2-((2-hydroxyethyl)amino)acetamide*
- *N-(4-(3-((5-(dimethylamino)naphthalene)-1-sulfonamido)phenyl)thiazol-2-yl)-2-((3-morpholinopropyl)amino)acetamide*
- *Diethyl-(2-((4-(3-((5-(dimethylamino)naphthalene)-1-sulfonamido)phenyl)thiazol-2-yl)amino)-2-oxoethyl)phosphoramidate*
- *N-(4-(3-((5-(dimethylamino)naphthalene)-1-sulfonamido)-4-fluorophenyl)thiazol-2-yl)acetamide*
- *2-((2-((4-(3-((5-(dimethylamino)naphthalene)-1-sulfonamido)phenyl)thiazol-2-yl)amino)-2-oxoethyl)amino)ethane-1-sulfonic*
- *N-(4-(3-((5-(dimethylamino)naphthalene)-1-sulfonamido)phenyl)thiazol-2-yl)-6-(4-methylpiperazin-1-yl)hexanamide*
- *N-(4-(3-((5-(dimethylamino)naphthalene)-1-sulfonamido)phenyl)thiazol-2-yl)-6-(4-(2-hydroxyethyl)piperazin-1-yl)hexanamide*
- *N-(4-(3-((5-(dimethylamino)naphthalene)-1-sulfonamido)-4-(prop-2-yn-1-yloxy)phenyl)thiazol-2-yl)acrylamide*
- *5-(dimethylamino)-N-(3-(2-((2-(4-methylpiperazin-1-yl)ethyl)amino)thiazol-4-yl)phenyl)naphthalene-1-sulfonamide*

In a yet an embodiment, the benzene sulfonamide thiazole compound of formula (I) is chosen among
- *2-(4-methylpiperazin-1-yl)-N-(4-(3-((4-pentylphenyl)sulfonamido)phenyl)thiazol-2 yl)acetamide*
- *2-(4-(2-hydroxyethyl)piperazin-1-yl)-N-(4-(3-((4-pentylphenyl)sulfonamido)phenyl)thiazol-2-yl)acetamide*
- *N-(5-(2-aminothiazol-4-yl)-2-methoxyphenyl)-4-pentylbenzenesulfonamide*
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide*
- *2-((2-(dimethylamino)ethyl)amino)-N-(4-(3-((4-pentylphenyl)sulfonamido)phenyl)thiazol-2-yl)acetamide*
- *6-(4-methylpiperazin-1-yl)-N-(4-(3-((4-pentylphenyl)sulfonamido)phenyl)thiazol-2-yl)hexanamide*
- *6-(4-(2-hydroxyethyl)piperazin-1-yl)-N-(4-(3-((4-pentylphenyl)sulfonamido)phenyl)thiazol-2-yl)hexanamide*
- *2-(4-methylpiperazin-1-yl)-N-(4-(4-(4-methylpiperazin-1-yl)-3-((4-pentylphenyl)sulfonamido)phenyl)thiazol-2-yl)acetamide*
- *N-(3-(2-((2-(4-methylpiperazin-1-yl)ethyl)amino)thiazol-4-yl)phenyl)-4-pentylbenzenesulfonamide.*
- *N-(2-(4-methylpiperazin-1-yl)ethyl)-N-(4-(3-(N-((4-pentylphenyl)sulfonyl)acetamido)phenyl)thiazol-2-yl)acetamide*
- *N-(2-(4-methylpiperazin-1-yl)ethyl)-N-(4-(3-(N-((4-pentylphenyl)sulfonyl)acrylamido)phenyl)thiazol-2-yl)acrylamide*
- *N-(3-(2-(4-methylpiperazin-1-yl)thiazol-4-yl)phenyl)-4-pentylbenzenesulfonamide*
- *N-(3-(2-(4-(2-hydroxyethyl)piperazin-1-yl)thiazol-4-yl)phenyl)-4-pentylbenzenesulfonamide*

In a preferred embodiment, the benzene sulfonamide thiazole compound of formula (I) is chosen among
- *2-(4-methylpiperazin-1-yl)-N-(4-(3-((4-pentylphenyl)sulfonamido)phenyl)thiazol-2 yl)acetamide,*
- *2-(4-(2-hydroxyethyl)piperazin-1-yl)-N-(4-(3-((4-pentylphenyl)sulfonamido)phenyl)thiazol-2-yl)acetamide,*
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide,*
- *2-((2-(dimethylamino)ethyl)amino)-N-(4-(3-((4-pentylphenyl)sulfonamido)phenyl)thiazol-2-yl)acetamide,*
- *6-(4-methylpiperazin-1-yl)-N-(4-(3-((4-pentylphenyl)sulfonamido)phenyl)thiazol-2-yl)hexanamide,*
- *6-(4-(2-hydroxyethyl)piperazin-1-yl)-N-(4-(3-((4-pentylphenyl)sulfonamido)phenyl)thiazol-2-yl)hexanamide,*
- *2-(4-methylpiperazin-1-yl)-N-(4-(4-(4-methylpiperazin-1-yl)-3-((4-pentylphenyl)sulfonamido)phenyl)thiazol-2-yl)acetamide,*
- *N-(3-(2-((2-(4-methylpiperazin-1-yl)ethyl)amino)thiazol-4-yl)phenyl)-4-pentylbenzenesulfonamide*
- *N-(2-(4-methylpiperazin-1-yl)ethyl)-N-(4-(3-(N-((4-pentylphenyl)sulfonyl)acetamido)phenyl)thiazol-2-yl)acetamide*

The benzene sulfonamide thiazole compounds according to the invention can be in the form of pharmaceutically acceptable salts. Pharmaceutically acceptable salts include the acid addition and base salts thereof.

Suitable acid addition salts are formed from acids, which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate and xinafoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see "Handbook of Pharmaceutical Salts: Properties, Selection, and Use" by Stahl and Wermuth (Wiley- VCH, Weinheim, Germany, 2002).

The benzene sulfonamide thiazole compounds used in the context of the present invention may exist in both unsolvated and solvated forms. The term 'solvate' describes a molecular complex comprising the benzene sulfonamide thiazole compound and a stoichiometric amount of one or more pharmaceutically acceptable solvent molecules, for example, ethanol. The term 'hydrate' is employed when said solvent is water. Also included are complexes such as clathrates, drug-host inclusion complexes wherein, in contrast to the aforementioned solvates, the drug and host inclusion are present in stoichiometric or non-stoichiometric amounts. Also included are complexes of the drug containing two or more organic and/or inorganic components, which may be in stoichiometric or non-stoichiometric amounts. The resulting complexes may be ionised, partially ionized, or non-ionized. For a review of such complexes, see J Pharm Sci, 64 (8), 1269-1288 by Haleblian (August 1975).

The benzene sulfonamide thiazole compounds of formula (I) thus include references to salts, solvates and complexes thereof and to solvates and complexes of salts thereof. The benzene sulfonamide thiazole compounds of formula (I) include all polymorphs and crystal habits thereof, prodrugs and isomers thereof (including optical, geometric and tautomeric isomers) and isotopically- labelled compounds of formula (I).

### Synthetic routes:

The compounds according to the invention may be prepared using conventional procedures such as by the following illustrative methods hereinafter described.

### Synthetic route for PB600, PB601, PB602, PB603, PB604, PB651, PB690, PB691, PB692, PB696

This route needs the synthesis of intermediate thiazole anilines 3a, 3b, 3c according to the following method. Each compounds 2a, 2b, 2c, 3a, 3b and 3c are hereinafter described in relation to each step.

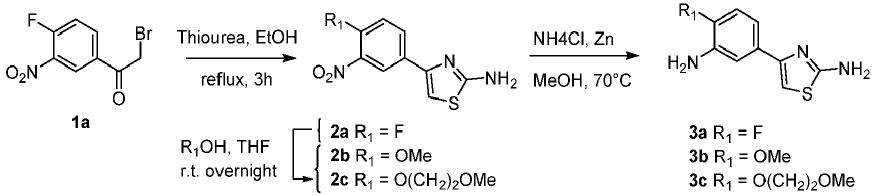

### 2a

*4-(4-fluoro-3-nitrophenyl)thiazol-2-amine* (**2a**). To a solution of α-bromo-acetophenone **1a** (7.20 g, 27.5 mmol, 1 eq) in ethanol was added the corresponding thiourea (thiourea (2.09 g, 27.5 mmol, 1 eq). The reaction mixture was then heated to 80°C for 3 hours and left to cool to room temperature. The precipitate was filtered and washed with ethanol and diethyl ether to afford the corresponding thiazole as yellow solids in 74% yield (6.52 g). ¹H NMR (DMSO-*d₆*, 200 MHz): *δ* 7.45 (s, 1H), 7.72 (dd, *J* = 11.2, 8.8 Hz, 1H), 8.19 (ddd, *J =* 8.8, 4.2, 2.4 Hz, 1H), 8.50 (dd, *J =* 7.1, 2.4 Hz, 1H).

### 2b

*4-(4-methoxy-3-nitrophenyl)thiazol-2-amine* (**2b**). To a suspension of fluoroaryl **2a** (2.40 g, 10.00 mmol) THF (15 mL) was slowly added sodium methoxide (1.08 g, 20.00 mmol). The reaction middle was stirred at r.t. until complete consumption of the starting material (TLC monitoring CHx:EtOAc 60:40). The THF was removed under reduced pressure and crude residue was diluted with 100 mL of water and extracted three times with EtOAc (50 mL). Combined organic layer was washed with brine (100 mL), dried over MgSO₄ and concentrated under reduced pressure. The crude was subjected to silica gel column chromatography (DCM:EtOAc 100:0 to 50:50) to afford **2b** (2.31 g, 91%). ¹H NMR (400 MHz, DMSO-d₆) δ 8.28 (d, J = 2.3 Hz, 1H), 8.05 (dd, J = 8.8, 2.3 Hz, 1H), 7.48 (d, J = 8.9 Hz, 1H), 7.28 (s, 1H), 3.98 (s, 3H). MS-ESI (m/z): [M+H]⁺= 252.5.

### 2c

*4-(4-(2-methoxyethoxy)-3-nitrophenyl)thiazol-2-amine* (**2c**). To a suspension of sodium hydride (60% in oil) (240.0 g, 10.08 mmol) in dry THF (33 mL) at 0°C was added dropwise 2-methoxyethanol (760.0 µL, 9.66 mmol). The resulting mixture was stirred 20 min at 0°C and a suspension of fluoroaryl **2a** (1.00 g, 4.20 mmol) in dry THF (15 mL) was added very slowly. The reaction middle was stirred 15 min at 0°C and let overnight at r.t. until complete consumption of the starting material (TLC monitoring CHx:EtOAc 60:40). The THF was removed under reduced pressure and crude residue was diluted with 100 mL of water and extracted three times with EtOAc (50 mL). Combined organic layer was washed with bruine (100 mL), dried over MgSO₄ and concentrated under reduced pressure. The crude was subjected to silica gel column chromatography (DCM:EtOAc 100:0 to 50:50) afforded 2-ethoxymethanoyl **2c** (1.22 g, 98%). ¹H NMR (Acetone-*d₆*, 200 MHz): *δ* 3.36 (s, 3H), 3.81 - 3.69 (m, 2H), 4.39 - 4.23 (m, 2H), 6.54 (s, 2H), 7.02 (s, 1H), 7.33 (d, *J =* 8.8 Hz, 1H), 8.05 (dd, *J =* 8.8, 2.3 Hz, 1H), 8.28 (d, *J =* 2.2 Hz, 1H).

### 3a

*4-(3-amino-4-fluorophenyl)thiazol-2-amine* (**3a**). To a stirred suspension of nitro-aryl **2a** (6.34 g, 19.80 mmol, 1.0 eq) in MeOH (0.01 M) was successively added ammonium chloride (2.11 g, 39.60 mmol, 2.0 eq) and zinc (9.71 g, 148.50 mmol, 7.5 eq). The reaction mixture was stirred at 70°C until complete consumption of the starting material and the crude was filtered through a pad of celite. The resulting filtrate was extracted three times with EtOAc and purified by silica gel flash chromatography (DCM:MeOH; 100:0 to 90:10) to afforded aniline **3a** as a white powder (4.10 g, 99%). ¹H NMR (DMSO-*d₆*, 200 MHz): *δ* 5.15 (s, 2H), 6.74 (s, 1H), 7.08 - 6.87 (m, 4H), 7.31 - 7.15 (m, 1H). ¹³C NMR (DMSO-*d₆*, 50 MHz): *δ* 100.3, 113.7 (dd, *J =* 14.7, 5.6 Hz), 114.9 (d, *J =* 18.7 Hz), 131.6 (d, *J =* 2.9 Hz), 136.11 (d, *J =* 13.2 Hz), 147.8, 149.8, 152.5, 168.0. ¹⁹F NMR (Acetone-*d₆*, 189 MHz): *δ* -136.5 (q, *J =* 8.8 Hz).

### 3b

*4-(3-amino-4-methoxyphenyl)thiazol-2-amine* (**3b**). To a stirred suspension of nitro-aryl **2b** (2.00 g, 8.00 mmol, 1.0 eq) in MeOH (0.01 M) was successively added ammonium chloride (880.0 mg, 16.00 mmol, 2.0 eq) and zinc (3.92 g, 148.50 mmol, 7.5 eq). The reaction mixture was stirred at 70°C until complete consumption of the starting material and the crude was filtered through a pad of celite. The resulting filtrate was extracted three times with EtOAc and purified by silica gel flash chromatography (DCM:MeOH; 100:0 to 90:10) afforded aniline **3b** as a white powder (1.68 g, 95%). ¹H NMR (DMSO-*d₆,* 400 MHz) δ 3.77 (s, 4H), 6.64 (s, 1H), 6.78 (d, *J =* 8.4 Hz, 1H), 6.97 (s, 2H), 7.01 (dd, *J =* 8.3, 1.9 Hz, 1H), 7.11 (d, *J* = 2.0 Hz, 1H).

### 3c

*4-(3-amino-4-(2-methoxyethoxy)phenyl)thiazol-2-amine* (**3c**). To a stirred suspension of nitro-aryl **2c** (1.22 g, 4.13 mmol, 1.0 eq) in MeOH (0.01 M) was successively added ammonium chloride (440.0 mg, 8.26 mmol, 2.0 eq) and zinc (2.03 g, 30.98 mmol, 7.5 eq). The reaction mixture was stirred at 70°C until complete consumption of the starting material and the crude was filtered through a pad of celite. The resulting filtrate was extracted three times with EtOAc and purified by silica gel flash chromatography (DCM:MeOH; 100:0 to 90:10) to afforded aniline **3c** as a white powder (560.0 mg, 33%). ¹H NMR (Acetone-*d₆*, 200 MHz): *δ* 3.37 (s, 3H), 3.80 - 3.61 (m, 2H), 4.19 - 4.03 (m, 2H), 6.43 (s, 2H), 6.63 (s, 1H), 6.80 (d, *J =* 8.4 Hz, 1H, H₈), 7.09 (dd, *J =* 8.3, 2.1 Hz, 1H, H₉), 7.23 (d, *J =* 2.1 Hz, 1H, H₅).

### Synthesis of compounds PB651, PB600 and PB603

These compounds are prepared using the following method hereinafter described.

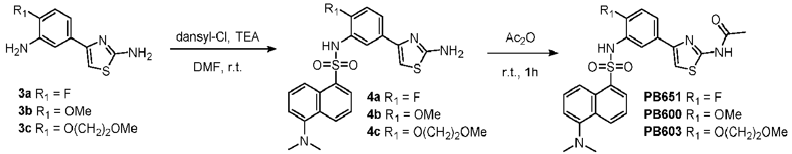

Compounds 4a, 4b, 4c and the compounds obtained are described hereinafter with each step.

### 4a

*N-(5-(2-aminothiazol-4-yl)-2-fluorophenyl)-5-(dimethylamino)naphthalene-1-sulfonamide* (**4a**). To a solution of 3a (2.00 g, 9.62 mmol, 1 eq) under argon in anhydrous DMF (0.2 M) were added anhydrous triethylamine (1.6 eq) and dansyl chloride (2.85 g, 10.58 mmol, 1.1 eq). The reaction mixture was allowed to react at room temperature until complete conversion of the starting material. DMF was removed under reduced pressure and the crude material was purified by silica gel column chromatography (DCM:EtOAc; 100:0 to 60:40) to afford the titled sulfonamide 4a as a yellow fluorescent powder (1.22 g, 29%). ¹H NMR (Acetone-d6, 200 MHz): δ 2.84 (s, 6H), 6.50 (s, 2H), 6.78 (s, 1H), 6.93 (dd, J = 10.2, 8.7 Hz, 1H), 7.26 (dd, J = 7.6, 0.9 Hz, 1H), 7.66 - 7.43 (m, 3H), 7.96 (dd, J = 7.8, 2.2 Hz, 1H), 8.24 (dd, J = 7.3, 1.2 Hz, 1H), 8.63 - 8.45 (m, 2H), 9.22 (s, 1H).

### 4b

*N-(5-(2-aminothiazol-4-yl)-2-methoxyphenyl)-5-(dimethylamino)naphthalene-1-sulfonamide* **(4b).** To a solution of 3b (222.0 mg, 0.50 mmol, 1 eq) under argon in anhydrous DMF (0.2 M) were added anhydrous triethylamine (1.6 eq) and dansyl chloride (148.0 mg, 0.55 mmol, 1.1 eq). The reaction mixture was allowed to react at r.t. until complete conversion of the starting material. DMF was removed under reduced pressure and the crude material was purified by silica gel column chromatography (DCM:EtOAc; 100:0 to 60:40) to afford the titled sulfonamide 4b as a pale yellow fluorescent powder (120.2 mg, 53%). ¹H NMR (Acetone-d6, 200 MHz): δ 2.83 (s, 6H), 3.24 (s, 3H), 6.42 (s, 2H), 6.70 (s, 2H), 7.26 (d, J = 7.5 Hz, 1H), 7.69 - 7.38 (m, 3H), 7.94 (d, J = 2.1 Hz, 1H), 8.13 (dd, J = 7.3, 1.2 Hz, 1H), 8.34 (s, 1H), 8.50 (dd, J = 8.6, 3.8 Hz, 2H).

### 4c

*N-(5-(2-aminothiazol-4-yl)-2-(2-methoxyethoxy)phenyl)-5-(dimethylamino)naphthalene-1-sulfonamide* (**4c**). To a solution of 3c (415.0 mg, 1.57 mmol, 1 eq) under argon in anhydrous DMF (0.2 M) were added anhydrous triethylamine (1.6 eq) and dansyl chloride (465.1 mg, 1.72 mmol, 1.1 eq). The reaction mixture was allowed to react at r.t. until complete conversion of the starting material. DMF was removed under reduced pressure and the crude material was purified by silica gel column chromatography (DCM:EtOAc; 100:0 to 70:30) to afford the titled sulfonamide 4c as a pale yellow fluorescent powder (326.3 mg, 42%). ¹H NMR (Acetone-d6, 200 MHz): δ 2.84 (s, 6H), 3.40 - 3.25 (m, 5H), 3.80 - 3.68 (m, 2H), 6.44 (s, 2H), 6.83 - 6.68 (m, 2H), 7.27 (dd, J = 7.6, 0.9 Hz, 1H), 7.66 - 7.38 (m, 3H), 8.01 (d, J = 2.1 Hz, 1H), 8.20 (dd, J = 7.3, 1.3 Hz, 1H), 8.60 - 8.39 (m, 3H).

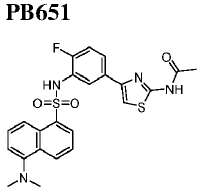

*N-(4-(3-((5-(dimethylamino)naphthalene)-1-sulfonamido)-4-fluorophenyl)thiazol-2-yl)acetamide* (**PB651**). The amine **4a** (25.2 mg, 0.06 mmol) was placed in pure acetic anhydride (600 µL) at 40°C for 1h. The solvent was then removed under reduced pressure. The crude product was precipitated using Et₂O, washed with Et₂O twice (10 mL) to afford pure amide **PB651** a yellow fluorescent powder (24.5 mg, 89%). ¹H NMR (Acetone-*d₆*, 200 MHz): *δ* 2.29 (s, 3H), 2.85 (s, 6H), 6.97 (dd, *J* = 10.2, 8.6 Hz, 1H), 7.36 - 7.12 (m, 2H), 7.63 - 7.47 (m, 3H), 8.07 (dd, *J =* 7.8, 2.2 Hz, 1H), 8.22 (dd, *J =* 7.4, 1.3 Hz, 1H), 8.52 (dd, *J =* 8.6, 5.1 Hz, 2H), 9.26 (s, 1H), 11.20 (s, 1H). ¹³C NMR (Acetone-*d₆*, 125 MHz): *δ* 22.9, 45.7, 108.4, 116.3, 116.6 (d, *J =* 20.4 Hz), 120.2, 122.9, 124.1, 124.38 (d, *J =* 7.4 Hz), 126.16 (d, *J =* 13.2 Hz), 129.1, 130.73 (d, *J =* 9.8 Hz), 130.9, 131.6, 132.47 (d, *J =* 3.2 Hz), 136.3, 154.3, 156.2, 158.9, 152.9, 159.1, 169.2. ¹⁹F NMR (Acetone-*d₆*, 188 MHz): *δ* -128.3. HRMS-ESI (m/z): [M+H]⁺ calcd for C₂₃H₂₂O₃N₄FS₂, 485.1112; found: 485.1116.

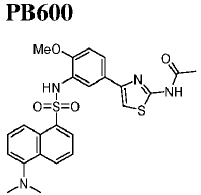

*N-(4-(3-((5-(dimethylamino)naphthalene)-1-sulfonamido)-4-methoxyphenyl)thiazol-2-yl)acetamide* (**PB600**). The amine **4b** (45.4 mg, 0.10 mmol) was placed in pure acetic anhydride (1 mL) at 40°C for 1h. The solvent was then removed under reduced pressure. The crude product was precipitated using Et₂O, washed with Et₂O twice (10 mL) to afford pure amide **PB600** a yellow fluorescent powder (35.7 mg, 72%). ¹H NMR (Acetone-*d₆*, 200 MHz): *δ* 2.29 (s, 3H), 2.83 (s, 6H), 3.27 (s, 3H), 6.72 (d, *J =* 8.6 Hz, 1H), 7.25 (d, *J =* 11.4 Hz, 2H), 7.69 - 7.38 (m, 3H), 8.04 (d, *J =* 2.1 Hz, 1H), 8.12 (dd, *J =* 7.3, 1.1 Hz, 1H), 8.40 (s, 1H), 8.51 (t, *J =* 7.8 Hz, 2H), 11.15 (s, 1H). ¹³C NMR (acetone-*d₆*, 50 MHz): *δ* 22.9, 45.7, 55.9, 106.8, 112.0, 116.1, 120.6, 121.5, 123.9, 124.0, 127.2, 128.7, 128.8, 130.6, 130.7, 130.8, 131.1, 136.4, 149.8, 151.6, 152.8, 158.9, 169.1. HRMS-ESI (m/z): [M+H]⁺ calcd for C₂₄H₂₅O₄N₄S₂, 497.1312, found: 497.1312. HPLC (λ₂₅₄) Purity: 96.1%.

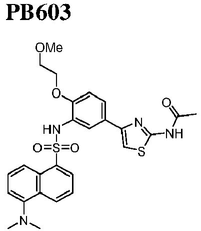

*N-(4-(3-((5-(dimethylamino)naphthalene)-1-sulfonamido)-4-(2-methoxyethoxy)phenyI)thiazol-2-yl)acetamide* (**PB603**). To a solution of acetic anhydride (2 mL) was added amine **4c** (115.0 mg, 0.23 mmol). The mixture was stirred at 40°C for 8h and the mixture was quenched with a 4M aqueous solution of sodium hydroxide (6 mL). The mixture was stirred 30 additional minutes and was diluted with 20 mL of water and 20 mL of EtOAc. The aqueous layer was extracted twice with EtOAc (2x20 mL), the combined organic layer was washed with water (2x20 mL), 40 mL of brine and dried over MgSO₄. The crude residue was subjected to silica gel chromatography DCM:EtOAc (100:0 to 60:40) yielded amide **PB603** as a yellow fluorescent powder (96.0 mg, 77%). ¹H NMR (Acetone-*d₆*, 200 MHz): *δ* 2.29 (s, 3H), 2.82 (s, 6H), 3.33 (d, *J =* 6.4 Hz, 5H), 3.84 - 3.68 (m, 2H), 6.81 (d, *J =* 8.6 Hz, 1H), 7.26 (d, *J =* 7.0 Hz, 2H), 7.69 - 7.40 (m, 3H), 8.24 - 8.05 (m, 2H), 8.49 (d, *J =* 8.3 Hz, 2H), 11.19 (s, 1H). ¹³C NMR (Acetone-*d₆*, 50 MHz): *δ* 22.9, 45.7, 59.0, 69.7, 71.1, 107.1, 114.7, 116.2, 120.4, 120.7, 123.6, 124.0, 128.2, 128.9, 129.4, 130.6, 130.7, 130.7, 131.2, 136.4, 149.7, 150.3, 152.8, 158.9, 169.1. HRMS-ESI (m/z): [M+H]⁺ calcd for C₂₆H₂₉O₅N₄S₂, 541.1574; found: 541.1578. HPLC (λ₂₅₄) Purity: 95.5 %; *t*_{R}: 7.04 min (method 1).

### Synthesis of compounds PB601, PB602 and PB604.

These compounds are prepared using the following method hereinafter described.

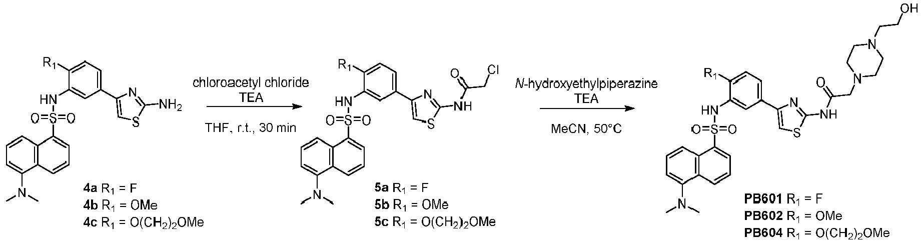

Compounds 4a, 5b, 5c and the compounds obtained are described hereinafter with each step.

### 5a

To a stirring solution of amine **4a** (1.00 g, 2.77 mmol) in dry THF (15 mL) was added DIEA (0.81 mL, 4.54 mmol). Then, the chloroacetyl chloride (0.36 mL, 4.54 mmol) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced. The crude residue was subjected to silica gel column chromatography (DCM:EtOAc 100:0 to 60:40) yielded pure chloroacetyl 5a as a yellow fluorescent powder (0.70 g, 60%). ¹H NMR (Acetone-*d₆*, 200 MHz): *δ* 2.84 (s, 6H), 4.51 (s, 2H), 6.98 (dd, *J =* 10.1, 8.7 Hz, 1H), 7.27 (d, *J =* 7.6 Hz, 1H), 7.41 (s, 1H), 7.66 - 7.45 (m, 3H), 8.09 (dd, *J =* 7.8, 2.1 Hz, 1H), 8.23 (dd, *J =* 7.3, 1.1 Hz, 1H), 8.52 (dd, *J =* 8.6, 3.7 Hz, 2H), 9.30 (s, 1H), 11.54 (s, 1H).

### 5b

To a stirring solution of amine **5a** (100.0 mg, 0.22 mmol) in dry THF (1.5 mL) was added DIEA (115.0 µL, 0.44 mmol). Then, the chloroacetyl chloride (38.0 µL, 0.44 mmol) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced. The crude residue was subjected to silica gel column chromatography (DCM:EtOAc 100:0 to 60:40) yielded chloroacetyl **5a** (42.0 mg, 36%). ¹H NMR (Acetone-*d₆*, 200 MHz): *δ* 2.84 (s, 6H), 3.28 (s, 3H), 4.50 (s, 2H), 6.75 (d, *J =* 8.6 Hz, 1H), 7.38 - 7.22 (m, 2H), 7.67 - 7.41 (m, 3H), 8.05 (d, *J =* 2.2 Hz, 1H), 8.12 (dd, *J =* 7.4, 1.2 Hz, 1H), 8.62 - 8.44 (m, 3H), 11.48 (s, 1H).

### 5c

To a stirring solution of amine **4c** (190.0 mg, 0.38 mmol) in dry THF (2 mL) was added DIEA (132.0 µL, 0.76 mmol). Then, the chloroacetyl chloride (61.0 µL, 0.76 mmol) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced. The crude residue was subjected to silica gel column chromatography (DCM:EtOAc 100:0 to 60:40) yielded chloroacetyl **5c** as a yellow fluorescent powder (175.2 mg, 80%). ¹H NMR (Acetone-*d₆*, 200 MHz): *δ* 2.84 (s, 6H), 3.34 (d, *J* = 8.4 Hz, 5H), 3.87 - 3.68 (m, 2H), 4.50 (s, 2H), 6.84 (d, *J* = 8.6 Hz, 1H), 7.28 (d, *J* = 7.6 Hz, 1H), 7.35 (s, 1H), 7.68 - 7.42 (m, 3H), 8.11 (d, *J* = 2.1 Hz, 1H), 8.18 (d*, J =* 6.3 Hz, 1H), 8.60 - 8.43 (m, 3H) 11.50 (s, 1H).

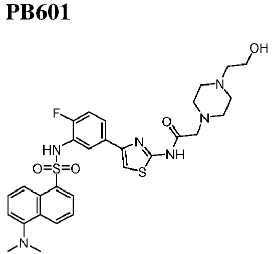

*N-(4-(3-((5-(dimethylamino)naphthalene)-1-sulfonamido)-4-fluorophenyl)thiazol-2-yl)-2-(4-(2-hydroxyethyl)piperazin-1-yl)acetamide* (**PB601**). To a suspension of the chloroalkyl **5a** (224.0 mg, 0.43 mmol, 1 eq) in acetonitrile or THF (0.2 M) was added triethylamine (1 eq) and *N-*hydroxyethylpiperazine (113.0 mg, 0.87 mmol, 3 eq). The reaction mixture was stirred at 50°C until complete consumption of starting material. The volatiles were removed under reduced pressure and crude residue was subjected to silica gel column chromatography (DCM:EtOAc 100:0 to 0:100) to afford **PB601** as yellow fluorescent powder (165.0 mg, 63%). ¹H NMR (Acetone-*d₆*, 200 MHz): *δ* 2.71 - 2.45 (m, 10H), 2.83 (s, 6H), 3.29 (s, 2H,), 3.62 (t, *J =* 5.8 Hz, 2H), 7.00 (dd, *J =* 10.2, 8.7 Hz, 1H), 7.26 (d, *J =* 7.5 Hz, 1H), 7.36 (s, 1H), 7.67 - 7.48 (m, 3H), 8.10 - 7.93 (m, 1H), 8.25 (dd, *J =* 7.3, 1.1 Hz, 1H), 8.61 - 8.45 (m, 2H). ¹³C NMR (Acetone-*d₆*, 125 MHz): *δ* 45.7, 54.2, 54.3, 59.5, 61.1, 61.9, 108.8, 116.3, 116.7 (d, *J =* 20.5 Hz), 120.2, 123.0, 124.1, 124.5 (d, *J =* 7.4 Hz), 126.3 (d, *J =* 13.3 Hz), 129.1, 130.7 (d, *J =* 8.7 Hz), 130.9, 131.6, 132.3 (d, *J =* 3.5 Hz), 136.4, 149.0, 152.9, 154.4, 156.3, 158.5, 169.7. HRMS-ESI (m/z): [M+H]⁺ calcd for C₂₉H₃₄O₄N₆FS₂, 613.2062, found: 613.2065. HPLC (λ₂₅₄) Purity: >99 %; *t*_{R}: 5.20 min (method 1).

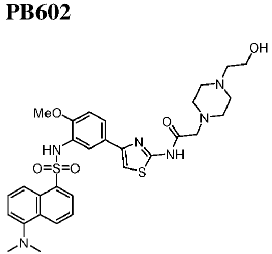

*N-(4-(3-((5-(dimethylamino)naphthalene)-1-sulfonamido)-4-methoxyphenyl)thiazol-2-yl)-2-(4-(2-hydroxyethyl)piperazin-1-yl)acetamide* (**PB602**). To a suspension of the chloroalkyl **5b** (33.0 mg, 0.062 mmol, 1 eq) in acetonitrile or THF (0.2 M) was added triethylamine (1 eq) and *N-*hydroxyethylpiperazine (20.0 µL, 0.19 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) afforded pure **PB602** as yellow fluorescent powder (23.1 mg, 56%). ¹H NMR (Acetone-*d₆*, 200 MHz): *δ* 2.74 - 2.45 (m, 10H), 2.84 (s, 6H), 3.29 (d, *J* = 2.0 Hz, 5H), 3.61 (t, *J* = 5.9 Hz, 2H), 6.76 (d, *J* = 8.5 Hz, 1H), 7.27 (d, *J* = 7.3 Hz, 2H), 7.70 - 7.43 (m, 3H), 8.01 (d, *J* = 2.1 Hz, 1H), 8.15 (dd, *J* = 7.3, 1.3 Hz, 1H), 8.52 (dd, *J =* 8.6, 2.7 Hz, 2H). ¹³C NMR (Acetone-*d₆*, 125 MHz): *δ* 45.7, 54.1, 54.2, 55.9, 59.5, 61.1, 61.9, 107.2, 112.0, 116.1, 120.6, 121.6, 123.9, 124.2, 127.3, 128.5, 128.8, 130.7, 130.9, 131.2, 136.5, 150.0, 151.8, 152.8, 158.2, 169.6. HRMS-ESI (*m*/*z*): [M+H]⁺ calcd for C₃₀H₃₇O₅N₆S₂, 625.2261; found: 625.2268. HPLC (λ₂₅₄) Purity: > 99 %; *t*_{R}: 5.27 min (method 1).

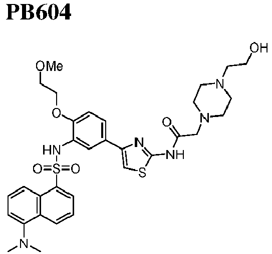

*N-(4-(3-((5-(dimethylamino)naphthalene)-1-sulfonamido)-4-(2-methoxyethoxy)phenyl)thiazol-2-yl)-2-(4-(2-hydroxyethyl)piperazin-1-yl)acetamide* (**PB604**). To a suspension of the chloroalkyl **5c** (151.0 mg, 0.26 mmol, 1 eq) in acetonitrile or THF (0.2 M) was added triethylamine (1 eq) and *N-*hydroxyethylpiperazine (102.0 mg, 0.79 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) to afford pure **PB604** as yellow fluorescent powder (142.3 mg, 81%). ¹H NMR (Acetone-*d₆*, 200 MHz): *δ* 2.74 - 2.42 (m, 10H), 2.83 (s, 6H), 3.45 - 3.21 (m, 7H), 3.61 (t, *J =* 5.8 Hz, 2H), 3.86 - 3.67 (m, 2H), 6.84 (d, *J =* 8.6 Hz, 1H), 7.39 - 7.19 (m, 2H), 7.70 - 7.42 (m, 3H), 8.07 (d, *J =* 2.0 Hz, 1H), 8.20 (d, *J* = 7.2 Hz, 1H), 8.49 (d, *J =* 8.5 Hz, 2H). ¹³C NMR (Acetone-*d₆*, 50 MHz): *δ* 45.6, 54.0, 54.2, 58.9, 59.4, 61.0, 61.8, 69.6, 71.0, 107.4, 114.5, 116.1, 120.4, 120.7, 123.7, 124.0, 128.1, 128.9, 129.1, 130.6, 130.6, 130.7, 131.1, 136.5, 149.9, 150.4, 152.8, 158.2, 169.5. HRMS-ESI (m/z): [M+H]⁺ calcd for C₃₂H₄₁O₃N₆S₂, 669.2524; found: 669.2524. HPLC (λ₂₅₄) Purity: 98.1 %; *t*_{R}: 5.28 min (method 1).

### Synthesis of compounds PB690, PB691 and PB692

These compounds are prepared using the following method hereinafter described.

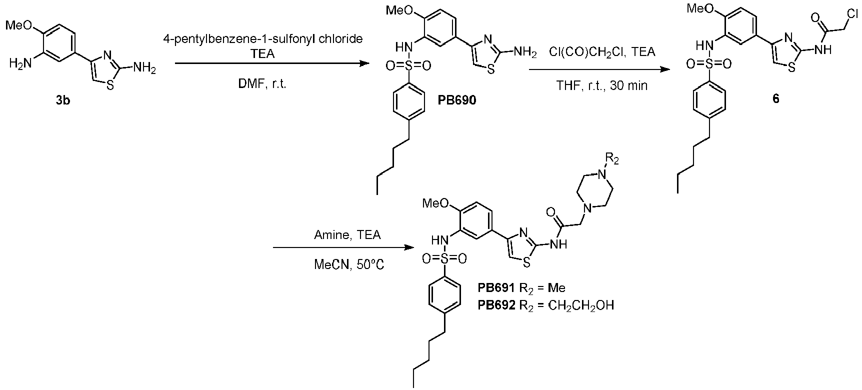

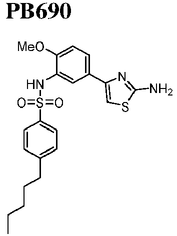

*N-(5-(2-aminothiazol-4-yl)-2-methoxyphenyl)-4-pentylbenzenesulfonamide* **(PB690). 3b** (1.00 g, 4.50 mmol, 1 eq) under argon in anhydrous DMF (0.2 M) were added anhydrous triethylamine (1.6 eq) and 4-pentylbenzene-1-sulfonyl chloride (1.23 g, 5.00 mmol, 1.1 eq). The reaction mixture was allowed to react at r.t. until complete conversion of the starting material. DMF was removed under reduced pressure and the crude material was purified by silica gel column chromatography (DCM:MeOH, 100:0 to 95:5) afforded the titled sulfonamide **PB690** as a white powder (410.2 mg, 21%). ¹H NMR (CD₂Cl₂-*d*₂, 400 MHz): δ 0.86 (t, *J =* 7.0 Hz, 3H), 1.34 - 1.23 (m, 4H), 1.60 - 1.53 (m, 2H), 2.60 (t, *J =* 7.9 Hz, 2H), 3.60 (s, 3H), 5.73 (br s, 2H), 6.63 (s, 1H), 6.74 (d, *J =* 8.6 Hz, 1H), 7.04 (s, 1H), 7.22 (d, *J =* 8.4 Hz, 2H), 7.47 (dd, *J =* 8.5, 2.2 Hz, 1H), 7.64 (d, *J =* 8.4 Hz, 2H), 7.89 (d, *J =* 2.1 Hz, 1H). ¹³C NMR (CD₂Cl₂-*d*₂, 101 MHz): δ 14.1, 22.8, 31.1, 31.7, 36.1, 56.1, 101.8, 111.1, 119.8, 123.5, 126.3, 127.6, 128.6, 129.2, 136.6, 149.3, 149.9, 150.5, 168.3, HRMS-ESI(*m*/*z*): [M+H]⁺calc. for C₂₁H₂₆N₃O₃S₂, 432.1410; Found: 432.1401. HPLC (λ₂₅₄): Purity > 99 %; *t*_{R}: 5.92 min (method mauro 3).

### Compound 6 of the reaction

To a stirring solution of **PB690** (350.0 mg, 0.81 mmol, 1 eq) in dry THF (10 mL) was added DIEA (420.0 µL, 2.43 mmol, 3 eq). Then, the chloroacetyl chloride (126.0 µL, 1.62 mmol, 2 eq) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced pressure to yield the respective chloroacetyl intermediate as a brown powder (400 mg, 98%). The crude chloroacetyl 6 was directly used without further purification for the synthesis of **PB691** and **PB692.**

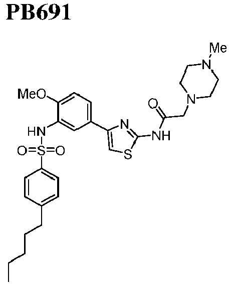

*N-(4-(4-methoxy-3-((4-pentylphenyl)sulfonamido)phenyl)thiazol-2-yl)-2-(4-methylpiperazin-1-yl)acetamide* (**PB691**). To a suspension of the chloroalkyl **6** (100.0 mg, 0.20 mmol, 1 eq) in acetonitrile (0.2 M) was added triethylamine (1 eq) and *N*-methylpiperazine (65.0 µL, 0.60 mmol, 3 eq). The reaction mixture was stirred at 50°C until complete consumption of starting material. The volatiles were removed under reduced pressure and crude residue was subjected to silica gel column chromatography (DCM:MeOH 100:0 to 95:5) to afford pure targeted **PB691** as a white powder (42.0 mg, 37%). ¹H NMR (CD₂Cl₂-*d*₂, 400 MHz): δ 0.86 (t, *J* = 6.7 Hz, 3H), 1.28 - 1.22 (m, 4H), 1.67 - 1.49 (m, 2H), 2.29 (s, 3H), 2.64 - 2.53 (m, 10H), 3.24 (s, 2H), 3.62 (s, 3H), 6.78 (d, *J =* 8.6 Hz, 1H), 7.07 (s, 2H), 7.22 (d, *J =* 8.5 Hz, 2H), 7.57 (dd, *J =* 8.5, 2.2 Hz, 1H), 7.68 - 7.61 (m, 2H), 7.99 (d, *J =* 2.1 Hz, 1H), 10.32 (s, 1H). ¹³CNMR (CD₂Cl₂-*d*₂,101MHz): δ 14.1, 22.8, 31.1, 31.7, 36.1, 46.1,54.4, 55.3, 56.1, 61.4, 107.0, 111.2, 119.8, 123.7, 126.6, 127.6, 128.2, 129.2, 136.7, 149.3, 149.5, 150.2, 157.5, 169.1. HRMS-ESI (m/z): [M+H]⁺ calc. for C₂₈H₃₈N₅O₄S₂, 572.2359; Found: 572.2348. HPLC (λ₂₅₄): Purity > 99 %; *t*_{R}: 5.25 min (method mauro 3).

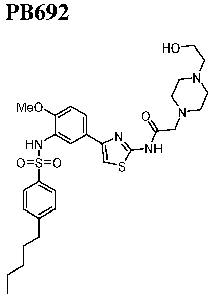

*2-(4-(2-hydroxyethyl)piperazin-1-yl)-N-(4-(4-methoxy-3-((4-pentylphenyl)sulfonamido) phenyl)thiazol-2-yl)acetamide* (**PB692**).

To a suspension of the chloroalkyl **6** (100.0 mg, 0.20 mmol, 1 eq) in acetonitrile (0.2 M) was added triethylamine (1 eq) and *N*-hydroxyethylpiperazine (78.0 mg, 0.60 mmol, 3 eq). The reaction mixture was stirred at 50°C until complete consumption of starting material. Purification by silica gel column chromatography (DCM:MeOH 100:0 to 95:5) afforded pure targeted **PB692** as a white powder (48.0 mg, 40%). ¹H NMR (CD₂Cl₂-*d*₂, 400 MHz): δ 0.86 (t, *J =* 7.0 Hz, 3H), 1.31 - 1.23 (m, 4H), 1.60 - 1.52 (m, 2H), 2.13 (br s, 1H) 2.67 - 2.58 (m, 12H), 3.26 (s, 2H), 3.62 - 3.58 (m, 5H), 6.78 (d, *J* = 8.6 Hz, 1H), 7.07 (s, 2H), 7.21 (d, *J =* 8.4 Hz, 2H), 7.56 (dd, *J =* 8.5, 2.2 Hz, 1H), 7.64 (d, *J =* 8.4 Hz, 2H), 8.00 (d, *J =* 2.1 Hz, 1H), 10.34 (s, 1H). ¹³C NMR (CD₂Cl₂-*d*₂, 101 MHz): δ 14.1. 22.8, 31.1, 31.7, 36.1, 53.1, 54.4, 56.1, 58.0, 59.8, 61.5, 107.1, 111.2, 119.9, 123.6, 126.6, 127.6, 128.2, 129.1, 136.7, 149.29, 149.5, 150.3, 157.6, 169.0. HRMS-ESI (m/z): [M+H]⁺ calc. for C₂₉H₄₀N₅O₅S₂, 602.2465; Found: 602.2460. HPLC (λ₂₅₄): Purity > 99 %; *t*_{R}: 5.21 min (method mauro 3).

### Synthesis of PB696

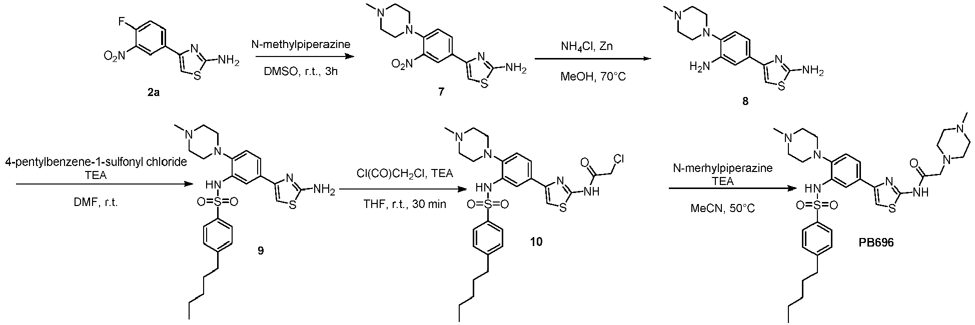

### Compound 7 of the reaction

*4-(4-(4-methylpiperazin-1-yl)-3-nitrophenyl)thiazol-2-amine* (7). To a stirred suspension of nitro-aryl **2a** (2.38 g, 10.0 mmol, 1.0 eq) in DMSO (0.1 M) was successively added N-methylpiperazine (3.00 g, 30 mmol; 3.0 eq). The reaction mixture was stirred at room temperature until complete consumption of the starting material. The crude was added to ice water, the reddish solids were filtered in vacuo to afford nitro product 7 in 72% (2.29 g). ¹H NMR (400 MHz, DMSO-d₆) δ 2.23 (s, 3H), 2.44 (d, J = 3.9 Hz, 4H), 3.08 - 2.87 (m, 4H), 7.11 (d, J = 21.8 Hz, 3H), 7.30 (d, J = 8.7 Hz, 1H), 7.96 (dd, J = 8.7, 1.9 Hz, 1H), 8.19 (d, J = 1.9 Hz, 1H).

### Compound 8 of the reaction

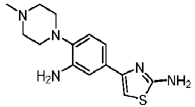

*4-(3-amino-4-(4-methylpiperazin-1-yl)phenyl)thiazol-2-amine* (**8**). To a stirred suspension of nitro-aryl **8** (3.0 g, 9.4 mmol, 1.0 eq) in DMSO (0.1 M) was successively added was successively added ammonium chloride (1.00 g, 18.8 mmol, 2.0 eq) and zinc (4.58 g, 141.00 mmol, 7.5 eq). The reaction mixture was stirred at 70°C until complete consumption of the starting material and the crude was filtered through a pad of celite. The resulting filtrate was concentrated to afford aniline **8** as a white powder (992.0 mg, 36%). ¹H NMR (400 MHz, DMSO-d₆) δ 2.72 (s, 3H), 3.07 (s, 4H), 3.28 (s, 4H), 4.89 (s, 2H), 6.69 (s, 1H), 6.85 (d, J = 8.2 Hz, 1H), 7.02 - 6.94 (m, 3H), 7.14 (d, J = 1.8 Hz, 1H).

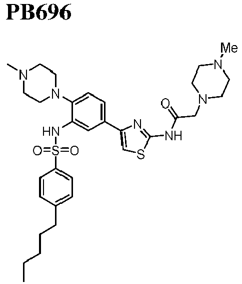

*2-(4-methylpiperazin-1-yl)-N-(4-(4-(4-methylpiperazin-1-yl)-3-((4-pentylphenyl)sulfonamido)phenyl)thiazol-2-yl)acetamide* (**PB696**).To a solution of **8** (900.0 mg, 3.10 mmol, 1 eq) under argon in anhydrous DMF (0.2 M) were added anhydrous triethylamine (1.6 eq) and 4-pentylbenzene-1-sulfonyl chloride (842.0 mg, 3.42 mmol, 1.1 eq). The reaction mixture was allowed to react at r.t. until complete conversion of the starting material. DMF was removed under reduced pressure and the crude material was purified by silica gel column chromatography (DCM:MeOH, 100:0 to 95:5) afforded the sulfonamide **9** as a white powder (402.2 mg, 26%). To a stirring solution of **9** (402.0 mg, 0.80 mmol, 1 eq) in dry THF (10 mL) was added DIEA (3 eq). Then, the chloroacetyl chloride (126.0 µL, 1.60 mmol, 2 eq) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced pressure to yield the respective chloroacetyl intermediate **10** as a brown powder (320 mg, 55%). The crude chloroacetyl **10** (150.0 mg, 0.26 mmol, 1 eq) in acetonitrile (0.2 M) was added triethylamine (1 eq) and *N*-methylpiperazine (100.0 µL, 0.78 mmol, 3 eq). The reaction mixture was stirred at 50°C until complete consumption of starting material. Purification by silica gel column chromatography (DCM:MeOH 100:0 to 95:5) afforded pure targeted **PB696** as a light yellow powder (16.0 mg, 10%). ¹H NMR (CD₃OD_*d*₄, 400 MHz): δ 0.86 (t, *J =* 7.1 Hz, 3H), 1.34 - 1.22 (m, 4H), 1.59 - 1.51 (m, 2H), 2.61 (t, *J* = 7.8 Hz, 2H), 2.72 (br s, 4H), 2.84 (d, *J* = 1.5 Hz, 6H), 2.92 (br s, 4H), 3.21 (br s, 4H), 3.27 (br s, 4H), 3.49 (s, 2H), 7.23 (d, *J =* 8.4 Hz, 1H), 7.34 - 7.30 (m, 3H), 7.64 (dd, *J =* 8.3, 2.0 Hz, 1H), 7.72 (d, *J =* 8.4 Hz, 2H), 8.11 (d, *J =* 2.0 Hz, 1H). ¹³C NMR (CD₃OD_*d*₄, 101 MHz): δ 14.4. 23.4, 31.9, 32.4, 36.5, 44.1, 44.4, 51.2, 51.5, 54.9, 55.3, 60.6, 109.5, 120.9, 123.5, 124.3, 128.2, 130.3, 133.9, 134.4, 138.5, 143.6, 150.1, 150.5, 159.1, 170.1. HRMS-ESI (m/z): [M+H]⁺ calc. for C₃₂H₄₆N₇O₃S₂, 640.3098; Found: 640.3088. HPLC (λ₂₅₄): Purity 97.7 %; *t*_{R}: 4.25 min (method mauro 7).

### Synthetic route for compounds PB631, PB624, PB633, PB628, PB649, PB635, PB625, PB638, PB639, PB640, PB643, PB644, PB654, PB605, PB697, PB698, PB688, PB689, PB693, PB694, PB695, PB704, PB707, PB708, PB705, PB706

Compounds 2a-c were first synthetized according to the following method:

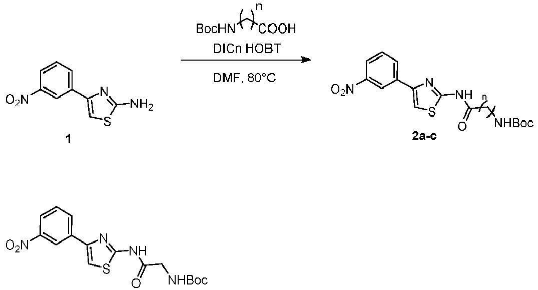

*Tert-butyl (2-((4-(3-nitrophenyl)thiazol-2-yl)amino)-2-oxoethyl)carbamate.* To a solution of *N*-Bocglycine (356.3 mg, 2.03 mmol) in dry DMF (10 mL) at r.t. and under argon were added successively HOBT (310.9 mg, 2.03 mmol), DIC (317.9 µL, 2.03 mmol) and triethylamine (282.9 µL, 2.03 mmol) for 15 minutes. **1** (300.0 mg, 1.34 mmol) was then added to the mixture, stirred for 3h hours at r.t. and at 80°C for additional 24 hours. DMF was removed under reduced pressure and crude residue was dissolved in technical EtOAc (15 mL) and organic layer was washed three times with distilled water (20 mL). Aqueous layer was extracted three times with EtOAc (15 mL) and combined organic layer was washed with brine (30 mL), dried over MgSO₄ and concentrated under reduced pressure. The crude was subjected to silica gel chromatography eluted with DCM/EtOAc (100:0 to 0:100) to afford amide **2a** as a yellow brown powder (200.2 mg, 39%). ¹H NMR (CD₃OD-*d*₄, 200 MHz): δ 1.40 (s, 9H), 3.88 (d, *J =* 6.1 Hz, 2H), 7.19 (t, *J* = 6.1 Hz, 1H), 7.72 (t*, J =* 8.0 Hz, 1H), 7.94 (s, 1H), 8.16 (ddd, *J* = 8.2, 2.4, 1.0 Hz, 1H), 8.33 (dt, *J =* 7.9, 1.2 Hz, 1H), 8.84 - 8.66 (m, 1H), 12.41 (s, 1H).

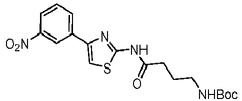

*Tert-butyl (4-((4-(3-nitrophenyl)thiazol-2-yl)amino)-4-oxobutyl)carbamate.* To a solution of 4-*N*-Boc-aminobutanoic acid (1.15 g, 5.67 mmol) in dry DMF (15 mL) at r.t. and under argon were added successively HOBT (0.87 g, 5.67 mmol), DIC (0.89 mL, 5.67 mmol) and triethylamine (0.79 mL, 5.67 mmol) for 15 minutes. 1 (1.00 g, 4.52 mmol) was then added to the mixture, stirred for 3h hours at r.t. and at 80°C for additional 24 hours. DMF was removed under reduced pressure and crude residue was dissolved in technical EtOAc (30 mL) and organic layer was washed three times with distilled water (50 mL). Aqueous layer was extracted three times with EtOAc (30 mL) and combined organic layer was washed with brine (60 mL), dried over MgSO₄ and concentrated under reduced pressure. The crude was subjected to silica gel chromatography eluted with DCM/EtOAc (100:0 to 70:30) to afford amide 2b as yellow powder (0.32 mg, 17%). ¹H NMR (DMSO-*d₆*, 200 MHz): *δ* 1.48 - 1.31 (m, 9H), 1.71 (quint., *J* = 6.9 Hz, 2H), 1.71 (p, *J* = 6.9 Hz, 2H), 2.96 (q, *J* = 6.7 Hz, 2H), 6.84 (d, *J* = 6.0 Hz, 1H), 7.72 (t, *J =* 8.1 Hz, 1H), 7.92 (s, 1H), 8.16 (ddd, *J =* 8.2, 2.4, 1.0 Hz, 1H), 8.33 (dt, *J =* 7.9, 1.2 Hz, 1H), 8.72 (t, *J* = 2.0 Hz, 1H), 12.34 (s, 1H).

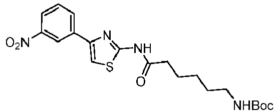

*Tert-butyl (6-((4-(3-nitrophenyl)thiazol-2-yl)amino)-6-oxohexyl)carbamate.* To a solution of 6-*N*-Boc-aminocaproic acid (1.07 g, 4.64 mmol) in dry DMF (20 mL) at r.t. and under argon were added successively HOBT (0.63 g, 4.64 mmol), DIC (0.729 mL, 4.64 mmol) and triethylamine (0.65 mL, 4.64 mmol) for 15 minutes. 1 (0.77 g, 3.45 mmol) was then added to the mixture, stirred for 3 hours at r.t. and heated at 80°C for additional 24 hours. DMF was removed under reduced pressure and crude residue was dissolved in technical EtOAc (50 mL) and organic layer was washed three times with distilled water (50 mL). Aqueous layer was extracted three times with EtOAc (50 mL) and combined organic layer was washed with bruine (100 mL), dried over MgSO₄ and concentrated under reduced pressure. The crude was subjected to silica gel chromatography eluted with DCM/EtOAc (100:0 to 60:40) to afford amide **2c** as yellow-orange powder (0.69 mg, 45%). ¹H NMR (DMSO-*d₆*, 200 MHz): *δ* 1.45 - 1.18 (m, 13H, H1), 1.60 (quint., *J =* 7.3, 6.7 Hz, 2H), 2.57 - 2.30 (m, 2H), 2.90 (q, *J =* 6.4 Hz, 2H), 6.79 (t, *J =* 5.7 Hz, 1H), 7.71 (t*, J =* 8.0 Hz, 1H), 7.90 (s, 1H), 8.15 (ddd, *J =* 8.2, 2.4, 1.0 Hz, 1H), 8.32 (dt*, J =* 7.9, 1.2 Hz, 1H), 8.71 (t, *J =* 2.0 Hz, 1H), 12.32 (s, 1H).

Then compounds **3a** to **3c** were synthetized according to the following method:

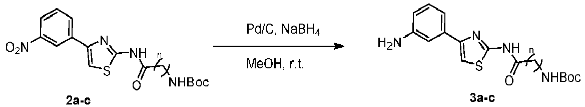

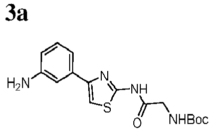

*Tert-butyl (2-((4-(3-aminophenyl)thiazol-2-yl)amino)-2-oxoethyl)carbamate.* To a suspension of nitro-aryl **2a** (90.0 mg, 0.50 mmol, 1 eq) and Pd/C - 10% (19.0 mg, 10% wt.) in methanol (0.01 M), under stirring and at 0°C, was added carefully by portions sodium borohydride (95.0 mg, 2.51 mmol, 5 eq). The reaction mixture was stirred at 0°C until complete dissolution of sodium borohydride and was then allowed to react at r.t. The crude mixture was filtered through a pad of celite, concentrated under reduced pressure and purified by silica gel column chromatography (DCM:EtOAc; 70:30 to 40:60) to afforded aniline **3a** as a white powder (54.7 mg, 31%). ¹H NMR (CD₃OD-*d*₄, 200 MHz): *δ* 1.47 (s, 9H), 3.97 (s, 2H), 6.68 (ddd, *J* = 7.7, 2.3, 1.3 Hz, 1H), 7.11 (t, *J =* 7.7 Hz, 1H), 7.33 - 7.17 (m, 3H, H7).

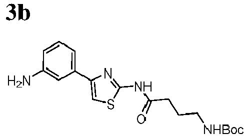

*Tert-butyl (4-((4-(3-aminophenyl)thiazol-2-yl)amino)-4-oxobutyl)carbamate.* To a suspension of nitro-aryl **2b** (266.0 mg, 0.65 mmol, 1 eq) and Pd/C - 10% (26.6 mg, 10% wt.) in methanol (0.01 M), under stirring and at 0°C, was added carefully by portions sodium borohydride (123.6 mg, 3.27 mmol, 5 eq). The reaction mixture was stirred at 0°C until complete dissolution of sodium borohydride and was then allowed to react at r.t. The crude mixture was filtered through a pad of celite, concentrated under reduced pressure and purified by silica gel column chromatography (DCM:MeOH; 100:0 to 90:10) to afford the titled compound **3b** as a white powder (190.1 mg, 77%). ¹H NMR (CD₃OD-*d*₄, 200 MHz): *δ* 1.42 (s, 9H), 1.86 (quint., *J =* 7.1 Hz, 2H), 2.49 (t, *J =* 7.4 Hz, 2H), 3.12 (t, *J =* 6.8 Hz, 2H), 6.68 (dd, *J =* 9.4, 1.7 Hz, 1H), 7.11 (t, *J =* 7.7 Hz, 1H), 7.31 - 7.16 (m, 3H).

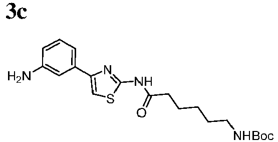

*Tert-butyl (6-((4-(3-nitrophenyl)thiazol-2-yl)amino)-6-oxohexyl)carbamate.* To a suspension of nitro-aryl 2c (346.0 mg, 0.80 mmol, 1 eq) and Pd/C - 10% (34.6 mg, 10% wt.) in methanol (0.01 M), under stirring and at 0°C, was added carefully by portions sodium borohydride (158.8 mg, 4.18 mmol, 5 eq). The reaction mixture was stirred at 0°C until complete dissolution of sodium borohydride and was then allowed to react at r.t. The crude mixture was filtered through a pad of celite, concentrated under reduced pressure and purified by silica gel column chromatography ((DCM:MeOH; 100:0 to 94:6) to afford the aniline **3c** as a white powder (166.5 mg, 52%). ¹H NMR (DMSO-*d₆*, 200 MHz): *δ* 1.42 - 1.22 (m, 13H), 1.59 (quint., *J =* 13.8, 7.1 Hz, 2H), 2.55 - 2.34 (m, 2H), 2.90 (q, *J =* 6.4 Hz, 2H), 5.12 (s, 2H), 6.52 (dt, *J =* 6.5, 2.6 Hz, 1H), 6.78 (t, *J =* 5.5 Hz, 1H), 7.16 - 6.95 (m, 3H), 7.35 (s, 1H), 12.18 (s, 1H).

Then, compounds 4a and PB631 and PB624 were synthetized according to the following methods:

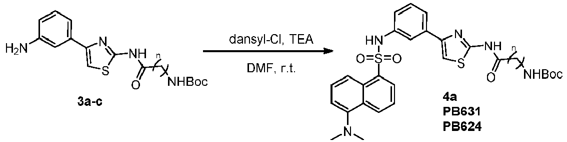

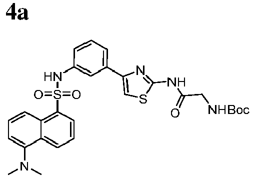

*Tert-butyl(2-((4-(3-((5-(dimethylamino)naphthalene)-1-sulfonamido)phenyl)thiazo1-2-yl)amino)-2-oxoethyl)carbamate.* To a solution of **3a** (54.0 mg, 0.16 mmol, 1 eq) under argon in anhydrous DMF (0.2 M) were added anhydrous triethylamine (1.6 eq) and dansyl chloride (49.9 mg, 0.19 mmol, 1.1 eq). The reaction mixture was allowed to react at r.t. until complete conversion of the starting material. DMF was removed under reduced pressure and the crude material was purified by silica gel column chromatography (DCM:EtOAc; 100:0 to 80:20) to afford **4a** as a yellow fluorescent powder (40.0 mg, 44%). ¹H NMR (CD₃OD-*d*₄, 200 MHz): *δ* 1.46 (s, 9H), 3.96 (s, 2H), 6.88 (dd, *J =* 8.0, 2.2 Hz, 1H), 7.10 - 6.98 (m, 2H), 7.17 (d, *J =* 7.6 Hz, 1H), 7.48 - 7.29 (m, 2H), 7.70 - 7.47 (m, 2H), 8.20 (d, *J =* 7.3 Hz, 1H), 8.41 (t, *J =* 7.9 Hz, 2H).

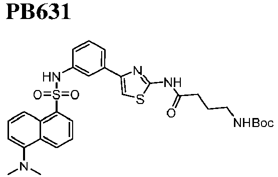

*Tert-butyl (4-((4-(3-((5-(dimethylamino)naphthalene)-1-sulfonamido)phenyl)thiazol-2-yl)amino)-4-oxobutyl)carbamate.* To a solution of **3b** (117.0 mg, 0.31 mmol, 1 eq) under argon in anhydrous DMF (0.2 M) were added anhydrous triethylamine (1.6 eq) and dansyl chloride (100.3 mg, 0.37 mmol, 1.1 eq). The reaction mixture was allowed to react at r.t. until complete conversion of the starting material. DMF was removed under reduced pressure and the crude material was purified by silica gel column chromatography (DCM:EtOAc; 100:0 to 70:30) to afford **PB631** as a yellow fluorescent powder (135.7 mg, 65%). ¹H NMR (CD₃OD-*d*₄, 200 MHz): *δ* 1.41 (s, 9H), 1.85 (quint., *J* = 7.2 Hz, 2H), 2.48 (t, *J =* 7.3 Hz, 2H), 2.79 (s, 6H), 3.18 - 3.05 (m, 2H), 6.96 - 6.82 (m, 1H), 7.16 - 7.01 (m, 2H), 7.22 (d, *J =* 7.6 Hz, 1H), 7.49 - 7.37 (m, 2H), 7.65 - 7.53 (m, 2H), 8.20 (d, *J =* 7.3 Hz, 1H), 8.44 (d, *J =* 8.6 Hz, 2H). ¹³C NMR (CD₃OD-*d*₄, 50 MHz): *δ* 26.6, 28.7, 33.9, 40.8, 45.7, 80.0, 108.9, 116.4, 119.1, 120.3, 120.8, 122.9, 124.1, 129.2, 130.2, 131.0, 131.0, 131.3, 131.5, 136.0, 136.8, 139.2, 150.4, 153.2, 158.5, 159.3, 173.2. MS-ESI (m/z): [M+H]⁺ = 610.3.

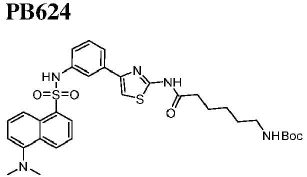

*Tert-butyl(6-((4-(3-((5-(dimethylamino)naphthalene)-1-sulfonamido)phenyl)thiazo1-2-yl)amino)-6-oxohexyl)carbamate.* To a solution of **3c** (250.0 mg, 0.62 mmol, 1 eq) under argon in anhydrous DMF (0.2 M) were added anhydrous triethylamine (1.6 eq) and dansyl chloride (199.6 mg, 0.74 mmol, 1.1 eq). The reaction mixture was allowed to react at r.t. until complete conversion of the starting material. DMF was removed under reduced pressure and the crude material was purified by silica gel column chromatography (DCM:EtOAc; 100:0 to 80:20) to afford **PB624** as a yellow fluorescent powder (319.5 mg, 81%). ¹H NMR (Acetone-*d₆*, 200 MHz): *δ* 1.28 - 1.56 (m, 13H), 1.74 (quint, *J* = 7.4 Hz, 2H), 2.58 (t, *J* = 7.4 Hz, 2H), 2.82 (s, 6H), 3.08 (q, *J* = 6.5 Hz, 2H), 5.95 (s, 1H), 7.01 (ddd, *J* = 8.0, 2.3, 1.1 Hz, 1H), 7.31 - 7.08 (m, 3H), 7.67 - 7.40 (m, 3H), 7.78 (t, *J* = 1.9 Hz, 1H), 8.28 (dd, *J* = 7.3, 1.2 Hz, 1H), 8.50 (dt, *J* = 8.8, 1.0 Hz, 2H), 9.40 (s, 1H), 11.10 (s, 1H). ¹³C NMR (Acetone-*d₆*, 50 MHz): *δ* 25.7, 27.1, 28.7, 36.3, 41.0, 45.6, 78.4, 108.7, 116.2, 118.4, 120.1, 122.5, 124.1, 129.1, 130.2, 130.6, 130.7, 131.1, 131.3, 136.1, 136.6, 139.2, 149.7, 152.9, 156.7, 159.0, 172.2. MS-ESI (*m*/*z*): [M+H]⁺= 638.4.

Intermediate compounds 5a, 5b and 5c were synthetized according to the following method:

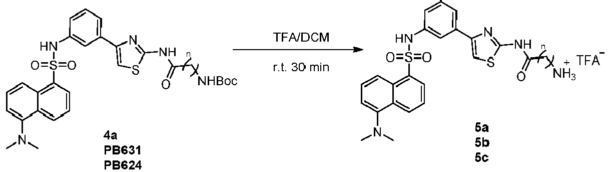

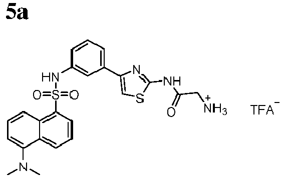

*2-ammonium-N-(4-(3-((5-(dimethylamino)naphthalene)-1-sulfonamido)phenyl)thiazol-2-yl)acetamide trifluoroacetate.* To a stirring solution of the BOC-protected **4a** (40.0 mg, 0.07 mmol) in DCM (1 mL) was added TFA (1 mL, excess). Mixture was allowed to react 30 min at r.t. and the mixture of TFA:DCM was removed under reduced pressure. Crude residue was suspended in a minimum amount of DCM and precipitated with diethyl ether and washed 3 times with diethyl ether (10 mL) to afford ammonium **5a** as yellow fluorescent powder (30.5 mg, 73%). ¹H NMR (CD₃OD-*d₄*, 200 MHz): *δ* 2.89 (s, 6H), 3.98 (s, 2H), 6.97 - 6.79 (m, 1H), 7.09 (t, *J* = 7.9 Hz, 1H), 7.24 (s, 1H), 7.35 (d, *J* = 7.6 Hz, 1H), 7.70 - 7.41 (m, 4H), 8.24 (d, *J* = 7.3 Hz, 1H), 8.48 (t, *J* = 13.9, 2H). ¹³C NMR (CD₃OD-*d₄*, 50 MHz): *δ* 41.9, 46.0, 109.6, 116.9, 119.2, 121.1, 123.0, 124.5, 129.4,130.4, 130.9,131.41, 131.3, 131.5, 136.3, 136.7, 139.4, 150.8, 152.4, 158.6, 165.9. HRMS-ESI (*m*/*z*): [M+H]⁺ calcd for C₂₃H₂₄O₃N₅S₂, 482.1315; found: 482.1315. HPLC (λ₂₅₄) Purity: 98.4 %; *t*_{R}: 5.78 min (method 3).

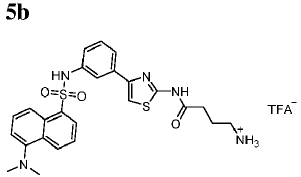

*4-((4-(3-((5-(dimethylamino(naptalene)-1-sulfonamido)phenyl)thiazol-2-yl)amino)-oxobutan-1-aminium trifluoroacetate.* To a stirring solution of the BOC-protected **PB631** (108.0 mg, 0.18 mmol) in DCM (1 mL) was added TFA (1 mL, excess). Mixture was allowed to react 30 min at r.t. and the mixture of TFA:DCM was removed under reduced pressure. Crude residue was suspended in a minimum amount of DCM and precipitated with diethyl ether and washed 3 times with diethyl ether (15 mL) to afford ammonium **5b** as yellow fluorescent powder (106.5 mg, 96%). ¹H NMR (CD₃OD-*d₄*, 200 MHz): *δ* 2.03 (p, *J* = 7.1 Hz, 2H), 2.64 (t, *J* = 7.0 Hz, 2H), 3.07 - 2.97 (m, 8H), 6.86 (ddd, *J* = 8.0, 2.3, 1.0 Hz, 1H), 7.28 - 7.01 (m, 2H), 7.75 - 7.37 (m, 5H), 8.26 (dd, *J* = 7.4, 1.2 Hz, 1H), 8.45 (d, *J* = 8.6, 1H), 8.59 (d, *J* = 8.7 Hz, 1H). ¹³C NMR (CD₃OD-*d₄*, 50 MHz): *δ* 23.9, 33.2, 40.4, 46.4, 109.2, 117.6, 119.2, 121.0, 122.4, 123.1, 125.1, 129.3, 130.3, 130.4, 130.5, 131.1, 131.7, 136.6, 136.9, 139.3, 150.3, 150.6, 159.3, 172.3. HRMS-ESI (*m*/*z*): [M+H]⁺ calcd for C₂₅H₂₈O₃N₅S₂, 510.1628; found: 510.1623.. HPLC (λ₂₅₄) Purity: 97.2%; *t*_{R}: 2.78 min (method 3).

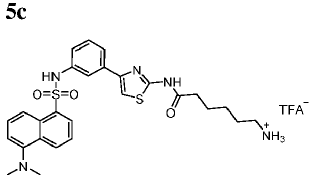

*6-((4-(3-((5-(dimethylamino)naphthalene)-1-sulfonamido)phenyl)thiazol-2-yl)amino)-6-oxohexan-1-aminium trifluoroacetate.* To a stirring solution of the BOC-protected **PB624** (1.25 g, 1.95 mmol) in DCM (4 mL) was added TFA (4.24 mL, 57.10 mmol). Mixture was allowed to react 30 min at r.t. and the mixture of TFA:DCM was removed under reduced pressure. Crude residue was suspended in a minimum amount of DCM and precipitated with diethyl ether and washed 3 times with diethyl ether (20 mL) to afford pure ammonium **5c** as yellow fluorescent powder (1.10 g, 87 %). ¹H NMR (DMSO-*d₆*, 200 MHz): *δ* 1.45 - 1.18 (m, 2H), 1.77 - 1.43 (m, 4H), 2.56 - 2.39 (m, 2H), 2.92 - 2.67 (m, 8H), 6.97 (d, *J* = 8.2 Hz, 1H), 7.25 - 7.15 (m, 2H), 7.49 - 7.37 (m, 2H), 7.72 - 7.51 (m, 3H), 7.85 (s, 3H), 8.33 - 8.16 (m, 1H), 8.40 (d, *J* = 8.4 Hz, 2H), 10.82 (s, 1H), 12.25 (s, 1H). ¹³C NMR (DMSO-*d₆*, 50 MHz): *δ* 24.2, 25.4, 26.8, 34.7, 45.0, 108.4, 115.3, 116.1, 118.2, 118.7, 120.9, 123.5, 128.2, 129.0, 129.5, 129.8, 130.1, 134.8, 135.2, 138.2, 148.1, 151.5, 158.0, 171.5. HRMS-ESI (*m*/*z*): [M+H]⁺ calcd for C₂₇H₃₃O₃N₅S₂, 538.1941; found: 538.1937. HPLC (λ₂₅₄)Purity: 97.5%; *t*_{R}: 7.17 min (method 2).

**Compounds PB633 and PB628 were synthetized based on 5b and 5c according to the following method:**

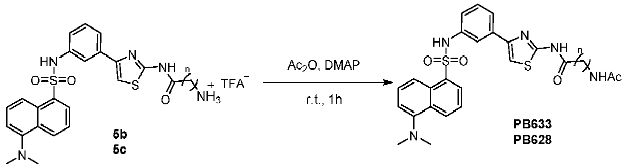

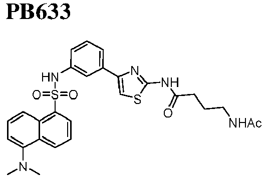

*4-((4-(3-((5-(dimethylamino(naptalene)-1-sulfonamido)phenyl)thiazol-2-yl)butanamide.* The corresponding ammonium **5b** (93.0 mg, 0.15 mmol) was dissolved in acetic anhydride (1 mL, excess) and DMAP (22.0 mg, 0.18 mmol) was then added to the mixture. The reaction middle was allowed to react at r.t. for 1h until complete consumption of the starting material and the product was precipitated using diethyl ether. The yellow fluorescent residue was filtrated, washed 3 times with Et₂O and dried afforded corresponding amide **PB633** as a yellow fluorescent powder (71.5 mg, 84%). ¹H NMR (DMSO-*d₆*, 200 MHz): *δ* 1.89 - 1.59 (m, 5H), 2.56 - 2.37 (m, 2H), 2.76 (s, 6H), 3.10 - 2.96 (m, 2H), 6.95 (d, *J* = 7.8 Hz, 1H), 7.26 - 7.09 (m, 2H), 7.47 - 7.41 (m, 2H), 7.68 - 7.51 (m, 3H), 7.86 (s, 1H), 8.22 (d, *J* = 7.3 Hz, 1H), 8.39 (d, *J* = 8.5 Hz, 2H), 10.76 (s, 1H), 12.19 (s, 1H). ¹³C NMR (CD₃OD-*d₄*, 50 MHz): *δ* 22.6, 24.7, 32.4, 38.0, 45.0, 108.4, 115.3, 116.1, 118.2, 118.6, 120.9, 123.5, 128.2, 129.0, 129.0, 129.4, 129.7, 130.1, 134.8, 135.2, 138.1, 148.1, 151.5, 157.9, 169.1, 171.3. HRMS-ESI (*m*/*z*): [M+H]⁺ calcd for C₂₇H₃₀O₄N₅S₂, 552.1734; found: 552.1733. HPLC (λ₂₅₄)Purity: > 99%; *t*_{R}: 7.42 min (method 4).

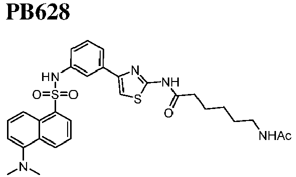

*6-acetamido-N-(4-(3-((5-(dimethylamino)naphthalene)-1-sulfonamido)phenyl)thiazol-2-yl)hexanamide.* The corresponding ammonium **5c** (50.0 mg, 0.08 mmol) was dissolved in acetic anhydride (1 mL, excess) and DMAP (12.3 mg, 0.10 mmol) was then added to the mixture. The reaction middle was allowed to react at r.t. for 1h until complete consumption of the starting material and the product was precipitated using diethyl ether. The yellow fluorescent residue was filtrated, washed 3 times with Et₂O and dried afforded corresponding amide **PB628** as a yellow fluorescent powder (43.6 mg, 94%). ¹H NMR (DMSO-*d₆*, 200 MHz): *δ* 1.51 - 1.17 (m, 4H), 1.59 (m, *J* = 7.4 Hz, 2H), 1.77 (s, 3H), 2.55 - 2.36 (m, 2H), 2.78 (s, 6H), 3.01 (q, *J* = 6.4 Hz, 2H), 7.3 - 6.91 (m, 1H), 7.30 - 7.13 (m, 2H), 7.43 (d, *J* = 8.6 Hz, 2H), 7.60 (ddd, *J* = 8.6, 7.4, 5.8 Hz, 3H), 7.82 (s, 1H), 8.24 (dd, *J* = 7.4, 1.2 Hz, 1H), 8.40 (dd, *J* = 8.5, 6.0 Hz, 2H), 10.79 (s, 1H), 12.21 (s, 1H). ¹³C NMR (DMSO-*d₆*, 50 MHz): *δ* 22.6, 24.4, 26.0, 28.9, 34.8, 38.4, 45.0, 108.3, 115.2, 116.2, 118.2, 118.7, 120.9, 123.5, 128.2, 129.0, 129.0, 129.4, 129.4, 129.7, 130.1, 134.8, 135.2, 138.1, 148.1, 151.4, 157.9, 168.9, 171.6. HRMS-ESI (*m*/*z*): [M+H]⁺ calcd for C₂₉H₃₄O₄N₅S₂, 580.2047; found: 580.2048. HPLC (λ₂₅₄)Purity: > 99%; *t*_{R}: 8.03 min (method 2).

Compound PB649 was synthetized based on compound 5a:

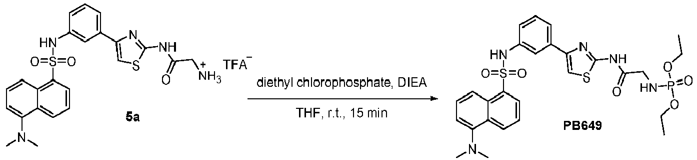

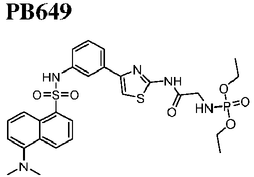

*Diethyl-(2-((4-(3-((5-(dimethylamino)naphthalene)-1-sulfonamido)phenyl)thiazol-2-yl)amino)-2-oxoethyl)phosphoramidate.* To a stirring solution of amine **5a** (75.0 mg, 0.16 mmol) in dry THF (1 mL) was successively added DIEA (41.0 mg, 0.32 mmol) and diethyl chlorophosphate (23.0 µL, 0.16 mmol). The mixture was allowed to react 15 min at r.t. until complete consumption of the starting material (TLC DCM:EtOAc 40:60). The reaction mixture was diluted with 10 mL of water and extracted 3 times with EtOAc (5 mL). Combined organic layer was washed 2 times with brine, dried over MgSO₄ and concentrated under reduced pressure. Purification by silica gel column chromatography (DCM:EtOAc 100:0 to 0:100) afforded **PB649** in 64% yield as yellow fluorescent powder (63.1 mg). ¹H NMR (Acetone-*d₆*, 500 MHz) δ 1.28 (td, *J* = 7.1, 0.8 Hz, 6H), 2.82 (s, 6H), 3.95 (dd, *J* = 13.1, 7.1 Hz, 2H), 4.06 - 4.14 (m, 4H), 4.69 (dt, *J* = 11.3, 7.1 Hz, 1H), 7.05 (ddd, *J* = 8.1, 2.3, 1.0 Hz, 1H), 7.16 (q, *J* = 7.9 Hz, 1H), 7.25 (d, *J* = 7.6 Hz, 1H), 7.31 (s, 1H), 7.49 (d, *J* = 8.2 Hz, 1H), 7.58 (ddd, *J* = 13.0, 8.6, 7.5 Hz, 2H), 7.77 (t, *J* = 1.9 Hz, 1H), 8.30 (dd, *J* = 7.3, 1.2 Hz, 1H), 8.50 (d, *J* = 8.2 Hz, 2H), 9.52 (s, 1H), 11.19 (s, 1H). ¹³C NMR (Acetone-*d₆*, 126 MHz) δ 16.6, 16.6, 45.4, 45.6, 63.2, 63.2, 109.0, 116.2, 118.2, 119.9, 120.1, 122.3, 124.2, 129.1, 130.2, 130.6, 130.7, 131.1, 131.3, 136.2, 136.4, 139.3, 149.9, 152.9, 158.5, 170.2, 206.2, 206.4, 206.5, 206. 7; ³¹P NMR (81 MHz, Acetone) δ 8.61. HRMS-ESI (*m*/*z*): [M+H]⁺ calc. for C₂₇H₃₃N₅O₆PS₂, 618.1604; Found: 618.1606. HPLC (λ₂₅₄): Purity 95.2 %; *t*_{R}: 6.35 min (method 1).

### Compound PB635

Compounds 6, 7 and 8 were synthetized according to the following method in order to obtain compound PB635

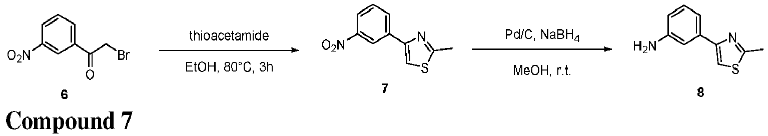

*2-methyl-4-(3-nitrophenyl)thiazole.* To a solution of α-bromo-3-nitroacetophenone **6** (1.00 g, 6.06 mmol 1 eq) in ethanol was added thioacetamide (460.0 mg, 6.06 mmol 1 eq). The reaction mixture was then heated to 80°C for 3 hours and left to cool to room temperature. The precipitate was filtered and washed with ethanol and diethyl ether to afford the thiazole 7 as yellow solid in 96% yield (1.28 g) as yellow solid. ¹H NMR (DMSO-*d₆*, 200 MHz): *δ* 2.72 (s, 3H), 7.68 (t, *J* = 8.0 Hz, 1H), 8.13 (ddd, *J* = 8.2, 2.3, 1.0 Hz, 1H), 8.21 (s, 1H), 8.33 (ddd, *J* = 7.8, 1.7, 1.0 Hz, 1H), 8.72 - 8.65 (m, 1H).

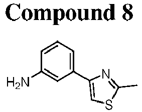

*2-methyl-4-(3-aminophenyl)thiazole.* To a suspension of nitro-aryl 7 (1.20 g, 5.50 mmol, 1 eq) and Pd/C - 10% (120.0 mg, 10% wt.) in methanol (0.01 M), under stirring and at 0°C, was added carefully by portions sodium borohydride (2.07 g, 27.50 mmol, 5 eq). The reaction mixture was stirred at 0°C until complete dissolution of sodium borohydride and was then allowed to react at r.t. The crude mixture was filtered through a pad of celite, concentrated under reduced pressure and purified by silica gel column chromatography (CHx:EtOAc; 100:0 to 50:50) to afford aniline **8** as a white powder (760.0 mg, 73%). ¹H NMR (CD₃OD-*d₄*, 200 MHz): *δ* 2.70 (s, 3H), 6.70 (dt, *J* = 6.5, 2.3 Hz, 1H), 7.29 - 7.05 (m, 3H), 7.45 (s, 1H).

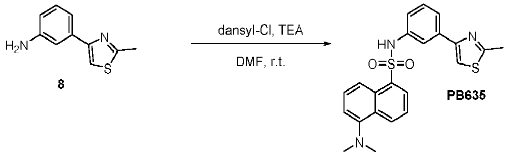

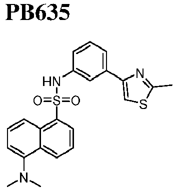

*5-(dimethylamino)-N-(3-(2-methylthiazol-4-yl)phenyl)naphthalene-1-sulfonamide.* To a solution of **8** (100.0 mg, 0.53 mmol, 1 eq) under argon in anhydrous DMF (0.2 M) were added anhydrous triethylamine (1.6 eq) and dansyl chloride (169.9 mg, 0.63 mmol, 1.1 eq). The reaction mixture was allowed to react at r.t. until complete conversion of the starting material. DMF was removed under reduced pressure. Pure compound was precipitated with Et₂O from DCM and washed three times with diethyl ether (20 mL) to obtain **PB635** as a yellow fluorescent powder (107.5, mg, 48%). ¹H NMR (Acetone-*d₆*, 200 MHz): δ 2.68 (s, 3H), 2.82 (s, 6H), 7.30 - 7.05 (m, 3H), 7.69 - 7.49 (m, 4H), 7.85 - 7.74 (m, 1H), 8.32 (dd, *J* = 7.4, 1.2 Hz, 1H), 8.51 (dd, *J* = 8.3, 1.1 Hz, 2H), 9.44 (s, 1H). ¹³C NMR (Acetone-*d₆*, 50 MHz): δ 19.2, 45.6,114.2, 116.3, 118.6, 119.9, 120.1, 122.6, 124.2, 129.1, 130.3, 130.7, 130.7, 131.2, 131.4, 136.2, 136.5, 139.3, 153.0, 154.9, 166.5. HRMS-ESI (m/z): [M+H]⁺ calcd for C₂₂H₂₂O₂N₃S₂, 424.1148; found: 424.1146. HPLC (λ₂₅₄)Purity: > 99%; tR: 4.81 min (method 4).

### Compound PB625

Compound 9 and 10 were synthetized according to the following method in order to obtain PB625

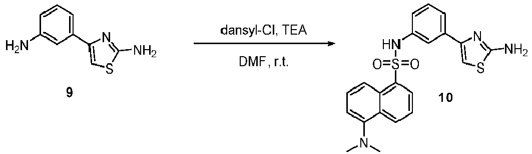

*N-(3-(2-aminothiazol-4-yl)phenyl)-5-(dimethylamino)naphthalene-1-sulfonamide.* To a solution of **9** (0.88 g, 4.60 mmol, 1 eq) under argon in anhydrous DMF (0.2 M) were added anhydrous triethylamine (1.6 eq) and dansyl chloride (1.37 g, 5.10 mmol, 1.1 eq). The reaction mixture was allowed to react at r.t. until complete conversion of the starting material. DMF was removed under reduced pressure and the crude material was purified by silica gel column chromatography (DCM:EtOAc; 100:0 to 70:30) to afford **10** as a pale yellow fluorescent powder (1.74 g, 89%). ¹H NMR (DMSO-*d₆*, 200 MHz): *δ* 2.74 (s, 6H), 6.82 (s, 1H), 7.26 - 6.89 (m, 4H), 7.35 (d, *J* = 7.7 Hz, 1H), 7.69 - 7.51 (m, 3H), 8.25 (d, *J* = 7.3 Hz, 1H), 8.41 (d, *J* = 8.6 Hz, 2H), 10.71 (s, 1H). ¹³C NMR (DMSO-*d₆*, 50 MHz): *δ* 45.0, 102.0, 115.3, 116.3, 117.5, 118.7, 120.6, 123.5, 128.2, 129.0, 129.0, 129.1, 129.7, 130.1, 134.8, 135.7, 137.9, 149.3, 151.4, 168.1. HRMS-ESI (*m*/*z*): [M+H]⁺ calcd for C₂₁H₂₁N₄O₂S₂, 425.1100; found: 425.1103. HPLC (λ₂₅₄)Purity: 99.2%; *t*_{R}: 3.34 min (method 3).

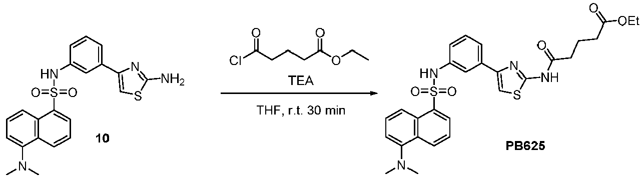

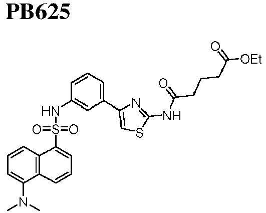

*Ethyl 5-((4-(3-((5-(dimethylamino)naphthalene)-1-sulfonamido)phenyl)thiazol-2-yl)amino)-5-oxopentanoate.* To a stirred solution of the amine 10 (101.5 mg, 0.24 mmol) in dry THF (1.2 mL), under argon, was added successively DIPEA (50.0 µL, 0.29 mmol) and glutaric acid monomethyl ester chloride (40.0 µL, 0.29 mmol). The mixture was stirred at r.t. for 1h until complete consumption of the starting material (monitored by TLC using DCM:EtOAC (70:30) as eluent). All the volatiles were removed under reduced pressure and the crude residue was subjected to silica gel flash chromatography purification (DCM:EtOAc; 100:0 to 70:30) to afford the corresponding amide **PB625** as a pale yellow fluorescent powder (83.1 mg, 92%). ¹H NMR (Acetone-*d₆*, 200 MHz): *δ* 1.20 (t, *J* = 7.1 Hz, 3H), 2.13 - 1.91 (m, 2H), 2.41 (t, *J* = 7.3 Hz, 2H), 2.65 (t, *J* = 7.3 Hz, 2H), 2.81 (s, 6H) 4.08 (q, *J* = 7.1 Hz, 2H), 7.00 (ddd, *J* = 8.0, 2.2, 1.1 Hz, 1H), 7.14 (t, *J* = 7.9 Hz, 1H), 7.33 - 7.20 (m, 2H), 7.69 - 7.40 (m, 3H), 7.77 (t, *J* = 1.9 Hz, 1H), 8.27 (dd, *J* = 7.3, 1.2 Hz, 1H), 8.49 (d, *J* = 8.8, 2H), 9.46 (s, 1H), 11.15 (s, 1H). ¹³C NMR (Acetone-*d₆*, 50 MHz): *δ* 14.6, 21.1, 33.9, 35.2, 45.6, 60.7, 108.7, 116.2, 118.3, 120.1, 122.4, 124.1, 129.1, 130.2, 130.6, 130.7, 131.1, 131.3, 136.1, 136.6, 139.2, 149.8, 152.9, 158.9, 171.7, 173.3. HRMS-ESI (*m*/*z*): [M+H]⁺ calcd for C₂₈H₃₁O₅N₄S₂, 567.1730; found: 527.1740. HPLC (λ₂₅₄)Purity: 96.0 %; *t*_{R}: 4.88 min (method 1).

### Compounds PB638, 639, 640, 643 and 644

Compounds 10 and 11 were first synthetized according to the following methods:

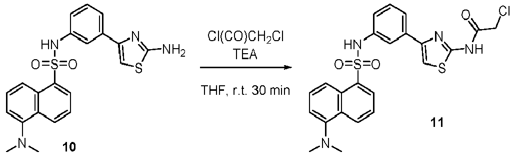

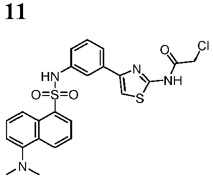

*2-chloro-N-(3-(2-(2-chloroacetamido)thiazol-4-yl)phenyl)-N-((5-(dimethylamino)naphthalen-1-yl)sulfonyl)acetamide.* To a stirring solution of amine **10** (1.08 g, 2.55 mmol) in dry THF (17 mL) was added DIEA (1.33 mL, 7.65 mmol). Then, the chloroacetyl chloride (0.41 mL, 5.10 mmol) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced pressure yielded chloroacetyl **11** as a brown-yellow fluorescent powder (1.36 g, quant.). ¹H NMR (Acetone-*d₆*, 200 MHz): *δ* 2.80 (s, 6H), 4.49 (s, 2H), 7.04 (d, *J* = 9.0 Hz, 1H), 7.28 - 7.09 (m, 2H), 7.36 (s, 1H), 7.64 - 7.41 (m, 1H), 7.81 (t, *J* = 1.7 Hz, 1H), 8.29 (dd, *J* = 7.3, 1.2 Hz, 1H), 8.49 (d, *J* = 11.7 Hz, 2H).

In order to obtain compounds PB638, 639, 640, 643 and 644 according to the following method:

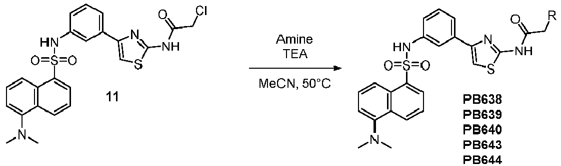

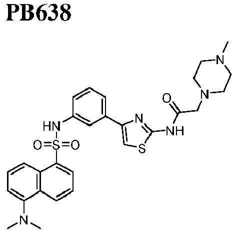

*N-(4-(3-((5-(dimethylamino)naphthalene)-1-sulfonamido)phenyl)thiazol-2-yl)-2-(4-methylpiperazin-1-yl)acetamide.* To a suspension of the chloroalkyl **11** (75.0 mg, 0.13 mmol, 1 eq) in acetonitrile (0.2 M) was added triethylamine (1 eq) and *N*-methylpiperazine (39.0 mg, 0.39 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) to afford **PB638** as yellow fluorescent powder (38.4 mg, 70%). ¹H NMR (Acetone-*d₆*, 200 MHz): *δ* 2.27 (s, 3H), 2.71 - 2.41 (m, 8H), 2.81 (s, 6H), 3.26 (s, 2H), 7.28 - 7.02 (m, 3H), 7.33 (s, 1H), 7.65 - 7.45 (m, 3H), 7.75 (t, *J* = 2.0 Hz, 1H), 8.31 (dd, *J* = 7.3, 1.3 Hz, 1H), 8.50 (d, *J* = 8.8, 1H). ¹³C NMR (Acetone-*d₆*, 50 MHz): *δ* 45.6, 46.1, 53.8, 55.7, 61.7, 109.0, 116.3, 118.3, 120.0, 120.1, 122.4, 124.1, 129.1, 130.2, 130.7, 130.7, 131.1, 131.3, 136.2, 136.4, 139.2, 149.9, 152.9, 158.3, 169.6. HRMS-ESI (*m*/*z*): [M+H]⁺ calcd for C₂₈H₃₃O₃N₆S₂, 565.2050; found: 565.2060. HPLC (λ₂₈₀) Purity: 96.9%; *t*_{R}: 5.80 min (method 4).

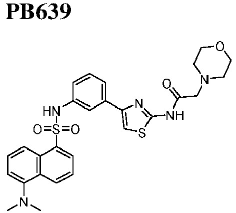

*N-(4-(3-((5-(dimethylamino)naphthalene)-1-sulfonamido)phenyl)thiazol-2-yl)-2-morpholinoacetamide.* To a suspension of the chloroalkyl **11** (75.0 mg, 0.13 mmol, 1 eq) in acetonitrile (0.2 M) was added triethylamine (1 eq) and morpholine (33.5 mg, 0.39 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) to afford morpholine **PB639** as yellow fluorescent powder (52.1 mg, 69%). ¹H NMR (Acetone-*d₆*, 200 MHz): *δ* 2.70 - 2.56 (m, 4H), 2.82 (s, 6H), 3.31 (s, 2H), 3.72 (dd, *J* = 5.7, 3.6 Hz, 4H), 7.38 - 6.99 (m, 4H), 7.68 - 7.43 (m, 3H), 7.75 (t, *J* = 1.9 Hz), 8.39 - 8.21 (m, 1H), 8.51 (ddt, *J* = 8.8, 3.8, 1.0 Hz, 2H), 9.43 (s, 1H), 10.76 (s, 1H). ¹³C NMR (CD₃OD-*d₄*, 125 MHz): *δ* 45.9, 54.9, 62.2, 67.9, 109.6, 116.6, 119.3, 120.5, 121.2, 123.1, 124.3, 129.4, 130.5, 131.1, 131.2, 131.5, 131.7, 136.2, 136.7, 139.5, 150.5, 153.36, 159.2, 170.6. HRMS-ESI (*m*/*z*): [M+H]⁺ calcd for C₂₇H₃₀O₄N₅S₂, 552.1734; found: 552.1741. HPLC (λ₂₈₀) Purity: 96.1 %; *t*_{R}: 3.18 min (method 3).

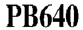

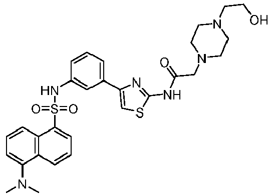

*N-(4-(3-((5-(dimethylamino)naphthalene)-1-sulfonamido)phenyl)thiazol-2-yl)-2-(4-(2-hydroxyethyl)piperazin-1-yl)acetamide.* To a suspension of the chloroalkyl 11 (75.0 mg, 0.13 mmol, 1 eq) in acetonitrile (0.2 M) was added triethylamine (1 eq) and morpholine (101.4 mg, 0.78 mmol, 6 eq). The reaction mixture was stirred at 50°C until complete consumption of starting material. The volatiles were removed under reduced pressure and crude residue was subjected to silica gel column chromatography (DCM:MeOH 100:0 to 90:10) to afford morpholine **PB640** as yellow fluorescent powder (45.2 mg, 58%). ¹H NMR (CD₃OD-*d₄*, 200 MHz): *δ* 2.69 - 2.54 (m, 10H), 2.81 (s, 6H), 3.29 (s, 2H), 3.70 (t, *J* = 6.0 Hz, 2H) 6.91 (ddd, *J* = 8.0, 2.3, 1.0 Hz, 1H), 7.11 (t, *J* = 7.9 Hz, 1H), 7.18 (s, 1H), 7.23 (d, *J* = 7.3 Hz, 1H), 7.49 - 7.35 (m, 2H), 7.63 - 7.53 (m, 2H), 8.20 (dd, *J* = 7.4, 1.3 Hz, 1H), 8.51 - 8.39 (m, 2H). ¹³C NMR (CD₃OD-*d₄*, 125 MHz): *δ* 45.9, 54.1, 54.4, 59.9, 61.3, 61.7, 109.5, 116.6, 119.3, 120.5, 121.2, 123.1, 124.3, 129.4, 130.5, 131.1, 131.2, 131.5, 131.7, 136.2, 136.7, 139.5, 150.5, 153.3, 159.3, 170.7. HRMS-ESI (*m*/*z*): [M+H]⁺ calcd for C₂₉H₃₅O₄N₆S₂, 595.2158; found: 595.2162. HPLC (λ₂₈₀) Purity: 95.7%; *t*_{R}: 5.76 min (method 4).

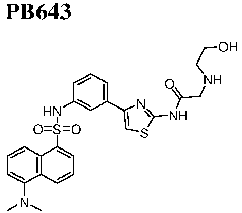

*N-(4-(3-((5-(dimethylamino)naphthalene)-1-sulfonamido)phenyl)thiazol-2-yl)-2-((2-hydroxyethyl)amino)acetamide.* To a suspension of the chloroalkyl **11** (150.0 mg, 0.26 mmol, 1 eq) in acetonitrile (0.2 M) was added triethylamine (1 eq) and morpholine (47.6 mg, 0.78 mmol, 3 eq). The reaction mixture was stirred at 50°C until complete consumption of starting material. The volatiles were removed under reduced pressure and crude residue was subjected to silica gel column (DCM:MeOH 100:0 to 90:10) afforded hydroxyl **PB643** as yellow fluorescent powder (84.1 mg, 62%). ¹H NMR (CD₃OD-*d₄*, 200 MHz): *δ* 2.85 - 2.61 (m, 8H), 3.54 (s, 2H), 3.67 (t, *J* = 5.4 Hz, 2H), 6.94 - 6.83 (m, 1H), 7.08 - 6.96 (m, 2H), 7.13 (dd, *J* = 7.6, 0.9 Hz, 1H), 7.43 - 7.25 (m, 2H), 7.62 - 7.48 (m, 2H), 8.19 (dd, *J* = 7.4, 1.2 Hz, 1H), 8.50 - 8.32 (m, 2H). ¹³C NMR (CD₃OD-*d₄*, 50 MHz): *δ* 45.8, 52.5, 52.6, 62.0, 109.3, 116.5, 119.1, 120.4, 120.8, 123.0, 124.2, 129.4, 130.4, 131.1, 131.1, 131.5, 131.7, 136.2, 136.7, 139.4, 150.4, 153.2, 159.2, 172.1. HRMS-ESI (*m*/*z*): [M+H]⁺ calcd for C₂₅H₂₈O₄N₅S₂, 526.1577; found: 526.1584. HPLC (λ₂₅₄) Purity: > 99 %; *t*_{R}: 5.09 min (method 1). 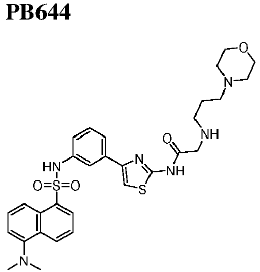 *N-(4-(3-((5-(dimethylamino)naphthalene)-1-sulfonamido)phenyl)thiazol-2-yl)-2-((3-morpholinopropyl)amino)acetamide.* To a suspension of the chloroalkyl **11** (150.0 mg, 0.26 mmol, 1 eq) in acetonitrile (0.2 M) was added triethylamine (1 eq) and morpholine (112.3 mg, 0.78 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) to afford morpholine **PB644** as yellow fluorescent powder (81.2 mg, 51%). ¹H NMR (CD₃OD-*d₄*, 200 MHz): *δ* 1.77 (m, 2H), 2.71 - 2.52 (m, 6H), 2.88 - 2.70 (m, 8H), 3.35 (s, 2H) 3.74 - 3.69 (m, 4H), 7.26 - 6.86 (m, 4H), 7.46 - 7.33 (m, 2H), 7.66 - 7.49 (m, 2H), 8.20 (d, *J* = 7.3 Hz, 1H), 8.41 (t, *J* = 7.7 Hz, 2H), . ¹³C NMR (CD₃OD-*d₄*, 50 MHz): *δ* 25.09. 25.3, 45.9, 51.8, 54.5, 58.6, 67.1, 109.5, 116.6, 119.1, 120.1, 120.9, 131.1, 131.1, 131.2, 131.4, 131.7, 136.2, 136.7, 139.5, 150.6, 153.3, 159.1, 170.7. HRMS-ESI (*m*/*z*): [M+H]⁺ calcd for C₃₀H₃₇O₄N₆S₂, 609.2312; found: 609.2321. HPLC (λ₂₈₀) Purity: 95.4%; *t*_{R}: 4.75 min (method 1).

### Compound PB654 and 605

Compounds 10 and 12 were first synthetized according to the following method:

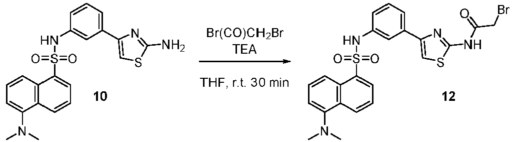

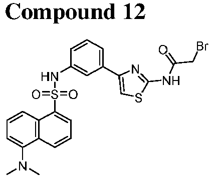

To a stirring solution of amine **10** (560.0 g, 1.32 mmol) in dry THF (7 mL) was added DIEA (274 µL, 1.58 mmol). Then, the bromoacetylbromide (138 µL, 1.58 mmol) was added dropwise to the mixture and allowed to react 60 min at r.t. until complete consumption of the starting material (TLC DCM:EtOAc 70:30). Next, the mixture was quenched with water (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄, concentrated under reduced pressure and purified using flash column chromatography with CH₂Cl₂:EtOAc (100:0 to 70:30) as eluent yielded bromoacetyl **12** as a brown-yellow fluorescent powder (648.0 mg, 90%). ¹H NMR (Acetone-*d₆*, 200 MHz): *δ* 2.75 (s, 6H), 4.23 (s, 2H), 7.25 - 6.95 (m, 4H), 7.31 (s, 1H), 7.65 - 7.37 (m, 3H), 7.78 (s, 1H), 8.26 (d, *J* = 7.3 Hz, 1H), 8.46 (t, *J* = 8.0 Hz, 2H), 9.45 (s, 1H), 11.57 (s, 1H).

Compound PB654 is synthetized based on compound 12:

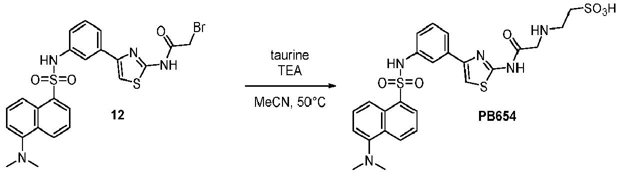

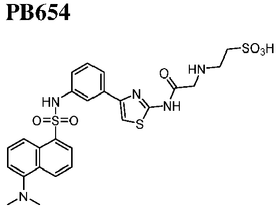

*2-((2-((4-(3-((5-(dimethylamino)naphthalene)-1-sulfonamido)phenyl)thiazol-2-yl)amino)-2-oxoethyl)amino)ethane-1-sulfonic.* To a mixture of H₂O and AcN (1:2 4mL) was added bromoacetyl **12** (110.0 mg, 0.2 mmol), DIPEA (69.5 µL, 0.4 mmol) and taurine (50.2 mg, 0.4 mmol). The mixture was allowed to react at r.t. for 2h and was then placed at 50°C for 5 additional hours until complete consumption of the starting material. The solvents were removed under reduced pressure and the crude was subjected to silica gel column chromatography (DCM:MeOH 95:5 to 85:15) yielded pure sulfonic acid **PB654** as a brown-yellow fluorescent product (89.6 mg, 76%). ¹H NMR (DMSO-*d₆*, 500 MHz): *δ* 2.66 (t, *J* = 6.7 Hz, 2H), 2.78 (s, 6H), 2.88 (t, *J* = 6.7 Hz, 2H), 3.52 (s, 2H), 6.98 (ddd, *J* = 8.1, 2.3, 1.0 Hz, 1H), 7.19 (t, *J* = 7.9 Hz, 1H), 7.24 (d, *J* = 7.2 Hz, 1H), 7.52 - 7.37 (m, 2H), 7.66 - 7.54 (m, 3H), 8.25 (dd, *J* = 7.4, 1.2 Hz, 1H), 8.41 (dd, *J* = 15.5, 8.6 Hz, 2H). ¹³C NMR (DMSO-*d₆*, 125 MHz): *δ* 45.0, 45.3, 50.5, 51.2, 108.6, 115.3, 116.1, 118.1, 118.7, 120.8, 123.5, 128.2, 129.0, 129.0, 129.4, 129.8, 130.1, 134.8, 135.0, 138.2, 148.2, 151.4, 157.6, 170.0. HRMS-ESI (*m*/*z*): [M+H]⁺ calcd for C₂₅H₂₈O₆N₅S₃, 590.1196; found: 590.1199. HPLC (λ₂₅₄)Purity: 96.4 %; *t*_{R}: 5.33 min (method 1).

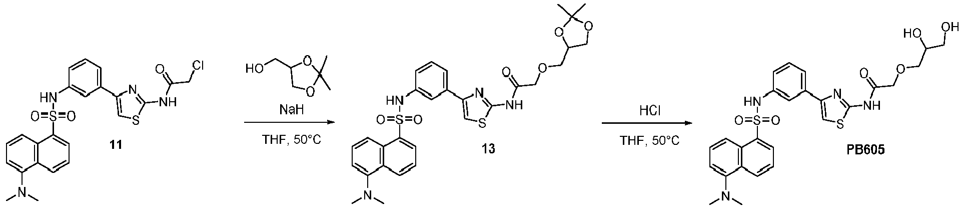

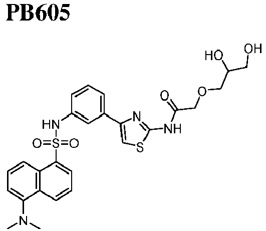

*2-(2,3-dihydroxypropoxy)-N-(4-(3-((5-(dimethylamino)naphthalene)-1-sulfonamido)phenyl)thiazol-2-yl)acetamide.* To a flask containing dry THF (1 mL), under argon and at 0°C, was added successively sodium hydride (17.6 mg, 0.44 mmol) followed by dropwise addition of a solution of glycerol isopropylidene (53.2 mg, 0.40 mmol). The resulting mixture was allowed to react 30 min at 0°C prior to the dropwise addition of a solution of chloroacetyl **11** (100.0 mg, 0.20 mmol) in THF (0.5 mL, 0.4 M). The mixture was reacted 5 min at 0°C and placed at r.t. for 1 additional hour. The mixture was quenched with water (2 mL) and volatiles were removed under reduced pressure. The resulting aqueous solution was extracted 3 times with EtOAc (5 mL) and combined organic layer was washed with brine and dried over MgSO₄. The crude residue was subjected so silica gel flash chromatography using DCM:EtOAc (100:0 to 60:40) as eluent afforded dioxolane intermediate **13** (54.1 mg, 45%). ¹H NMR (Acetone-*d₆*, 200 MHz): *δ* 1.37 (s, 3H), 1.45 (s, 3H), 2.81 (s, 6H), 4.17 - 3.63 (m, 4H), 4.48 - 4.28 (m, 3H), 7.11 - 6.95 (m, 1H), 7.30 - 7.11 (m, 2H), 7.33 (s, 1H), 7.70 - 7.47 (m, 3H), 7.79 (t, *J* = 1.9 Hz, 1H), 8.31 (d, *J* = 7.2 Hz, 1H), 8.50 (d, *J* = 8.5 Hz, 2H), 9.43 (s, 1H), 10.82 (s, 1H). The dioxolane intermediate **13** (45.0 mg, 0.075 mmol) was dissolved in THF (6 mL) and conc. HCl (37%, 2 mL) was added to the reaction middle. The mixture was stirred for 2h at r.t. and diluted with 10 mL of water. The crude was extracted 2 times with EtOAc (10 mL) and combined organic layer was washed with 20 mL of brine, dried over MgSO₄ and concentrated under reduced pressure. The crude residue was subjected to silica gel chromatography (DCM:MeOH 100:0 to 90:10) to afford the di-hydroxyl **PB605** in 82% yield (34.0 mg) ¹H NMR (Acetone-*d₆*, 200 MHz): δ 2.82 (s, 6H), 4.05 - 3.55 (m, 6H), 4.30 (s, 2H), 4.58 (s, 1H), 7.09 - 6.99 (m, 2H), 7.29 - 7.11 (m, 2H), 7.32 (s, 1H), 7.66 - 7.46 (m, 3H), 7.75 (t, *J* = 1.9 Hz, 1H), 8.32 (dd, *J* = 7.4, 1.3 Hz, 1H), 8.55 - 8.44 (m, 2H), 9.47 (s, 1H), 11.18 (s, 1H). ¹³C NMR (Acetone-d₆, 50 MHz): *δ* 45.6, 64.1, 70.8, 71.7, 74.8, 109.1, 116.2, 118.3, 119.9, 120.1, 122.5, 124.2, 129.1, 130.2, 130.6, 130.7, 131.2, 131.4, 136.1, 136.5, 139.2, 150.0, 152.9, 158.2, 169.9. HRMS-ESI (*m*/*z*): [M+H]⁺ calcd for. HRMS-ESI (*m*/*z*): [M+H]⁺ calcd for C₂₆H₂₉O₆N₄S₂, 557.1523; found: 557.1526. HPLC (λ₂₅₄) Purity: 97.5 %; *t*_{R}: 6.22 min (method 1).

### Compounds PB697 and PB698

Compounds 10 and 14 were used in order to obtain compounds PB697 and PB698 as follows:

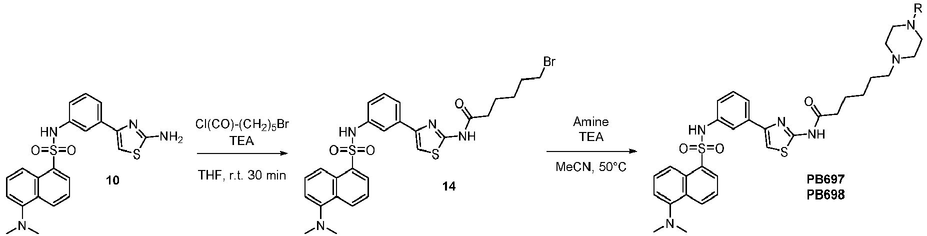

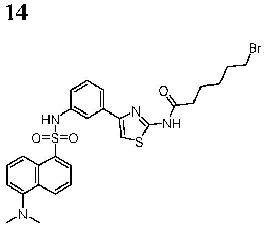

To a stirring solution of amine **10** (450 mg, 1.06 mmol) in dry THF (10 mL) was added DIEA (0.54 mL, 3.13 mmol). Then, the 6-bromohexanoyl chloride (0.33 mL, 2.15 mmol) was added dropwise to the mixture and allowed to react 30 min at r.t. until complete consumption of the starting material (TLC DCM:EtOAc 80:20). Next, the mixture was quenched with a 4M aqueous solution of sodium hydroxide (5 mL) and stirred for additional 30 min at r.t. The reaction mixture was diluted with 10 mL of water and extracted 3 times with EtOAc (30 mL). Combined organic layer was washed 2 times with brine, dried over MgSO₄ and concentrated under reduced pressure to give the bromo alkyl **14** (230 mg, 36% yield) as a brown-yellow powder. The crude bromo alkyl was directly used without further purification.

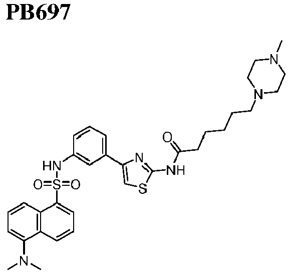

*N-(4-(3-((5-(dimethylamino)naphthalene)-1-sulfonamido)phenyl)thiazol-2-yl)-6-(4-methylpiperazin-1-yl)hexanamide.* To a suspension of the bromoalkyl **14** (100.0 mg, 0.17 mmol, 1 eq) in acetonitrile (0.2 M) was added triethylamine (1 eq) and N-methylpiperazine (51.0 mg, 0.51 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) to afford **PB697** in 30% yield as yellow fluorescent powder (29.2 mg). ¹H NMR (CD₂Cl₂-*d*₂, 400 MHz): δ 1.10 - 1.02 (m, 2H), 1.53 - 1.34 (m, 4H), 2.08 (t, *J* = 7.6 Hz, 2H), 2.49 (s, 3H), 2.66 (t, *J* = 7.1 Hz, 2H), 2.80 (s, 6H), 3.00 (br s, 8H), 7.00 (s, 1H), 7.16 - 7.11 (m, 3H), 7.44 - 7.40 (m, 2H), 7.58 (t, *J* = 7.8 Hz, 1H), 7.68 (s, 1H), 8.25 (dd, *J* = 7.3, 0.9 Hz, 1H), 8.46 (t, *J* = 6.2 Hz, 2H). ¹³C NMR (CD₂Cl₂-*d*₂ ,101 MHz,): δ 24.6, 24.7, 26.5, 35.9, 44.8, 45.5, 51.2, 52.7, 57.3, 108.8, 115.7, 118.1, 119.2, 119.9, 122.4, 123.4, 129.1, 129.9, 129.9, 130.1, 130.6, 131.3, 134.6, 135.7, 138.2, 149.0, 152.4, 159.5, 171.6. HRMS-ESI (*m*/*z*): [M+H]⁺ calc. for C₃₂H₄₁N₆O₃S₂, 621.2676; Found: 621.2664. HPLC (λ₂₅₄): Purity 94.1 %; *t*_{R}: 4.42 min (method mauro 7).

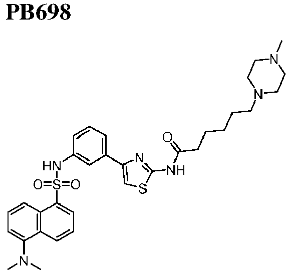

*N-(4-(3-((5-(dimethylamino)naphthalene)-1-sulfonamido)phenyl)thiazol-2-yl)-6-(4-(2-hydroxyethyl)piperazin-1-yl)hexanamide.* To a suspension of the bromoalkyl **14** (100.0 mg, 0.17 mmol, 1 eq) in acetonitrile (0.2 M) was added triethylamine (1 eq) and *N*-hydroxyethylpiperazine (66.0 mg, 0.51 mmol, 3 eq). The reaction mixture was stirred at 50°C until complete consumption of starting material. The volatiles were removed under reduced pressure and crude residue was subjected to silica gel column chromatography (DCM:MeOH 100:0 to 90:10) to afford **PB698** in 18% yield as yellow fluorescent powder (19.5 mg). ¹H NMR (CD₂Cl₂-*d*₂, 400 MHz): δ 1.32 - 1.25 (m, 2H), 1.72 - 1.60 (m, 4H), 2.37 (t, *J* = 7.5 Hz, 2H), 2.85 - 2.73 (m, 11H), 3.02 (br s, 4H), 3.12 (br s, 4H), 3.73 - 3.70 (m, 2H), 7.03 - 6.99 (m, 2H), 7.14 (dd, *J* = 15.6, 7.7 Hz, 2H), 7.39 (d, *J* = 7.8 Hz, 1H), 7.44 (dd, *J* = 8.4, 7.5 Hz, 1H), 7.60 - 7.56 (m, 2H), 8.24 (dd, *J* = 7.4, 1.1 Hz, 1H), 8.40 (d, *J* = 8.7 Hz, 1H), 8.46 (d, *J* = 8.5 Hz, 1H). ¹³C NMR (CD₂Cl₂-*d*₂, 101 MHz): δ 24.3, 24.6, 26.3, 35.7, 45.5, 50.8, 51.7, 57.2, 57.8, 59.2, 108.6, 115.7, 118.0, 119.0, 119.5, 122.1, 123.5, 128.9, 129.9, 129.9, 130.2, 130.5, 131.2, 134.8, 135.7, 138.2, 149.1, 152.4, 158.8, 172.0. HRMS-ESI (*m*/*z*): [M+H]⁺ calc. for C₃₃H₄₃N₆O₄S₂, 651.2769; Found: 651.2781. HPLC (λ₂₅₄): Purity 95.1 %; *t*_{R}: 4.36 min (method mauro 7).

### Compounds PB688, PB689 and PB693

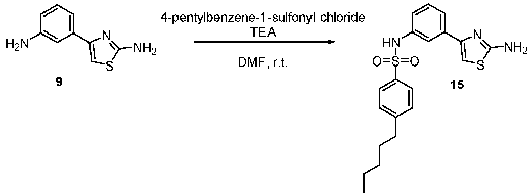

*N-(3-(2-aminothiazol-4-yl)phenyl)-4-pentylbenzenesulfonamide.* To a solution of **9** (1.06 g, 5.50 mmol, 1 eq) under argon in anhydrous DMF (0.2 M) were added anhydrous triethylamine (1.6 eq) and 4-pentylbenzene-1-sulfonyl chloride (1.50 g, 6.10 mmol, 1.1 eq). The reaction mixture was allowed to react at r.t. until complete conversion of the starting material. DMF was removed under reduced pressure and the crude material was purified by silica gel column chromatography (DCM:MeOH, 100:0 to 95:5) afforded the titled sulfonamide **15** as a white powder (1.16 g, 53%). ¹H NMR (CD₂Cl₂-*d₂*, 400 MHz) δ 0.86 (t, *J* = 7.0 Hz, 3H), 1.34 - 1.25 (m, 4H), 1.61 - 1.53 (m, 2H), 2.61 (t, *J* = 7.6 Hz, 2H), 5.29 (br s, 2H), 6.70 (s, 1H), 6.88 (s, 1H), 6.97 - 6.95 (m, 1H), 7.21 (t, *J* = 8.1 Hz, 1H), 7.25 (d, *J* = 8.3 Hz, 2H), 7.50 - 7.48 (m, 2H), 7.67 (d, *J* = 8.3 Hz, 2H). ¹³C NMR (CD₂Cl₂-*d₂*, 101 MHz) δ 14.1, 22.8, 31.2, 31.7, 36.1, 104.0, 119.4, 120.7, 123.0, 127.6, 129.4, 129.8 136.3, 136.6, 137.4, 149.5, 150.4, 167.9. HRMS-ESI (*m*/*z*): [M+H]⁺ calcd for C₂₀H₂₄N₃O₂S₂, 402.1304; found: 402.1298. HPLC (λ₂₅₄)Purity: > 96.6 %; *t*_{R}: 6.12 min (method mauro 3)

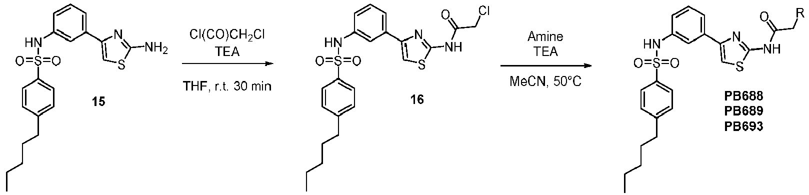

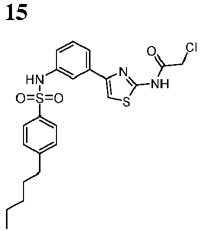

*2-chloro-N-(4-(3-((4-pentylphenyl)sulfonamido)phenyl)thiazol-2-yl)acetamide.* To a stirring solution of amine 15 (900.0 mg, 2.25 mmol, 1eq) in dry THF (12 mL) was added DIEA (1.17 mL, 6.75 mmol, 3eq). Then, the chloroacetyl chloride (0.35 mL, 4.50 mmol, 2 eq) was added dropwise and the mixture was allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with saturated brine solution, dried over MgSO₄ and concentrated under reduced pressure to yield the respective α-chloroacetyl **16** as a brown powder (976.0 mg, 91%). The crude chloroacetyl was directly used without further purification.

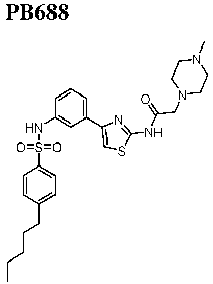

*2-(4-methylpiperazin-1-yl)-N-(4-(3-((4-pentylphenyl)sulfonamido)phenyl)thiazol-2 yl)acetamide.* To a suspension of the chloroalkyl **16** (120.0 mg, 0.25 mmol, 1 eq) in acetonitrile (0.2 M) was added triethylamine (1 eq) and *N*-methylpiperazine (83.0 µL, 0.75 mmol, 3 eq). The reaction mixture was stirred at 50°C until complete consumption of starting material. The volatiles were removed under reduced pressure and crude residue was subjected to silica gel column chromatography (DCM:MeOH 100:0 to 95:5) afforded pure targeted **PB688** as a white powder (70.0 mg, 52%). ¹H NMR (CD₂Cl₂-*d₂*, 400 MHz): δ 0.85 (t, *J* = 7.0 Hz, 3H), 1.31 - 1.2 (m, 4H), 1.60 - 1.53 (m, 2H), 2.39 (s, 3H), 2.61 (t, *J* = 7.9 Hz, 2H), 2.73 - 2.69 (m, 8H), 3.27 (s, 2H), 7.02 (ddd, *J* = 8.0, 2.2, 0.9 Hz, 1H), 7.27 - 7.24 (m, 3H), 7.13 (s, 1H), 7.58 - 7.55 (m, 1H), 7.60 - 7.59 (m, 1H), 7.68 (d, *J* = 8.4 Hz, 2H). ¹³C NMR (CD₂Cl₂-*d₂*, 101 MHz): δ 14.1, 22.8, 31.0, 31.7, 36.1, 45.6, 53.2, 55.0, 61.2, 108.9, 119.3, 120.8, 123.0, 127.6, 129.4 (2C), 130.0 (2C), 135.9, 136.6, 137.7, 149.2, 149.5, 157.7, 168.9. HRMS-ESI (*m*/*z*): [M+H]⁺ calc. for C₂₇H₃₆N₅O₃S₂, 542.2254; Found: 542.2241. HPLC (λ₂₅₄): Purity 95.7 %; *t*_{R}: 5.11 min (method mauro 3).

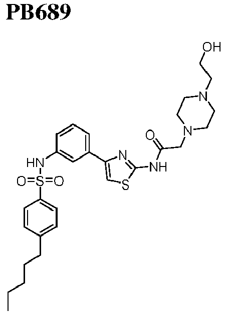

*2-(4-(2-hydroxyethyl)piperazin-1-yl)-N-(4-(3-((4-pentylphenyl)sulfonamido)phenyl)thiazol-2-yl)acetamide.* To a suspension of the chloroalkyl **16** (120.0 mg, 0.25 mmol, 1 eq) in acetonitrile (0.2 M) was added triethylamine (1 eq) and N-hydroxyethylpiperazine (100.0 mg, 0.75 mmol, 3 eq). The reaction mixture was stirred at 50°C until complete consumption of starting material. The volatiles were removed under reduced pressure and crude residue was subjected to silica gel column chromatography (DCM:MeOH 100:0 to 95:5) to afford pure targeted **PB689** as a white powder (51.0 mg, 36%). ¹H NMR (CD₂Cl₂-*d₂*, 400 MHz): δ 0.85 (t, *J* = 7.0 Hz, 3H), 1.31 - 1.24 (m, 4H), 1.60 - 1.53 (m, 2H), 2.61 (t, *J* = 7.9 Hz, 2H), 2.69 (t, *J* = 5.2 Hz, 2H), 2.74 (br s, 8H), 3.27 (s, 2H), 3.66 (t, *J* = 5.3 Hz, 2H), 7.00 (dd, *J* = 8.0, 1.3 Hz, 1H), 7.14 (s, 1H), 7.28 - 7.24 (m, 3H), 7.57 (d, *J* = 7.8 Hz, 1H), 7.61 - 7.60 (m, 1H), 7.68 (d, *J* = 8.4 Hz, 2H). ¹³C NMR (CD₂Cl₂-*d₂*, 101 MHz): δ 14.1. 22.8, 31.0, 31.72, 36.1, 53.1, 57.8 (2C), 59.7, 61.3, 108.9, 119.4, 120.9, 123.1, 127.6, 129.4, 130.0, 135.9, 136.6, 137.6, 149.2, 149.5, 157.7, 168.9. HRMS-ESI (*m*/*z*): [M+H]⁺ calc. for C₂₈H₃₈N₅O₄S₂, 572.2359; Found: 572.2347. HPLC (λ₂₅₄): Purity 95.2 %; *t*_{R}: 5.19 min (method mauro 3).

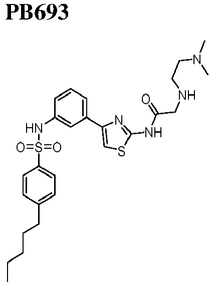

*2-((2-(dimethylamino)ethyl)amino)-N-(4-(3-((4-pentylphenyl)sulfonamido)phenyl)thiazol-2-yl)acetamide.* To a suspension of the chloroalkyl **16** (120.0 mg, 0.25 mmol, 1 eq) in acetonitrile (0.2 M) was added triethylamine (1 eq) and *N,N*-dimethylethane-1,2-diamine (83 µL, 0.75 mmol, 3 eq). The reaction mixture was stirred at 50°C until complete consumption of starting material. The volatiles were removed under reduced pressure and crude residue was subjected to silica gel column chromatography (DCM:MeOH 100:0 to 95:5) afforded pure targeted **PB693** as a light yellow powder (15.0 mg, 12%). ¹H NMR (CD₂Cl₂-*d₂*, 400 MHz): δ 0.82 (t, *J* = 7.0 Hz, 3H), 1.28 - 1.19 (m, 4H), 1.52 - 1.44 (m, 2H), 2.51 (t, *J* = 7.9 Hz, 2H), 2.83 (s, 6H), 2.97 (t, *J* = 5.4 Hz, 2H), 3.13 (t, *J* = 5.4 Hz, 2H), 3.56 (s, 3H), 7.03 (s, 1H), 7.17 - 7.15 (m, 3H), 7.41 - 7.39 (m, 1H), 7.73 (s, 1H), 7.76 (d, *J* = 8.3 Hz, 2H), ¹³C NMR (CD₂Cl₂-*d₂*, 101 MHz): δ 14.1, 22.8, 30.9, 31.7, 36.0, 44.4, 45.0, 52.4, 57.9, 108.8, 118.9, 119.9, 122.3, 127.7, 129.4, 129.9, 135.7, 136.6, 138.2, 148.9, 149.3, 158.2, 170.9, HRMS-ESI (*m*/*z*): [M+H]⁺ calc. for C₂₆H₃₆N₅O₃S₂, 530.2254; Found: 530.2245. HPLC (λ₂₅₄): Purity 97.3 %; *t*_{R}: 4.83 min (method mauro 3).

### Compounds PB624, PB625

Compounds PB624 and PB625 were synthetized based on compounds 15 and 17 according to the following method:

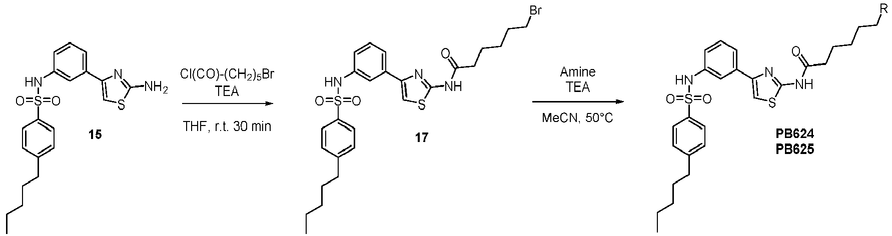

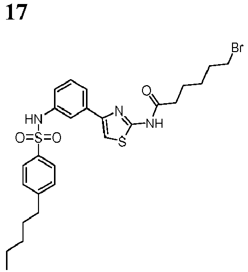

*6-bromo-N-(4-(3-((4-pentylphenyl)sulfonamido)phenyl)thiazol-2-yl)hexanamide.* To a stirring solution of amine **15** (200 mg, 0.5 mmol, 1eq) in dry THF (5 mL) was added DIEA (260.0 µL, 1.50 mmol, 3eq). Then, the 6-bromohexanoyl chloride (152.0 µL, 1.00 mmol, 2eq) was added dropwise to the mixture and allowed to react 30 min at r.t. until complete consumption of the starting material (TLC DCM:EtOAc 70:30). Next, the mixture was quenched with a 4M aqueous solution of sodium hydroxide (10 mL) and stirred for additional 30 min at r.t. The reaction mixture was diluted with 10 mL of water and extracted 3 times with EtOAc (30 mL). Combined organic layer was washed 2 times with aqueous saturated sodium chloride solution, dried over MgSO₄ and concentrated under reduced pressure yielded the respective bromo alkyl **17** as a brown powder (193.2 mg, 64%). The crude bromo alkyl was directly used without further purification for the synthesis of **PB694** and **PB695.**

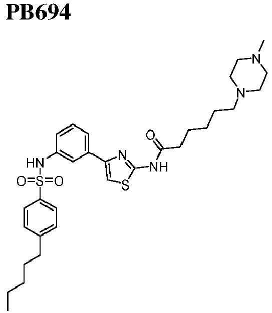

*6-(4-methylpiperazin-1-yl)-N-(4-(3-((4-pentylphenyl)sulfonamido)phenyl)thiazol-2-yl)hexanamide.* To a suspension of the bromoalkyl **17** (60.0 mg, 0.10 mmol, 1 eq) in acetonitrile (0.2 M) was added triethylamine (1 eq) and *N*-methylpiperazine (33 µL, 0.30 mmol, 3 eq). The reaction mixture was stirred at 50°C until complete consumption of starting material. The volatiles were removed under reduced pressure and crude residue was subjected to silica gel column chromatography (DCM:MeOH 100:0 to 95:5) to afford pure targeted **PB694** as a white powder (30.0 mg, 51%). ¹H NMR (CD₃OD-*d*₄, 400 MHz): δ 0.85 (t, *J* = 7.1 Hz, 3H), 1.33 - 1.21 (m, 4H), 1.48 - 1.40 (m, 2H), 1.60 - 1.52 (m, 2H), 1.68 - 1.61 (m, 2H), 1.80 - 1.73 (m, 2H), 2.51 (t, *J* = 7.3 Hz, 2H), 2.63 - 2.59 (m, 5H), 2.70 (t, *J* = 6.7 Hz, 2H), 2.95 (br s, 8H), 6.96 (ddd, *J* = 8.0, 2.2, 0.9 Hz, 1H), 7.21 (t, *J* = 7.9 Hz, 1H), 7.28 - 7.26 (m, 3H), 7.59 - 7.56 (m, 1H), 7.67 (d, *J* = 8.4 Hz, 2H), 7.72 (t, *J* = 1.8 Hz, 1H). ¹³C NMR (CD₃OD-*d*₄, 101 MHz): δ 14.3, 23.4, 25.9, 26.3, 27.4, 31.9, 32.4, 36.3, 36.6, 44.6, 52.2, 54.3, 58.1, 109.0, 119.9, 121.6, 123.3, 128.3, 129.9, 130.3, 136.9, 138.2, 139.50, 149.9, 150.5, 159.3, 173.6, HRMS-ESI (*m*/*z*): [M+H]⁺ calc. for C₃₁H₄₄N₅O₃S₂, 598.2880; Found: 598.2870. HPLC (λ₂₅₄): Purity 97.8 %; *t*_{R}: 4.97 min (method mauro 3).

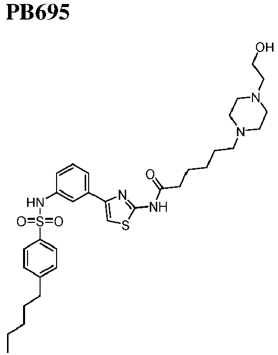

*6-(4-(2-hydroxyethyl)piperazin-1-yl)-N-(4-(3-((4-pentylphenyl)sulfonamido)phenyl)thiazol-2-yl)hexanamide.* To a suspension of the bromoalkyl **17** (60.0 mg, 0.10 mmol, 1 eq) in acetonitrile (0.2 M) was added triethylamine (1 eq) and *N*-hydroxyethylpiperazine (40.0 mg, 0.30 mmol, 3 eq). The reaction mixture was stirred at 50°C until complete consumption of starting material. The volatiles were removed under reduced pressure and crude residue was subjected to silica gel column chromatography (DCM:MeOH 100:0 to 95:5) afforded pure targeted **PB695** as a white powder (39.0 mg, 63%). ¹H NMR (CD₃OD-*d₄*, 400 MHz): δ 0.85 (t, *J* = 7.1 Hz, 3H), 1.33 - 1.21 (m, 4H), 1.48 - 1.40 (m, 2H), 1.60 - 1.52 (m, 2H), 1.70 - 1.63 (m, 2H), 1.81 - 1.73 (m, 2H), 2.51 (t, *J* = 7.3 Hz, 2H), 2.61 (t, *J* = 7.6 Hz, 2H), 2.76 - 2.74 (m, 4H), 2.91 (s, 8H), 3.71 (t, *J* = 5.6 Hz, 2H), 6.97 (ddd, *J* = 8.0, 2.2, 0.9 Hz, 1H), 7.21 (t, *J* = 7.9 Hz, 1H), 7.28 - 7.26 (m, 3H), 7.59 - 7.57 (m, 1H), 7.67 (d, *J* = 8.4 Hz, 2H), 7.71 (t, *J* = 1.8 Hz, 1H). ¹³C NMR (CD₃OD-*d*₄, 101 MHz): δ 14.3, 23.4, 25.9, 26.1, 27.5, 31.9, 32.4, 36.3, 36.6, 52.7, 52.7, 58.4, 58.9, 60.3, 109.0, 119.9, 121.6, 123.3, 128.3, 129.9, 130.3, 136.9, 138.21, 139.5, 149.9, 150.5, 159.4, 173.6. HRMS-ESI (*m*/*z*): [M+H]⁺ calc. for C₃₂H₄₆N₅O₄S₂, 628.2986; Found: 628.2976. HPLC (λ₂₅₄): Purity > 99 %; *t*_{R}: 4.95 min (method mauro 3).

### Compounds PB703, PB704, PB707, PB705 and PB706

These compounds were synthetized according to the following methods:

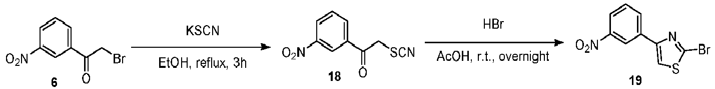

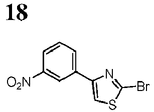

*2-bromo-4-(3-nitrophenyl)thiazole.* To a solution of α-bromo-acetophenone **6** (4.88 g, 20.0 mmol, 1 eq) in ethanol was added potassium isothiocyanate (3.88 g, 40.0 mmol, 2 eq). The reaction mixture was then heated to 80°C for 3 hours and left to cool to room temperature. The precipitate was filtered and washed with cold ethanol and diethyl ether. The obtained solid **(18)** was resuspended in glacial acetic acid (10 mL) and HBr/AcOH (10 mL, 33% HBr) was added dropwise over a period of 30 minutes. After the addition, mixture was allowed to react overnight. The reaction was diluted with AcOH (20 mL), the solids were filtered, washed with AcOH (10 mL) and diethyl ether (40 mL). Finally, the obtained white powder was dissolved in EtOAc (50 mL) and washed three times with saturated NaHCO₃ solution (20 mL). The organic phase was dried over MgSO₄ and concentrated under reduced pressure to afford the 2-bromothiazole **19** in 72% yield (4.10 g) as a white powder. ¹H NMR (CD₂Cl₂-*d*₂, 400 MHz): δ 7.75 (t, *J* = 8.0 Hz, 1H), 8.21 (ddd, *J* = 8.2, 2.2, 0.8 Hz, 1H), 8.37 - 8.35 (m, 1H), 8.49 (s, 1H), 8.68 (t, *J* = 1.9 Hz, 1H).

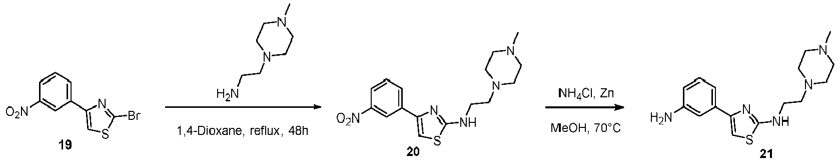

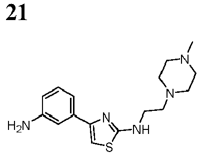

*4-(3-aminophenyl)-N-(2-(4-methylpiperazin-1-yl)ethyl)thiazol-2-amine.* To a solution of 2-bromo-4-(3-nitrophenyl)thiazole **19** (1.15 g, 4.00 mmol, 1 eq) in anhydrous dioxane (0.2 M) was added 2-(4-methylpiperazin-1-yl)ethan-1-amine (2.30 g, 16.00 mmol, 4.0 eq). The reaction mixture was heated to reflux during 48 hours. The solvent was removed under reduced pressure, and the crude material **(20)** was redissolved in methanol (20 mL) and treated with Zn powder (1.56 g, 24.00 mmol, 6 eq) and ammonium chloride (1.07 g, 20.00 mmol, 5 eq) during one additional hour. The reaction was diluted with methanol (30mL) and filtered through a pad of celite. The volatiles were removed in vacuo and the crude product was was subjected to silica gel chromatography (DCM:MeOH 100:0 to 90:10) to afford the aniline **21** in 65% yield (824.0 mg) as a yellow solid. ¹H NMR (DMSO-*d*₆, 400 MHz): δ, 2.15 (s, 3H). 2.33 (br s, 4H), 2.44 (br s, 4H), 2.53 - 2.50 (m, 2H), 3.37 (q, *J* = 5.9 Hz, 2H), 5.04 (s, 2H), 6.47 (d, *J* = 7.5 Hz, 1H), 6.80 (s, 1H), 7.01 - 6.94 (m, 2H), 7.05 (s, 1H), 7.41 (t, *J* = 5.3 Hz, 1H).

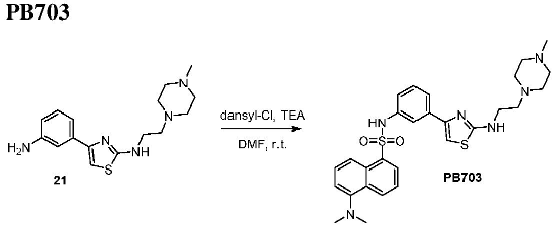

*5-(dimethylamino)-N-(3-(2-((2-(4-methylpiperazin-1-yl)ethyl)amino)thiazol-4-yl)phenyl)naphthalene-1-sulfonamide.* To a solution of aniline **21** (85.0 mg, 0.22 mmol, 1 eq) under argon in anhydrous DMF (0.2 M) were added anhydrous triethylamine (1.6 eq) and dansyl chloride (74.0 mg, 0.27 mmol, 1.1 eq). The reaction mixture was allowed to react at r.t. until complete conversion of the starting material. DMF was removed under reduced pressure and the crude material was purified by silica gel column. chromatography (DCM:MeOH, 100:0 to 95:5) afforded the titled sulfonamide **PB703** as a yellow fluorescent powder (26.1 mg, 20%). ¹H NMR (CD₂Cl₂-*d*₂, 400 MHz): δ 2.68 (s, 3H), 2.93 - 2.72 (m, 12H), 3.06 (s, 4H), 3.53 (t, *J* = 6.2 Hz, 2H), 6.64 (s, 1H), 6.84 (dd, *J* = 8.0, 1.4 Hz, 1H), 7.07 (t, *J* = 7.9 Hz, 1H), 7.24 (d, *J* = 7.4 Hz, 1H), 7.35 (d, *J* = 7.9 Hz, 1H), 7.47 (dd, *J* = 8.4, 7.5 Hz, 1H), 7.54 (t, *J* = 1.7 Hz, 1H), 7.60 (dd, *J* = 8.5, 7.7 Hz, 1H), 8.22 (dd, *J* = 7.3, 1.1 Hz, 1H), 8.44 (d, *J* = 8.7 Hz, 1H), 8.48 (d, *J* = 8.5 Hz, 1H). ¹³C NMR (CD₂Cl₂-*d*₂, 101 MHz): δ 42.6, 44.2, 45.8, 51.8, 54.9, 57.5, 102.4, 116.4, 119.3, 120.3, 120.5, 122.8, 124.1, 129.3, 130.1, 131.0, 131.1, 131.4, 131.6, 136.1, 137.2, 139.1, 151.4, 153.2, 170.9. HRMS-ESI (*m*/*z*): [M+H]⁺ calc. for C₂₈H₃₅N₆O₂S₂, 551.2257; Found: 551.2225. HPLC (λ₂₅₄): Purity 99.1 %; *t*_{R}: 4.32 min (method mauro 7).

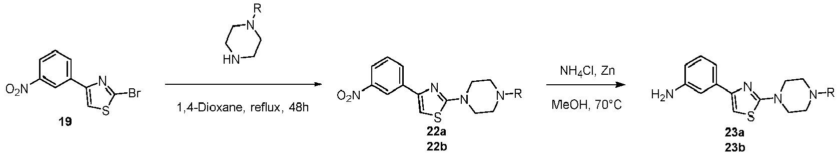

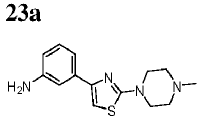

*3-(2-(4-methylpiperazin-1-yl)thiazol-4-yl)aniline.* To a solution of 2-bromo-4-(3-nitrophenyl)thiazole **19** (800.0 mg, 2.80 mmol, 1 eq) in anhydrous dioxane (0.2 M) was added *N*-methylpiperazine (1.12 g, 16.00 mmol, 4.0 eq). The reaction mixture was heated to reflux during 48 hours. The solvent was removed under reduced pressure, and the crude material **(22a)** was redissolved in methanol (20 mL) and treated with Zn powder (1.56 g, 24.00 mmol, 6 eq) and ammonium chloride (1.07 g, 20.00 mmol, 5 eq) during one additional hour. The reaction was diluted with methanol (30 mL) and filtered through a pad of celite. The volatiles were removed in vacuo and the crude product was was subjected to silica gel chromatography (DCM:MeOH 100:0 to 90:10) to afford the aniline **23a** in 41% yield (314.0 mg) as a yellow solid. ¹H NMR (DMSO-*d*₆, 400 MHz): δ 2.23 (s, 3H). 2.44 (br s, 4H), 3.43 (br s, 4H), 5.06 (s, 2H), 6.48 (d, *J* = 6.2 Hz, 1H), 7.09 - 6.96 (m, 4H).

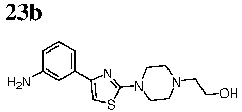

*2-(4-(4-(3-aminophenyl)thiazol-2-yl)piperazin-1-yl)ethan-1-ol.* To a solution of 2-bromo-4-(3-nitrophenyl)thiazole **19** (1.15 g, 4.00 mmol, 1 eq) in anhydrous dioxane (0.2 M) were added *N-*hydroxyethyl piperazine (2.08 g, 16.00 mmol, 4.0 eq). The reaction mixture was heated to reflux during 48 hours. The solvent was removed under reduced pressure, and the crude material **(22b)** was redissolved in methanol (20 mL) and treated with Zn powder (1.56 g, 24.00 mmol, 6 eq) and ammonium chloride (1.07 g, 20.00 mmol, 5 eq) during 1 hour. Next, the reaction was diluted with methanol, filtered through a pad of celite. The solvent was removed in vacuo and the crude product purified by silica gel column chromatography (DCM:MeOH, 100:0 to 95:5) to afford the aniline product **23b** as a yellowish oil (250 mg, 20% yield). ¹H NMR (DMSO-*d*₆, 400 MHz) δ 2.45 (t, *J* = 6.2 Hz, 3H). 2.57 - 2.54 (m, 4H), 3.44 - 3.42 (m, 4H), 3.54 (q, *J* = 6.0 Hz, 2H), 4.45 (t, *J* = 5.4 Hz, 1H), 6.49 - 6.46 (m, 1H), 5.06 (s, 2H), 7.02 - 6.96 (m, 2H), 7.03 (s, 1H), 7.08 (s, 1H)

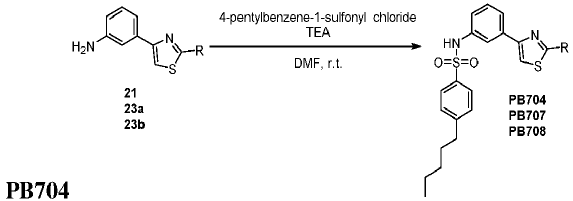

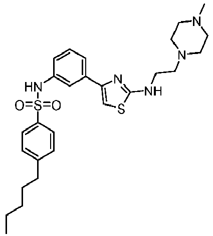

*N-(3-(2-((2-(4-methylpiperazin-1-yl)ethyl)amino)thiazol-4-yl)phenyl)-4-pentylbenzenesulfonamide.* To a solution of aniline **21** (85.0 mg, 0.22 mmol, 1 eq) under argon in anhydrous DMF (0.2 M) were added anhydrous triethylamine (1.6 eq) and 4-pentylbenzene-1-sulfonyl chloride (66.0 mg, 0.27 mmol, 1.1 eq). The reaction mixture was allowed to react at r.t. until complete conversion of the starting material. DMF was removed under reduced pressure and the crude material was purified by silica gel column chromatography (DCM:MeOH, 100:0 to 95:5) to afford the titled sulfonamide **PB704** as a white powder (58.1 mg, 41%). ¹H NMR (CD₃OD-*d*₄, 400 MHz): δ 0.86 (t, *J* = 7.1 Hz, 3H), 1.35 - 1.22 (m, 4H), 1.62 - 1.54 (m, 2H), 2.28 (s, 3H), 2.53 (s, 8H), 2.64 - 2.60 (m, 2H), 2.67 (t, *J* = 6.6 Hz, 2H), 3.49 (t, *J* = 6.6 Hz, 2H), 6.71 (s, 1H), 6.98 (dd, *J* = 8.0, 1.3 Hz, 1H), 7.17 (t, *J* = 7.9 Hz, 1H), 7.27 (d, *J* = 8.3 Hz, 2H), 7.44 (d, *J* = 7.8 Hz, 1H), 7.51 (t, *J* = 1.8 Hz, 1H), 7.68 (d, *J* = 8.3 Hz, 2H). ¹³C NMR (CD₃OD-*d*₄, 101 MHz): δ 14.3, 23.5, 31.9, 32.5, 36.6, 43.0, 46.0, 53.7, 55.7, 57.9, 102.1, 119.9, 121.3, 122.8, 128.3, 129.9, 130.0, 137.3, 138.8, 140.3, 149.5, 151.7, 171.0. HRMS-ESI (*m*/*z*): [M+H]⁺ calc. for C₂₇H₃₈N₅O₂S₂, 528.2461; Found: 528.2430. HPLC (λ₂₈₀): Purity 98.2 %; *t*_{R}: 5.26 min (method mauro 3).

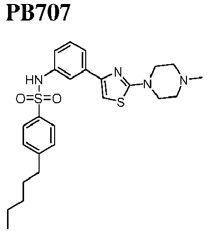

*N-(3-(2-(4-methylpiperazin-1-yl)thiazol-4-yl)phenyl)-4-pentylbenzenesulfonamide.* To a solution of aniline **23a** (60.0 mg, 0.22 mmol, 1 eq) under argon in anhydrous DMF (0.2 M) were added anhydrous triethylamine (1.6 eq) and 4-pentylbenzene-1-sulfonyl chloride (66.0 mg, 0.27 mmol, 1.1 eq). The reaction mixture was allowed to react at r.t. until complete conversion of the starting material. DMF was removed under reduced pressure and the crude material was purified by silica gel column chromatography (DCM:MeOH, 100:0 to 95:5) to afford the titled sulfonamide **PB707** as a white powder (75.2 mg, 71%). ¹H NMR (CD₂Cl₂-*d*₂/CD₃OD-*d*₄, 400 MHz) δ 0.85 (t, *J* = 7.0 Hz, 3H), 1.31 - 1.24 (m, 4H), 1.61 - 1.53 (m, 2H), 2.35 (s, 3H), 2.63 - 2.56 (m, 6H), 3.55 - 3.53 (m, 4H), 6.83 (s, 1H), 7.02 (ddd, *J* = 8.0, 2.1, 0.9 Hz, 1H), 7.20 (t, *J* = 7.9 Hz, 1H), 7.25 (d, *J* = 8.4 Hz, 2H), 7.51 -7.49 (m, 1H), 7.54-7.53 (m, 1H), 7.68 (d, *J* = 8.4 Hz, 2H). ¹³C NMR (CD₂Cl₂-d₂/CD₃OD-*d*₄, 101 MHz) δ 14.2, 23.2, 31.5, 32.1, 36.4, 46.2, 48.7, 54.8, 103.3, 119.5, 120.9, 122.9, 127.9, 129.7, 129.9, 136.9, 137.8, 138.8, 149.5, 151.9, 172.1. HRMS-ESI (*m*/*z*): [M+H]⁺ calc. for C₂₅H₃₃N₄O₂S₂, 485.2039; Found: 485.2008. HPLC (λ₂₈₀): Purity > 99.9 %; *t*_{R}: 5.36 min (method mauro 3).

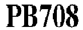

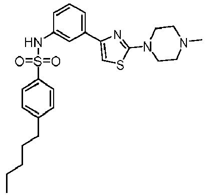

*N-(3-(2-(4-(2-hydroxyethyl)piperazin-1-yl)thiazol-4-yl)phenyl)-4-pentylbenzenesulfonamide.* To a solution of aniline **23b** (85.0 mg, 0.20 mmol, 1 eq) under argon in anhydrous DMF (0.2 M) were added anhydrous triethylamine (1.6 eq) and 4-pentylbenzene-1-sulfonyl chloride (53.0 mg, 0.22 mmol, 1.1 eq). The reaction mixture was allowed to react at r.t. until complete conversion of the starting material. DMF was removed under reduced pressure and the crude material was purified by silica gel column chromatography (DCM:MeOH, 100:0 to 95:5) to afford the titled sulfonamide **PB708** as a white powder (19.1 mg, 20%). ¹H 1H NMR (CD₃OD-*d*₄, 400 MHz) δ 0.86 (t, *J* = 7.1 Hz, 3H), 1.30 - 1.26 (m, 4H), 1.61 - 1.54 (m, 2H), 2.64 - 2.60 (m, 4H), 2.70 - 2.68 (m, 4H), 3.56 - 3.53 (m, 4H), 3.73 (t, *J* = 5.8 Hz, 2H), 6.88 (s, 1H), 7.01 (ddd, *J* = 8.0, 2.1, 0.8 Hz 1H), 7.19 (t, *J* = 7.9 Hz, 1H), 7.28 (d, *J* = 8.3 Hz, 2H), 7.50 (d, *J* = 7.9 Hz, 1H), 7.54 (t, *J* = 1.8 Hz, 1H), 7.68 (d, *J* = 8.4 Hz, 2H). ¹³C NMR (CD₃OD-*d₄*, 101 MHz) δ 14.3, 23.5, 31.9, 32.5, 36.6, 53.7, 59.8, 61.2, 103.5, 119.9, 121.3, 123.2, 128.4, 129.9, 130.2, 137.4, 138.3, 139.3, 149.8, 152.3, 172.5. HRMS-ESI (*m*/*z*): [M+H]⁺ calc. for C₂₆H₃₅N₄O₃S₂⁺, 515.2145; Found: 515.2113. HPLC (λ₂₈₀): Purity > 99.9 %; *t*_{R}: 5.30 min (method mauro 3).

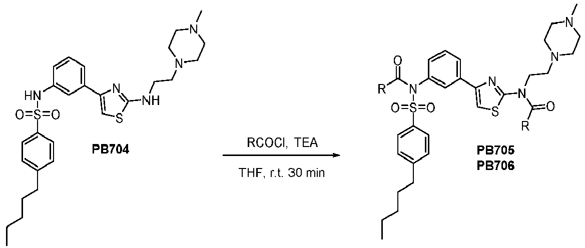

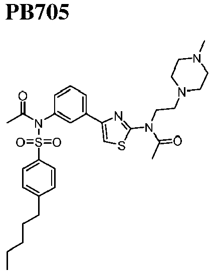

*N-(2-(4-methylpiperazin-1-yl)ethyl)-N-(4-(3-(N-((4-pentylphenyl)sulfonyl)acetamido)phenyl)thiazol-2-yl)acetamide.* To a stirring solution of **PB704** (105 mg, 0.2 mmol, leq) in dry THF (5 mL) was added TEA (104.0 µL, 0.60 mmol, 3eq). Then acetyl chloride (28.0 µL, 0.4 mmol, 2eq) was added dropwise to the mixture and allowed to react 30 min at r.t. until complete consumption of the starting material. Next, the reaction mixture was diluted with 10 mL of water and extracted 3 times with EtOAc (30 mL). Combined organic layer was washed 2 times with aqueous saturated sodium chloride solution, dried over MgSO₄ and concentrated under reduced pressure. The crude product was purified by silica gel column chromatography (DCM:MeOH, 100:0 to 95:5) to afford the titled sulfonamide **PB705** as a white powder (11.2 mg, 9%).

¹H NMR (400 MHz, CD₃OD-*d*₄) δ 8.06 (d, J = 8.2 Hz, 1H), 7.93 (d, J = 8.4 Hz, 3H), 7.55 (t, J = 7.9 Hz, 2H), 7.45 (d, J = 8.4 Hz, 2H), 7.26 (dd, J = 7.8, 1.2 Hz, 1H), 4.44 (t, J = 6.8 Hz, 2H), 2.80 - 2.48 (m, 15H), 2.28 (s, 3H), 1.92 (s, 3H), 1.72 - 1.64 (m, 2H), 1.41 - 1.32 (m, 4H), 0.92 (t, J = 6.9 Hz, 3H). ¹³C NMR (101 MHz, CD₃OD-*d*₄) δ 172.48, 171.99, 160.57, 151.50, 148.55, 138.78, 137.96, 137.74, 131.19, 130.32, 130.18, 130.00, 128.83, 128.14, 111.76, 56.59, 55.79, 54.11, 46.79, 45.93, 36.86, 32.62, 32.02, 25.15, 23.54, 22.71, 14.34. HPLC (λ₂₈₀): Purity > 99.9 %; *t*_{R}: 5.57 min (method mauro 3).

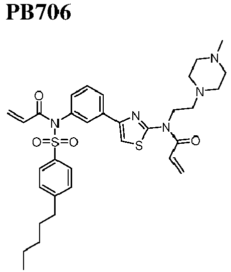

*N-(2-(4-methylpiperazin-1-yl)ethyl)-N-(4-(3-(N-((4-pentylphenyl)sulfonyl)acrylamido)phenyl)thiazol-2-yl)acrylamide.* To a stirring solution of **PB704** (105 mg, 0.2 mmol, 1eq) in dry THF (5 mL) was added TEA (104.0 µL, 0.60 mmol, 3eq). Then acryloyl chloride (35.0 µL, 0.4 mmol, 2eq) was added dropwise to the mixture and allowed to react 30 min at r.t. until complete consumption of the starting material. Next, the reaction mixture was diluted with 10 mL of water and extracted 3 times with EtOAc (30 mL). Combined organic layer was washed 2 times with aqueous saturated sodium chloride solution, dried over MgSO₄ and concentrated under reduced pressure. The crude product was purified by silica gel column chromatography (DCM:MeOH, 100:0 to 95:5) to afford the titled sulfonamide **PB706** as a white powder (8.0 mg, 6.3%). ¹H NMR (400 MHz, CD₃OD-*d*₄) δ 8.07 (d, J = 8.1 Hz, 1H), 7.93 (d, J = 8.4 Hz, 2H), 7.87 (t, J = 1.7 Hz, 1H), 7.60 (s, 1H), 7.56 (t, J = 7.9 Hz, 1H), 7.45 (d, J = 8.4 Hz, 2H), 7.20 (dd, J = 7.8, 1.2 Hz, 1H), 7.02 (dd, J = 16.6, 10.5 Hz, 1H), 6.54 (dd, J = 16.6, 1.6 Hz, 1H), 6.33 (dd, J = 16.8, 1.5 Hz, 1H), 5.98 (ddd, J = 23.6, 12.7, 6.0 Hz, 2H), 4.54 (t, J = 7.1 Hz, 2H), 2.65 (ddd, J = 140.1, 52.2, 13.6 Hz, 11H), 1.80 - 1.60 (m, 2H), 1.37 (dd, J = 7.2, 3.7 Hz, 4H), 0.92 (t, J = 6.9 Hz, 3H). ¹³C NMR (101 MHz, CD₃OD-*d*₄) δ 167.19, 166.39, 160.85, 151.62, 148.68, 137.87, 137.64, 137.45, 132.07, 131.85, 131.22, 130.59, 130.43, 130.01, 129.78, 129.15, 128.52, 128.20, 112.43, 57.06, 55.76, 54.01, 45.95, 45.85, 36.88, 32.64, 31.98, 23.53, 14.35.

HPLC (λ₂₈₀): Purity = 98.8 %; *t*_{R}: 5.80 min (method mauro 3).

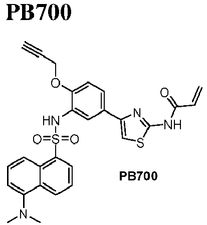

*N-(4-(3-((5-(dimethylamino)naphthalene)-1-sulfonamido)-4-(prop-2-yn-1-yloxy)phenyl)thiazol-2-yl)acrylamide.* To a solution of N-(5-(2-aminothiazol-4-yl)-2-(prop-2-yn-1-yloxy)phenyl)-5-(dimethylamino)naphthalene-1-sulfonamide (45 mg, 0.1 mmol, 1 equiv) in anhydrous THF (0.05 M) were added anhydrous triethylamine (44 µL, 0.3 mmol, 3.0 equiv) and acryloyl chloride (13 µL, 0.2 mmol, 2.0 equiv). The reaction mixture was allowed to react at rt until complete conversion of the starting material. 4M NaOH (5mL) was added and the reaction was kept for 10 minutes. The reaction was diluted with water and extracted with ethyl acetate (3 x 10 mL), the organic phase was washed with brine, dried over MgSO₄ and concentrated in vacuo. The crude material was purified by silica gel column chromatography (DCM:EA, 100:0 to 80:20) to afford the desired compound (12 mg, 22%). ¹H (400 MHz, DMSO-*d*₆) δ 12.47 (s, 1H), 9.84 (s, 1H), 8.41 (dd, J = 10.6, 9.2 Hz, 2H), 8.05 (dd, J = 7.3, 0.9 Hz, 1H), 7.85 (d, J = 2.1 Hz, 1H), 7.59 - 7.50 (m, 3H), 7.41 (s, 1H), 7.25 (d, J = 7.4 Hz, 1H), 6.95 (d, J = 8.7 Hz, 1H), 6.57 (dd, J = 17.1, 10.2 Hz, 1H), 6.40 (dd, J = 17.1, 1.7 Hz, 1H), 5.91 (dd, J = 10.2, 1.7 Hz, 1H), 4.20 (d, J = 2.1 Hz, 2H), 3.43 (t, J = 2.3 Hz, 1H), 2.81 (s, 6H). ¹³C NMR (101 MHz, DMSO-*d*₆) δ 163.0, 157.8, 151.2, 150.1, 148.3, 136.0, 129.7, 129.5, 129.4, 129.3, 129.0, 128.6, 127.6, 125.9, 123.6, 123.3, 123.0, 122.2, 119.5, 115.1, 113.3, 107.4, 78.5, 78.4, 55.6, 45.1. HRMS-ESI (*m*/*z*): [M+H]⁺ calc. for C₂₇H₂₅N₄O₄S₂⁺, 533.1312; Found: 533.1301.
HPLC (λ₂₈₀): Purity 96.5 %; *t*_{R}: 6.51 min (method mauro 3).

### Therapeutic use

The endoplasmic reticulum (ER) is the principal organelle responsible for multiple cellular functions including protein folding and maturation and the maintenance of cellular homeostasis. ER stress is activated by a variety of factors and triggers the unfolded protein response (UPR), which restores homeostasis or activates cell death. Multiple studies have clarified the link between ER stress and cancer, and particularly the involvement of the UPR. The UPR seems to adjust the paradoxical microenvironment of cancer and, as such, is one of resistance mechanisms against cancer therapy.

### Unfolded Protein Response Regulator GRP78/BiP/HSPA5

Endoplasmic reticulum (ER) luminal glucose-regulated protein 78 (GRP78) functions as a unfolded protein response (UPR) signalling regulator by binding to and maintaining the ER stress sensors (PRKR-like ER kinase (PERK), activating transcription factor 6 (ATF6) and inositol-requiring enzyme 1 (IRE1)) in inactive forms. It also binds to ER-associated caspase 7 and caspase 12 and suppresses their activation. Upon ER stress, GRP78 is titrated away through binding to misfolded proteins. This triggers the UPR, as exemplified by the dimerization of PERK and IRE1, and triggers the activation of their downstream signalling pathways, which leads to arrest of translation and ER-associated protein degradation (ERAD). The UPR also generates the active nuclear form of ATF6 (ATF6(N)), as well as ATF4 and the spliced form of X box-binding protein 1 (XBP1s), which function together with other transcriptional factors, including YY1, nuclear transcription factor Y (NFY), TFII-I and chromatin modifiers, to activate the ER stress response element (ERSE) present in the promoters of ER stress-responsive genes. A major UPR response is to induce the transcription of ER folding proteins, such as the GRPs, to increase the ER protein folding capacity, and to induce the transcription of the mitochondrial chaperone GRP75. Stressed cells actively promote the relocalization of GRP78 and GRP94 to the plasma membrane and, in some instances, their secretion; these cells also generate a cytosolic isoform of GRP78 (GRP78va) through alternative splicing. Nonetheless, UPR can also induce transcription of the pro-apoptotic transcription factor CHOP; and following release from GRP78, caspase 7 and caspase 12 are activated, thereby triggering apoptosis. Thus, the UPR regulates the balance between survival and cell death in stressed cells, and the upregulation of the GRPs represents a major adaptive, protective action that occurs through the maintenance of cellular homeostasis. eIF2α, eukaryotic translation initiation factor 2α; P, phosphorylation (Lee, A. Glucose-regulated proteins in cancer: molecular mechanisms and therapeutic potential. Nat Rev Cancer 14, 263-276 (2014). https://doi.org/10.1038/nrc3701).

### CHOP (C/-EBP homologous protein)

CHOP is a DNA damage-inducible transcript 3, also known as C/EBP homologous protein, and is a pro-apoptotic transcription factor that is encoded by the DDIT3 gene

CHOP is a key player of ER stress and is an initiating factor of ER stress-related cell death. Hence, CHOP is commonly used as a marker of endoplasmic reticulum (ER) stress (Cerezo et al., Compounds Triggering ER Stress Exert Anti-Melanoma Effects and Overcome BRAF Inhibitor Resistance, Cancer Cell (2016), http://dx.doi.org/10.1016/j.ccell.2016.04.013)

The inventors advantageously demonstrate that compounds of the present invention target the ER stress axis to induce specific cancer cell death by concomitant induction of autophagy and apoptosis.

Hence, in an embodiment, the present invention pertains to a benzene sulfonamide thiazole compound of formula (I): 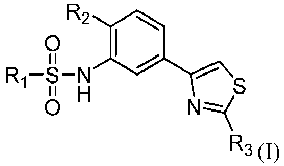 wherein
R₁ is a 5-(dimethylamino)naphthalene group or a 4-pentylphenyl group
R₂ is selected from H, halogen, OR₆
R₃ is selected from C₁-C₈ alkyl, NR₄R₅
R₄ is selected from H, alkyl optionally substituted with a nitrogen-containing heterocycle
Rs is selected from H, alkyl optionally substituted with a nitrogen-containing heterocycle, COR₇
R₄ and R₅ can alternatively form together a nitrogen-containing heterocycle optionally substituted
R₆ is selected from C₁-C₈ alkyl, C₁-C₈ alkyl-O-C₁-C₈ alkyl,
R₇ is selected from
   - C₁-C₈ alkyl optionally substituted with
      ∘ a nitrogen-containing heterocycle optionally substituted
      ∘ O-alkyl, NHCO-alkyl, NHCOO-alkyl, COO-alkyl, NH-alkyl, NH-alkyl-OH, NHP(O)(O-alkyl)₂, NH-alkyl-SO₃H, NH-alkyl-N(alkyl)₂
      ∘ Alkenyl.
for inducing an early endoplasmic reticulum stress.

These compounds also exhibit strong efficacy in melanoma cells as well as in NCI-60 Human Tumor Cell Lines.

Hence, the benzene sulfonamide thiazole compound according to the present invention are used for the treatment of cancer in a patient.

The present disclosure thus provides a method for treating cancer comprising administering, to a patient in need thereof, a therapeutically effective amount of the benzene sulfonamide thiazole as defined above.

The present disclosure also relates to the use of benzene sulfonamide thiazole as defined above for the treatment of cancer.

The present disclosure also relates to the use of benzene sulfonamide thiazole as defined above in the manufacture of a medicament for the treatment of cancer.

The present disclosure also concerns a pharmaceutical composition for treatment of cancer, comprising a benzene sulfonamide thiazole as defined above.

The terms **"Subject"** and **"Patient"** refer to a human or an animal suffering from cancer. Preferably, the patient is human.

For the avoidance of doubt, references herein to **"treatment"** include references to curative, palliative and prophylactic treatment. A **"treatment"** aims at reversing, alleviating, inhibiting the progress of, or preventing the disorder or condition to which such term applies, or reversing, alleviating, inhibiting the progress of, or preventing one or more symptoms of the disorder or condition to which such term applies.

The term **"cancer"** herein refers to the physiological condition in subjects that is characterized by unregulated or dysregulated cell growth or death. The term "cancer" includes solid tumors and liquid cancer.

In an embodiment, the invention relates to a benzene sulfonamide thiazole compound of formula (I): 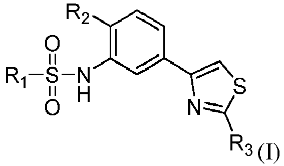 wherein
R₁ is a 5-(dimethylamino)naphthalene group or a 4-pentylphenyl group
R₂ is selected from H, halogen, OR₆
R₃ is selected from C₁-C₈ alkyl, NR₄R₅
R₄ is selected from H, alkyl optionally substituted with a nitrogen-containing heterocycle
R₅ is selected from H, alkyl optionally substituted with a nitrogen-containing heterocycle, COR₇
R₄ and R₅ can alternatively form together a nitrogen-containing heterocycle optionally substituted
R₆ is selected from C₁-C₈ alkyl, C₁-C₈ alkyl-O-C₁-C₈ alkyl,
R₇ is selected from
   - C₁-C₈ alkyl optionally substituted with
      ∘ a nitrogen-containing heterocycle optionally substituted
      ∘ O-alkyl, NHCO-alkyl, NHCOO-alkyl, COO-alkyl, NH-alkyl, NH-alkyl-OH, NHP(O)(O-alkyl)₂, NH-alkyl-SO₃H, NH-alkyl-N(alkyl)₂
      ∘ Alkenyl
for use for the treatment of cancer.

All the substituents previously described are covered in this embodiment.

In an embodiment, benzene sulfonamide thiazole compounds for use for the treatment of cancer are selected among,
- *N-(4-(3-((5-(dimethylamino)naphthalene)-1-sulfonamido)-4-methoxyphenyl)thiazol-2-yl)acetamide*
- *N-(4-(3-((5-(dimethylamino)naphthalene)-1-sulfonamido)-4-fluorophenyl)thiazol-2-yl)-2-(4-(2-hydroxyethyl)piperazin-1-yl)acetamide*
- *N-(4-(3-((5-(dimethylamino)naphthalene)-1-sulfonamido)-4-methoxyphenyl)thiazol-2-yl)-2-(4-(2-hydroxyethyl)piperazin-1-yl)acetamide*
- *N-(4-(3-((5-(dimethylamino)naphthalene)-1-sulfonamido)-4-(2-methoxyethoxy)phenyl)thiazol-2-yl)acetamide*
- *N-(4-(3-((5-(dimethylamino)naphthalene)-1-sulfonamido)-4-(2-methoxyethoxy)phenyl)thiazol-2-yl)-2-(4-(2-hydroxyethyl)piperazin-1-yl)acetamide*
- *N-(4-(3-((5-(dimethylamino)naphthalene)-1-sulfonamido)phenyl)thiazol-2-yl)-2-(4-methylpiperazin-1-yl)acetamide*
- *N-(4-(3-((5-(dimethylamino)naphthalene)-1-sulfonamido)phenyl)thiazol-2-yl)-2-morpholinoacetamide*
- *N-(4-(3-((5-(dimethylamino)naphthalene)-1-sulfonamido)phenyl)thiazol-2-yl)-2-(4-(2-hydroxyethyl)piperazin-1-yl)acetamid*
- *N-(4-(3-((5-(dimethylamino)naphthalene)-1-sulfonamido)phenyl)thiazol-2-yl)-2-((3-morpholinopropyl)amino)acetamide*
- *Diethyl-(2-((4-(3-((5-(dimethylamino)naphthalene)-1-sulfonamido)phenyl)thiazol-2-yl)amino)-2-oxoethyl)phosphoramidate*
- *2-((2-((4-(3-((5-(dimethylamino)naphthalene)-1-sulfonamido)phenyl)thiazol-2-yl)amino)-2-oxoethyl)amino)ethane-1-sulfonic*
- *N-(4-(3-((5-(dimethylamino)naphthalene)-1-sulfonamido)phenyl)thiazol-2-yl)-6-(4-methylpiperazin-1-yl)hexanamide*
- *N-(4-(3-((5-(dimethylamino)naphthalene)-1-sulfonamido)phenyl)thiazol-2-yl)-6-(4-(2-hydroxyethyl)piperazin-1-yl)hexanamide*
- *N-(4-(3-((5-(dimethylamino)naphthalene)-1-sulfonamido)-4-(prop-2-yn-1-yloxy)phenyl)thiazol-2- yl)acrylamide*
- *5-(dimethylamino)-N-(3-(2-((2-(4-methylpiperazin-1-yl)ethyl)amino)thiazol-4-yl)phenyl)naphthalene-1-sulfonamide*

In another embodiment, benzene sulfonamide thiazole compounds for use for the treatment of cancer are selected among:
- *2-(4-methylpiperazin-1-yl)-N-(4-(3-((4-pentylphenyl)sulfonamido)phenyl)thiazol-2 yl)acetamide.*
- *2-(4-(2-hydroxyethyl)piperazin-1-yl)-N-(4-(3-((4-pentylphenyl)sulfonamido)phenyl)thiazol-2-yl)acetamide.*
- *N-(5-(2-aminothiazol-4-yl)-2-methoxyphenyl)-4-pentylbenzenesulfonamide*
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide*
- *2-((2-(dimethylamino)ethyl)amino)-N-(4-(3-((4-pentylphenyl)sulfonamido)phenyl)thiazol-2-yl)acetamide.*
- *6-(4-methylpiperazin-1-yl)-N-(4-(3-((4-pentylphenyl)sulfonamido)phenyl)thiazol-2-yl)hexanamide.*
- *6-(4-(2-hydroxyethyl)piperazin-1-yl)-N-(4-(3-((4-pentylphenyl)sulfonamido)phenyl)thiazol-2-yl)hexanamide.*
- *2-(4-methylpiperazin-1-yl)-N-(4-(4-(4-methylpiperazin-1-yl)-3-((4-pentylphenyl)sulfonamido)phenyl)thiazol-2-yl)acetamide*
- *N-(3-(2-((2-(4-methylpiperazin-1-yl)ethyl)amino)thiazol-4-yl)phenyl)-4-pentylbenzenesulfonamide.*
- *N-(2-(4-methylpiperazin-1-yl)ethyl)-N-(4-(3-(N-((4-pentylphenyl)sulfonyl)acetamido)phenyl)thiazol-2-yl)acetamide.*
- *N-(2-(4-methylpiperazin-1-yl)ethyl)-N-(4-(3-(N-((4-pentylphenyl)sulfonyl)acrylamido)phenyl)thiazol-2-yl)acrylamide.*
- *N-(3-(2-(4-methylpiperazin-1-yl)thiazol-4-yl)phenyl)-4-pentylbenzenesulfonamide.*
- *N-(3-(2-(4-(2-hydroxyethyl)piperazin-1-yl)thiazol-4-yl)phenyl)-4-pentylbenzenesulfonamide.*

In a preferred embodiment, benzene sulfonamide thiazole compounds for use for the treatment of cancer are selected among:
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide*
- *6-(4-methylpiperazin-1-yl)-N-(4-(3-((4-pentylphenyl)sulfonamido)phenyl)thiazol-2-yl)hexanamide*
- *2-(4-methylpiperazin-1-yl)-N-(4-(4-(4-methylpiperazin-1-yl)-3-((4-pentylphenyl)sulfonamido)phenyl)thiazol-2-yl)acetamide*
- *N-(3-(2-((2-(4-methylpiperazin-1-yl)ethyl)amino)thiazol-4-yl)phenyl)-4-pentylbenzenesulfonamide*
- *N-(2-(4-methylpiperazin-1-yl)ethyl)-N-(4-(3-(N-((4-pentylphenyl)sulfonyl)acetamido)phenyl)thiazol-2-yl)acetamide*
- *N-(4-(3-((5-(dimethylamino)naphthalene)-1-sulfonamido)-4-(prop-2-yn-1-yloxy)phenyl)thiazol-2-yl)acrylamide*

In an embodiment, the patient suffers from a solid cancer or a liquid cancer.

In an embodiment, the patient suffers from a liquid cancer such as lymphoma, leukemia and hematopoietic cancer.

In an embodiment, the patient suffers from a solid cancer selected from the group consisting of skin cancer (e.g. melanoma, nonmelanoma skin cancer), gastrointestinal carcinoid tumors (e.g. choriocarcinoma, chorioadenoma destruens), prostate cancer, colorectal cancer, breast cancer (e.g. ductal carcinoma in situ, infiltrating ductal carcinoma, infiltrating lobular carcinoma, lobular carcinoma in situ), Kidney cancer, Autonomic ganglia cancer, Oeasophagus cancer, Stomach and Gastric cancer, Endometrium cancer, Upper Aerodigestive cancer, Ovarian cancer, Large Intestine cancer, Liver cancer, Central Neural System cancer, Pancreas cancer, Lung cancer, Urinary Tract cancer, Soft Tissue cancer, Biliary tract cancer, Thyroid cancer, Pleura cancer (incl. mesothelioma cancer), Bone cancer, Salivary cancer

Inventors demonstrate that expression of HSPA5 in melanoma cell lines corelates with sensitivity to HA15. Without wishing to be bound by any theory, the inventors believe that compounds efficacy is correlated to HSPA5 expression in cancer tissue.

Multiple type of cancer has a HSPA5 expression level which is above the expression level in A375 cells lines (as exemplified in example 9). Since compounds efficacy is correlated to HSPA5 expression in cancer tissue, the applicability and efficacy of the compounds according to the invention can reasonably be extrapolate to cancerous cell lines with high HSPA5 expression level such as Autonomic ganglia cancer, Oeasophagus cancer, Stomach/Gastric cancer, Endometrium cancer, Upper Aerodigestive cancer, Ovarian cancer, Large Intestine cancer, Liver cancer, Central Neural System cancer, Pancreas cancer, Lung cancer, Urinary Tract cancer, Soft Tissue cancer, Biliary tract cancer, Thyroid cancer, Pleura cancer (incl. mesothelioma cancer), Bone cancer, Salivary cancer.

Hence, in an embodiment, the cancer is a cancer over-expressing HSPA5.

In an embodiment, the patient suffers from a solid cancer selected from the group consisting of skin cancer (e.g. melanoma, nonmelanoma skin cancer), esophagus cancer, gastric cancer, gastrointestinal carcinoid tumors (e.g. choriocarcinoma, chorioadenoma destruens), stomach cancer, prostate cancer, colon cancer and breast cancer (e.g. ductal carcinoma in situ, infiltrating ductal carcinoma, infiltrating lobular carcinoma, lobular carcinoma in situ).

According to a preferred embodiment, the cancer treated according to the present invention is a stomach and gastric cancer, an esophagus cancer or a skin cancer, preferably cutaneous melanoma.

In an embodiment, benzene sulfonamide thiazole compounds for use for the treatment of skin cancer, preferably melanoma, are selected among:
- *N-(4-(3-((5-(dimethylamino)naphthalene)-1-sulfonamido)-4-methoxypheny)thiazol-2-yl)acetamide*
- *N-(4-(3-((5-(dimethylamino)naphthalene)-1-sulfonamido)-4-fluorophenyl)thiazol-2-yl)-2-(4-(2-hydroxyethyl)piperazin-1-yl)acetamide*
- *N-(4-(3-((5-(dimethylamino)naphthalene)-1-sulfonamido)-4-methoxyphenyl)thiazol-2-yl)-2-(4-(2-hydroxyethyl)piperazin-1-yl)acetamide*
- *N-(4-(3-((5-(dimethylamino)naphthalene)-1-sulfonamido)-4-(2-methoxyethoxy)phenyl)thiazol-2-yl)acetamide*
- *N-(4-(3-((5-(dimethylamino)naphthalene)-1-sulfonamido)-4-(2-methoxyethoxy)phenyl)thiazol-2-yl)-2-( 4-(2-hydroxyethyl)piperazin-1-yl)acetamide*
- *2-(2,3-dihydroxypropoxy)-N-(4-(3-((5-(dimethylamino)naphthalene)-1-sulfonamido)phenyl)thiazol-2- yl)acetamide*
- *Tert-butyl(6-((4-(3-((5-(dimethylamino)naphthalene)-1-sulfonamido)phenyl)thiazol-2-yl)amino)-6-oxohexyl)carbamate.*
- *Ethyl 5-((4-(3-((5-(dimethylamino)naphthalene)-1-sulfonamido)phenyl)thiazol-2-yl)amino)-5-oxopentanoate*
- *6-acetamido-N-(4-(3-((5-(dimethylamino)naphthalene)-1-sulfonamido)phenyl)thiazol-2-yl)hexanamide*
- *Tert-butyl (4-((4-(3-((5-(dimethylamino)naphthalene)-1-sulfonamido)phenyl)thiazol-2-yl)amino)-4-oxobutyl)carbamate*
- *4-((4-(3-((5-(dimethylamino(naptalene)-1-sulfonamido)phenyl)thiazol-2-yl)butanamide*
- *5-(dimethylamino)-N-(3-(2-methylthiazol-4-yl)phenyl)naphthalene-1-sulfonamide.*
- *N-(4-(3-((5-(dimethylamino)naphthalene)-1-sulfonamido)phenyl)thiazol-2-yl)-2-(4-methylpiperazin-1-yl)acetamide*
- *N-(4-(3-((5-(dimethylamino)naphthalene)-1-sulfonamido)phenyl)thiazol-2-yl)-2-morpholinoacetamide.*
- *N-(4-(3-((5-(dimethylamino)naphthalene)-1-sulfonamido)phenyl)thiazol-2-yl)-2-(4-(2-hydroxyethyl)piperazin-1-yl)acetamide*
- *1-(2-((4-(3-((5-(dimethylamino)naphthalene)-1-sulfonamido)phenyl)thiazol-2-yl)amino)-2-oxoethyl)piperidine-4-carboxamide*
- *N-(4-(3-((5-(dimethylamino)naphthalene)-1-sulfonamido)phenyl)thiazol-2-yl)-2-((2-hydroxyethyl)amino)acetamide*
- *N-(4-(3-((5-(dimethylamino)naphthalene)-1-sulfonamido)phenyl)thiazol-2-yl)-2-((3-morpholinopropyl)amino)acetamide.*
- *Diethyl-(2-((4-(3-((5-(dimethylamino)naphthalene)-1-sulfonamido)phenyl)thiazol-2-yl)amino)- 2-oxoethyl)phosphoramidate*
- *N-(4-(3-((5-(dimethylamino)naphthalene)-1-sulfonamido)-4-fluorophenyl)thiazol-2-yl)acetamide*
- *2-((2-((4-(3-((5-(dimethylamino)naphthalene)-1-sulfonamido)phenyl)thiazol-2-yl)amino)-2-oxoethyl)amino)ethane-1-sulfonic*

In an yet preferred embodiment, *2-(4-methylpiperazin-1-yl)-N-(4-(3-((4-pentylphenyl)sulfonamido)phenyl)thiazol-2yl)acetamide,2-(4-(2-hydroxyethyl)piperazin-1-yl)-N-(4-(3-((4-pentylphenyl)sulfonamido)phenyl)thiazol-2-yl)acetamide, N-(4-(4-methoxy-3-((4-pentylphenyl)sulfonamido)phenyl)thiazol-2-yl)-2-(4-methylpiperazin-1-yl)acetamide* , *2-(4-(2-hydroxyethyl)piperazin-1-yl)-N-(4-(4-methoxy-3-((4-pentylphenyl)sulfonamido) phenyl)thiazol-2-yl)acetamide, 2-((2-(dimethylamino)ethyl)amino)-N-(4-(3-((4-pentylphenyl)sulfonamido)phenyl)thiazol-2-yl)acetamide, 6-(4-methylpiperazin-1-yl)-N-(4-(3-((4-pentylphenyl)sulfonamido)phenyl)thiazol-2-yl)hexanamide, 6-(4-(2-hydroxyethyl)piperazin-1-yl)-N-(4-(3-((4-pentylphenyl)sulfonamido)phenyl)thiazol-2-yl)hexanamide, 2-(4-methylpiperazin-1-yl)-N-(4-(4-(4-methylpiperazin-1-yl)-3-((4-pentylphenyl)sulfonamido)phenyl)thiazol-2-yl)acetamide, N-(3-(2-((2-(4-methylpiperazin-1-yl)ethyl)amino)thiazol-4-yl)phenyl)-4-pentylbenzenesulfonamid* or *N-(2-(4-methylpiperazin-1-yl)ethyl)-N-(4-(3-(N-((4-pentylphenyl)sulfonyl)acetamido)phenyl)thiazol-2-yl)acetamide,* are used for treating skin cancer and preferably melanoma.

In a yet preferred embodiment, *2-(4-methylpiperazin-1-yl)-N-(4-(3-((4-pentylphenyl)sulfonamido)phenyl)thiazol-2 yl)acetamide, 2-(4-(2-hydroxyethyl)piperazin-1-yl)-N-(4-(3-((4-pentylphenyl)sulfonamido)phenyl)thiazol-2-yl)acetamide, N-(4-(4-methoxy-3-((4-pentylphenyl)sulfonamido)phenyl)thiazol-2-yl)-2-(4-methylpiperazin-1-yl)acetamide, 2-(4-(2-hydroxyethyl)piperazin-1-yl)-N-(4-(4-methoxy-3-((4-pentylphenyl)sulfonamido) phenyl)thiazol-2-yl)acetamide, 2-((2-(dimethylamino)ethyl)amino)-N-(4-(3-((4-pentylphenyl)sulfonamido)phenyl)thiazol-2-yl)acetamide, 6-(4-methylpiperazin-1-yl)-N-(4-(3-((4-pentylphenyl)sulfonamido)phenyl)thiazol-2-yl)hexanamide 6-(4-(2-hydroxyethyl)piperazin-1-yl)-N-(4-(3-((4-pentylphenyl)sulfonamido)phenyl)thiazol-2-yl)hexanamide, N-(3-(2-((2-(4-methylpiperazin-1-yl)ethyl)amino)thiazol-4-yl)phenyl)-4-pentylbenzenesulfonamide* or *N-(2-(4-methylpiperazin-1-yl)ethyl)-N-(4-(3-(N-((4-pentylphenyl)sulfonyl)acetamido)phenyl)thiazol-2-yl)acetamide* are used for treating skin cancer and preferably melanoma.

In another preferred embodiment, *2-(4-methylpiperazin-1-yl)-N-(4-(3-((4-pentylphenyl)sulfonamido)phenyl)thiazol-2 yl)acetamide, 2-(4-(2-hydroxyethyl)piperazin-1-yl)-N-(4-(3-((4-pentylphenyl)sulfonamido)phenyl)thiazol-2-yl)acetamide, N-(4-(4-methoxy-3-((4-pentylphenyl)sulfonamido)phenyl)thiazol-2-yl)-2-(4-methylpiperazin-1-yl)acetamide, 2-(4-(2-hydroxyethyl)piperazin-1-yl)-N-(4-(4-methoxy-3-((4-pentylphenyl)sulfonamido) phenyl)thiazol-2-yl)acetamide, 2-((2-(dimethylamino)ethyl)amino)-N-(4-(3-((4-pentylphenyl)sulfonamido)phenyl)thiazol-2-yl)acetamide, 6-(4-methylpiperazin-1-yl)-N-(4-(3-((4-pentylphenyl)sulfonamido)phenyl)thiazol-2-yl)hexanamide, 6-(4-(2-hydroxyethyl)piperazin-1-yl)-N-(4-(3-((4-pentylphenyl)sulfonamido)phenyl)thiazol-2-yl)hexanamide, 2-(4-methylpiperazin-1-yl)-N-(4-(4-(4-methylpiperazin-1-yl)-3-((4-pentylphenyl)sulfonamido)phenyl)thiazol-2-yl)acetamide, N-(3-(2-((2-(4-methylpiperazin-1-yl)ethyl)amino)thiazol-4-yl)phenyl)-4-pentylbenzenesulfonamide* or *N-(2-(4-methylpiperazin-1-yl)ethyl)-N-(4-(3-(N-((4-pentylphenyl)sulfonyl)acetamido)phenyl)thiazol-2-yl)acetamide* are used for treating stomach and gastric and/or esophagus cancer.

In a yet preferred embodiment, *2-(4-methylpiperazin-1-yl)-N-(4-(3-((4-pentylphenyl)sulfonamido)phenyl)thiazol-2 yl)acetamide, 2-(4-(2-hydroxyethyl)piperazin-1-yl)-N-(4-(3-((4-pentylphenyl)sulfonamido)phenyl)thiazol-2-yl)acetamide, N-(4-(4-methoxy-3-((4-pentylphenyl)sulfonamido)phenyl)thiazol-2-yl)-2-(4-methylpiperazin-1-yl)acetamide, 2-(4-(2-hydroxyethyl)piperazin-1-yl)-N-(4-(4-methoxy-3-((4-pentylphenyl)sulfonamido) phenyl)thiazol-2-yl)acetamide, 2-((2-(dimethylamino)ethyl)amino)-N-(4-(3-((4-pentylphenyl)sulfonamido)phenyl)thiazol-2-yl)acetamide, 6-(4-methylpiperazin-1-yl)-N-(4-(3-((4-pentylphenyl)sulfonamido)phenyl)thiazol-2-yl)hexanamide, 6-(4-(2-hydroxyethyl)piperazin-1-yl)-N-(4-(3-((4-pentylphenyl)sulfonamido)phenyl)thiazol-2-yl)hexanamide, N-(3-(2-((2-(4-methylpiperazin-1-yl)ethyl)amino)thiazol-4-yl)phenyl)-4-pentylbenzenesulfonamide* or *N-(2-(4-methylpiperazin-1*- *yl)ethyl)-N-(4-(3-(N-((4-pentylphenyl)sulfonyl)acetamido)phenyl)thiazol-2-yl)acetamide* are used for treating stomach and gastric and/or esophagus cancer.

The compounds used in the context of the present invention may be administered by any suitable route. The skilled person knows which route of administration to use as well as the corresponding dosages. Compounds useful in the context of the present invention are typically administered via parenteral (e. g. , intravenous, intramuscular or subcutaneous) administration.

The invention will be further illustrated by the following figures and examples. However, these examples and figures should not be interpreted in any way as limiting the scope of the present invention.

### Examples

### Example 1: Stability improvement compared to HA15

The compounds of the present invention display higher chemical stability than HA15. As representative example, compound PB704 shows full stability in the following conditions: PBS (pH 7.4), 20% DMSO, 24h; aqueous HCl (0.1M), 24h; PBS (pH 7.4), 20% DMSO, 24h, 80°C while HA15 shows partial degradation in the latter condition.

### Example 2: Solubility improvement compared to HA15

The compounds of the present invention display higher aqueous solubility than HA15. The following table provides examples of the aqueous solubility of HA15 and some compounds of the present invention.

| **Compound** | **Solublity (µM)** |
|---|---|
| HA15 | 1.2±0.2 |
| PB601 | 5.2±1.9 |
| PB602 | 3.4±0.7 |
| PB604 | 4.1±0.4 |
| PB605 | 2.8±0.5 |
| PB638 | 4.0±2.0 |
| PB640 | 4.2±0.4 |
| PB643 | 3.0±1.0 |
| PB654 | 88±3 |
| PB688 | 17.0±7.0 |
| PB692 | 7.0±5.0 |
| PB696 | 49±16 |
| PB704 | 1.4±0.3 |

### Example 3: exposition improvement compared to HA15

The compounds of the present invention demonstrate augmented exposition in mice than HA15. The following table provides examples of the AUC of some compounds of the present invention compared to HA15.

| **Compound** | **T1/2 IV (min)** (PO administration 1 mpk) |
|---|---|
| HA15 | 66 |
| PB688 | 76 |
| PB689 | 110 |
| PB691 | 77 |
| PB696 | 312 |

### Example 4: half-life improvement compared to HA15

The compounds of the present invention display longer half-lifes in mice than HA15. The following table provides examples of the AUC of some compounds of the present invention compared to HA15.

| **Compound** | **AUC IV (min.ng/mL/mpk)** |
|---|---|
| HA15 | 60529 |
| PB688 | 112348 |
| PB692 | 488474 |
| PB704 | 197707 |

| **Compound** | **AUC PO (min.ng/mL/mpk)** |
|---|---|
| HA15 | 1409 |
| PB688 | 3000 |
| PB691 | 6266 |
| PB692 | 3978 |
| PB704 | 4435 |

### Example 5: The effect of compounds according to the invention on cell viability on A375 melanoma cells.

Cell viability was assessed by measuring the number of cells alive in sample of different cells. The measure of cell viability was performed by cell counting using the trypan blue exclusion method. Results were expressed as the percentage of cells alive relatively to the number of living cells in the presence of DMSO, which corresponds to the negative control associated to the 100% value.

Viability at 48 hours was measured. Different doses were tested for these compounds (10µM ; 5µM and 1µm).

**Table 5.1: Effect of compounds according to the invention on cell viability of A375 melanoma cells in culture expressed percentage of cells alive relative to negative control (100%)**

| **Compound** | **Viability A375 cells 10µM/ 48h** | **Viability A375 cells 5µM/ 48h** | **Viability A375 cells 1µM/ 48h** |
|---|---|---|---|
| PB600 | 48.8±4.3 | / | / |
| PB601 | 10.2±2.7 | 22.5±5.3 | 47.2±6.7 |
| PB602 | 1.0±1.0 | 1.7±0.9 | 47.6±2.4 |
| PB603 | 43.1±8.7 | / | / |
| PB604 | 16.3±9.8 | 19.0±8.6 | 57.7±16.5 |
| PB605 | 6.4±0.6 | 29.4±9.8 | 64.5±4.3 |
| PB624 | 33.3±5.5 | 87.8±16.2 | / |
| PB625 | 36.5±7.5 | 93.0±3.0 | / |
| PB628 | 12.5±6.7 | 25.2±6.1 | / |
| PB631 | 35.4±14.2 | 31.3±4.9 | / |
| PB633 | 43.8±12.7 | 65.2±9.9 | / |
| PB635 | 46.9±4.4 | 47.0±13.5 | / |
| PB638 | 0.0±0.0 | 0.9±1.0 | 37.5±5.9 |
| PB639 | 24.0±0.0 | 63.5±16.0 | 100.0±23.6 |
| PB640 | 0.0±0.0 | 67.0±10.6 | 54.2±5.9 |
| PB641 | 40.0±0.0 | 105.3±18.9 | 104.2±5.9 |
| PB643 | 0.0±0.0 | 29.8±6.5 | 108.3±11.8 |
| PB644 | 8.0±0.0 | 11.7±1.2 | 83.3±23.6 |
| PB649 | 26.4±4.5 | 77.7±19.8 | / |
| PB651 | 25.0±8.1 | 89.4±5.6 | / |
| PB654 | 70.0±13.8 | 89.4±6.5 | / |
| / means not measured | | | |

The compounds according to the invention affect the viability of melanoma cells.

### Example 6: NCI-60 Human Tumor Cell Lines Screen

Developmental Therapeutics Program of the National Cancer Institute, DTP-NCI-60 Human Tumor Cell line screen has been used to screen PB compounds for potential anticancer activity. The operation of this screen utilizes 60 different human tumor cell lines, representing leukemia, melanoma and cancers of the lung, colon, brain, ovary, breast, prostate, and kidney cancers.

The compounds have been evaluated against the 60 cells panel at one dose and then at five concentration levels for the most active compounds.

The One-dose data will be reported as a mean graph of the percent growth of treated cells and will be similar in appearance to mean graphs from the 5-dose assay. The number reported for the One-dose assay is growth relative to the no-drug control, and relative to the time zero number of cells. This allows detection of both growth inhibition (values between 0 and 100) and lethality (values less than 0). This is the same as for the 5-dose assay.

For example, a value of 100 means no growth inhibition. A value of 40 would mean 60% growth inhibition. A value of 0 means no net growth over the course of the experiment. A value of -40 would mean 40% lethality. A value of -100 means all cells are dead.

**Table 6.1 Growth inhibition (positive values) or lethality (negative values) of compounds according to the invention compared to non-drug treated NCI-60 Human Tumor Cell Lines.**

| **Compound** | **Mean growth inhibition value** (data from DTP-NCI60) |
|---|---|
| HA15 | 63.82 |
| PB691 | -8.43 |
| PB692 | 23.99 |
| PB694 | 37.26 |
| PB696 | -14.81 |
| PB704 | 55.54 |
| PB705 | 5.64 |

The compounds according to the invention exhibit stronger efficacy than HA15 against NCI-60 Human Tumor Cell Lines.

### Example 7: Action of the compounds according to the invention on ER stress markers (CHOP)

Starved A375 melanoma cells, Kyse 70, SH10TC and KATO III, were treated during 24 or 48h by indicated compounds. Cells were then lysed, and lysates were analyzed by western blot using CHOP as ER stress marker antibodies.

| LEGEND Induction of CHOP in comparison to control condition | |
|---|---|
| - | No induction of CHOP |
| + | Induction of CHOP |
| ++ | Significant induction of CHOP |
| +++ | Strong induction of CHOP |

**Table 7.1 The results of CHOP induction are produced in the table hereinafter.**

| | **A375 (serum free)** | **Kyse70 (serum free)** | **SH10TC (serum free)** | **Kato III (1 % SVF)** |
|---|---|---|---|---|
| | **24h** | **24h** | | |
| PB688 | +++ | +++ | ++ | + |
| PB689 | + | + | +++ | ++ |
| PB691 | + | ++ | - | + |
| PB692 | + | + | + | + |
| PB693 | ++ | + | - | - |
| PB694 | + | ++ | ++ | + |
| PB695 | + | + | ++ | - |
| PB696 | + | - | ++ | + |
| PB704 | ++ | + | - | + |
| PB705 | ++ | ND | ND | ++ |

The compounds according to the present invention kill cancer cells by targeting ER stress markers CHOP to increase ER stress.

### Example 8: IC₅₀ determination in A375 cells at 24h/48h (10%SVF)

IC₅₀ of the compound according to the present invention were compared to HA15 in A375 melanoma cells: Starved A375 melanoma cells were treated during 24 by indicated compounds. Cells were then counted using blue trypan exclusion method. The results are presented in the table below.

**Table 8.1: IC50 of compounds according to the invention**

| | **IC50 (µM)** |
|---|---|
| | **A375 (serum free)** |
| | **24h** |
| HA15 | 2,5 |
| PB688 | 1 |
| PB689 | 1,3 |
| PB691 | 1,7 |
| PB692 | 1,6 |
| PB693 | 1 |
| PB694 | 0,75 |
| PB695 | 0,6 |
| PB696 | 0,9 |
| PB697 | 2 |
| PB698 | 1,75 |
| PB704 | 1,2 |
| PB705 | 1,3 |
| PB707 | 2,5 |
| PB708 | 2,5 |

These results indicated that compounds according to the present invention have advantageously a better IC50 than HA15, a benzene sulfonamide thiazole compound described in WO2014072486 which is active in the treatment of cancer, especially on melanoma cells.

### Example 9: Toxicity of the compounds according to the present invention in FHN at 24h in comparison to HA15 in normal fibroblast cells

Normal human fibroblasts (FHN) were treated during 24 h by indicated compounds at the concentration of IC50 or 10-fold IC50 determined on A375 cells (exemple 4). Cells were then counted using blue trypan exclusion method. "OK" means that the compound is not toxic.

**Table 9.1: Toxicity of compounds according to the invention on HA15 normal fibroblast cells**

| | **Toxicity assessed at IC50 or 10*IC50** |
|---|---|
| | **FHN (10% SVF)** |
| | **24h** |
| HA15 | OK |
| PB688 | OK |
| PB689 | OK |
| PB691 | OK |
| PB692 | OK |
| PB693 | OK |
| PB694 | OK |
| PB695 | OK |
| PB698 | OK |
| PB704 | OK |
| PB705 | OK |

| | |
|---|---|
| *OK = non-toxic (at IC50 or 10* IC50 concentration determined in example 8)* | |

These results indicate that new HA15-derivatives according to the invention have no toxicity against normal human fibroblasts, which is surprising effect considering the significantly improved anti proliferative effect comparatively to HA15.

### Example 10: correlation between HSPA5 expression level and compounds efficacy

Inventors demonstrate that expression of HSPA5 in melanoma cell lines corelates with sensitivity to HA15. Without wishing to be bound by any theory, the inventors believe that compounds efficacy is correlated to HSPA5 expression in cancer tissue.

As indicated in the table below, multiple type of cancer has a HSPA5 expression level which is above A375. Since compounds efficacy is correlated to HSPA5 expression in cancer tissue, the applicability and efficacy of the compounds according to the invention can reasonably be extrapolate to all indications cited in the table.

**Table 10.1. Average HSPA5 mRNA level in multiple type of cancer according to CCLE Broad Institute Database of Cancer cell lines.**

| Organ/Tissue | Average HSPA5 mRNA level | Number of Cell Lines |
|---|---|---|
| Autonomic ganglia | 9.96 | 1 |
| Oeasophagus | 9.17 | 12 |
| Stomach | 9.06 | 9 |
| Prostate | 9.04 | 4 |
| Endometrium | 8.86 | 13 |
| Upper Aerodigestive | 8.82 | 21 |
| Ovary | 8.82 | 19 |
| Large Intestine | 8.82 | 10 |
| Hematopoietic & Lymphoid | 8.78 | 35 |
| Liver | 8.76 | 6 |
| Central Neural System | 8.72 | 47 |
| Pancreas | 8.71 | 16 |
| Lung | 8.7 | 44 |
| Overall skin | 8.68 | 30 |
| Urinary Tract | 8.65 | 10 |
| Soft Tissue | 8.62 | 12 |
| Breast | 8.6 | 12 |
| Kidney | 8.54 | 10 |
| Biliary tract | 8.48 | 3 |
| Thyroid | 8.47 | 8 |
| Pleura | 8.41 | 4 |
| Bone | 8.4 | 6 |
| Salivary | 8.31 | 1 |
| **A375 Melanoma** | **8.26** | 1 |

Hence, the compounds according to the invention can target HSPA5 to induce specific cancer cell death and be useful in the future treatment of a various spectrum of cancers.

## Claims

1. A benzene sulfonamide thiazole compound of formula (I): 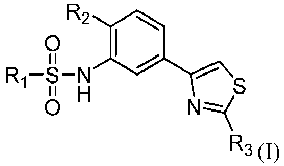 wherein
R₁ is a 5-(dimethylamino)naphthalene group or a 4-pentylphenyl group
R₂ is selected from H, halogen, OR₆
R₃ is selected from C₁-C₈ alkyl, NR₄R_{5;}
R₄ is selected from H, alkyl optionally substituted with a nitrogen-containing heterocycle
R₅ is selected from H, alkyl optionally substituted with a nitrogen-containing heterocycle, COR₇
R₄ and R₅ can alternatively form together a nitrogen-containing heterocycle optionally substituted
R₆ is selected from C₁-C₈ alkyl, C₁-C₈ alkyl-O-C₁-C₈ alkyl,
R₇ is selected from
- C₁-C₈ alkyl optionally substituted with
∘ a nitrogen-containing heterocycle optionally substituted
∘ O-alkyl, NHCO-alkyl, NHCOO-alkyl, COO-alkyl, NH-alkyl, NH-alkyl-OH, NHP(O)(O-alkyl)₂, NH-alkyl-SO₃H, NH-alkyl-N(alkyl)₂
∘ Alkenyl.

2. The benzene sulfonamide thiazole compound according to claim 1, wherein the benzene sulfonamide thiazole compound is a compound of formula (II)

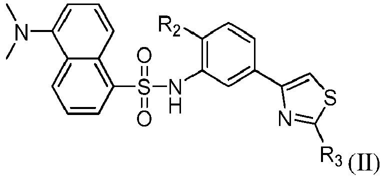

3. The benzene sulfonamide thiazole compound according to claim 1, wherein the benzene sulfonamide thiazole compound is a compound of formula

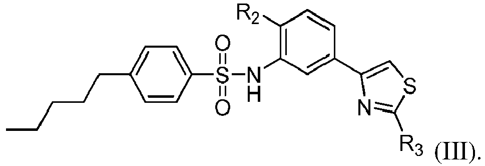

4. The benzene sulfonamide thiazole compound according to anyone of claims 1 to 3, wherein said compound is selected from the group consisting of:
- *N-(4-(3-((5-(dimethylamino)naphthalene)-1-sulfonamido)-4-methoxyphenyl)thiazol-2-yl)acetamide*
- *N-(4-(3-((5-(dimethylamino)naphthalene)-1-sulfonamido)-4-fluorophenyl)thiazol-2-yl)-2-(4-(2-hydroxyethyl)piperazin-1-yl)acetamide*
- *N-(4-(3-((5-(dimethylamino)naphthalene)-1-sulfonamido)-4-methoxyphenyl)thiazol-2-yl)-2-(4-(2-hydroxyethyl)piperazin-1-yl)acetamide*
- *N-(4-(3-((5-(dimethylamino)naphthalene)-1-sulfonamido)-4-(2-methoxyethoxy)phenyl)thiazol-2-yl)acetamide*
- *N-(4-(3-((5-(dimethylamino)naphthalene)-1-sulfonamido)-4-(2-methoxyethoxy)phenyl)thiazol-2-yl)-2-(4-(2-hydroxyethyl)piperazin-1-yl)acetamide*
- *2-(2,3-dihydroxypropoxy)-N-(4-(3-((5-(dimethylamino)naphthalene)-1-sulfonamido)phenyl)thiazol-2-yl)acetamide*
- *Tert-butyl(6-((4-(3-((5-(dimethylamino)naphthalene)-1-sulfonamido)phenyl)thiazol-2-yl)amino)-6-oxohexyl)carbamate*
- *Ethyl 5-((4-(3-((5-(dimethylamino)naphthalene)-1-sulfonamido)phenyl)thiazol-2-yl)amino)-5-oxopentanoate*
- *6-acetamido-N-(4-(3-((5-(dimethylamino)naphthalene)-1-sulfonamido)phenyl)thiazol-2-yl)hexanamide*
- *Tert-butyl (4-((4-(3-((5-(dimethylamino)naphthalene)-1-sulfonamido)phenyl)thiazol-2-yl)amino)-4-oxobutyl)carbamate*
- *4-((4-(3-((5-(dimethylamino(naptalene)-1-sulfonamido)phenyl)thiazol-2-yl)butanamide*
- *5-(dimethylamino)-N-(3-(2-methylthiazol-4-yl)phenyl)naphthalene-1-sulfonamide*
- *N-(4-(3-((5-(dimethylamino)naphthalene)-1-sulfonamido)phenyl)thiazol-2-yl)-2-(4-methylpiperazin-1-yl)acetamide*
- *N-(4-(3-((5-(dimethylamino)naphthalene)-1-sulfonamido)phenyl)thiazol-2-yl)-2-morpholinoacetamide.*
- *N-(4-(3-((5-(dimethylamino)naphthalene)-1-sulfonamido)phenyl)thiazol-2-yl)-2-(4-(2-hydroxyethyl)piperazin-1-yl)acetamide*
- *1-(2-((4-(3-((5-(dimethylamino)naphthalene)-1-sulfonamido)phenyl)thiazol-2-yl)amino)-2-oxoethyl)piperidine-4-carboxamide*
- *N-(4-(3-((5-(dimethylamino)naphthalene)-1-sulfonamido)phenyl)thiazol-2-yl)-2-((2-hydroxyethyl)amino)acetamide*
- *N-(4-(3-((5-(dimethylamino)naphthalene)-1-sulfonamido)phenyl)thiazol-2-yl)-2-((3-morpholinopropyl)amino)acetamide*
- *Diethyl-(2-((4-(3-((5-(dimethylamino)naphthalene)-1-sulfonamido)phenyl)thiazol-2-yl)amino)-2-oxoethyl)phosphoramidate*
- *N-(4-(3-((5-(dimethylamino)naphthalene)-1-sulfonamido)-4-fluorophenyl)thiazol-2-yl)acetamide*
- *2-((2-((4-(3-((5-(dimethylamino)naphthalene)-1-sulfonamido)phenyl)thiazol-2-yl)amino)-2-oxoethyl)amino)ethane-1-sulfonic*
- *2-(4-methylpiperazin-1-yl)-N-(4-(3-((4-pentylphenyl)sulfonamido)phenyl)thiazol-2 yl)acetamide*
- *2-(4-(2-hydroxyethyl)piperazin-1-yl)-N-(4-(3-((4-pentylphenyl)sulfonamido)phenyl)thiazol-2-yl)acetamide*
- *N-(5-(2-aminothiazol-4-yl)-2-methoxyphenyl)-4-pentylbenzenesulfonamide*
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide*
- *2-((2-(dimethylamino)ethyl)amino)-N-(4-(3-((4-pentylphenyl)sulfonamido)phenyl)thiazol-2-yl)acetamide*
- *6-(4-methylpiperazin-1-yl)-N-(4-(3-((4-pentylphenyl)sulfonamido)phenyl)thiazol-2-yl)hexanamide*
- *6-(4-(2-hydroxyethyl)piperazin-1-yl)-N-(4-(3-((4-pentylphenyl)sulfonamido)phenyl)thiazol-2-yl)hexanamide*
- *2-(4-methylpiperazin-1-yl)-N-(4-(4-(4-methylpiperazin-1-yl)-3-((4-pentylphenyl)sulfonamido)phenyl)thiazol-2-yl)acetamide*
- *N-(4-(3-((5-(dimethylamino)naphthalene)-1-sulfonamido)phenyl)thiazol-2-yl)-6-(4-methylpiperazin-1-yl)hexanamide*
- *N-(4-(3-((5-(dimethylamino)naphthalene)-1-sulfonamido)phenyl)thiazol-2-yl)-6-(4-(2-hydroxyethyl)piperazin-1-yl)hexanamide*
- *N-(4-(3-((5-(dimethylamino)naphthalene)-1-sulfonamido)-4-(prop-2-yn-1-yloxy)phenyl)thiazol-2-yl)acrylamide*
- *5-(dimethylamino)-N-(3-(2-((2-(4-methylpiperazin-1-yl)ethyl)amino)thiazol-4-yl)phenyl)naphthalene-1-sulfonamide*
- *N-(3-(2-((2-(4-methylpiperazin-1-yl)ethyl)amino)thiazol-4-yl)phenyl)-4-pentylbenzenesulfonamide*
- *N-(2-(4-methylpiperazin-1-yl)ethyl)-N-(4-(3-(N-((4-pentylphenyl)sulfonyl)acetamido)phenyl)thiazol-2-yl)acetamide*
- *N-(2-(4-methylpiperazin-1-yl)ethyl)-N-(4-(3-(N-((4-pentylphenyl)sulfonyl)acrylamido)phenyl)thiazol-2-yl)acrylamide*
- *N-(3-(2-(4-methylpiperazin-1-yl)thiazol-4-yl)phenyl)-4-pentylbenzenesulfonamide*
- *N-(3-(2-(4-(2-hydroxyethyl)piperazin-1-yl)thiazol-4-yl)phenyl)-4-pentylbenzenesulfonamide*

5. The benzene sulfonamide thiazole compound according to anyone of claims 1 to 4, wherein said compound is selected from the group consisting of:
- *2-(4-methylpiperazin-1-yl)-N-(4-(3-((4-pentylphenyl)sulfonamido)phenyl)thiazol-2 yl)acetamide*
- *2-(4-(2-hydroxyethyl)piperazin-1-yl)-N-(4-(3-((4-pentylphenyl)sulfonamido)phenyl)thiazol-2-yl)acetamide*
- *N-(4-(4-methoxy-3-((4-pentylphenyl)sulfonamido)phenyl)thiazol-2-yl)-2-(4-methylpiperazin-1-yl)acetamide*
- *2-(4-(2-hydroxyethyl)piperazin-1-yl)-N-(4-(4-methoxy-3-((4-pentylphenyl)sulfonamido) phenyl)thiazol-2-yl)acetamide*
- *2-((2-(dimethylamino)ethyl)amino)-N-(4-(3-((4-pentylphenyl)sulfonamido)phenyl)thiazol-2-yl)acetamide*
- *6-(4-methylpiperazin-1-yl)-N-(4-(3-((4-pentylphenyl)sulfonamido)phenyl)thiazol-2-yl)hexanamide*
- *6-(4-(2-hydroxyethyl)piperazin-1-yl)-N-(4-(3-((4-pentylphenyl)sulfonamido)phenyl)thiazol-2-yl)hexanamide*
- *2-(4-methylpiperazin-1-yl)-N-(4-(4-(4-methylpiperazin-1-yl)-3-((4-pentylphenyl)sulfonamido)phenyl)thiazol-2-yl)acetamide*
- *N-(3-(2-((2-(4-methylpiperazin-1-yl)ethyl)amino)thiazol-4-yl)phenyl)-4-pentylbenzenesulfonamide* and
- *N-(2-(4-methylpiperazin-1-yl)ethyl)-N-(4-(3-(N-((4-pentylphenyl)sulfonyl)acetamido)phenyl)thiazol-2-yl)acetamide.*

6. A benzene sulfonamide compound according to any one of claims 1 to 5 for use in the treatment of cancer.

7. The compound for use according to claim 6, wherein said cancer is selected from the group consisting of a solid cancer and liquid cancer.

8. The compound for use according to claim 7, wherein the liquid cancer is lymphoma, leukemia and hematopoietic cancer.

9. The compound for use according to claim 7, wherein the solid cancer is selected from the group consisting of skin cancer, gastrointestinal carcinoid tumors, prostate cancer, colorectal cancer, breast cancer, Kidney cancer, Autonomic ganglia cancer, Oeasophagus cancer, Stomach and Gastric cancer, Endometrium cancer, Upper Aerodigestive cancer, Ovarian cancer, Large Intestine cancer, Liver cancer, Central Neural System cancer, Pancreas cancer, Lung cancer, Urinary Tract cancer, Soft Tissue cancer, Biliary tract cancer, Thyroid cancer, Pleura cancer, Bone cancer, Salivary cancer.

10. The compound for use according to claims 7 or 9, wherein the cancer is selected from the group consisting of stomach and gastric cancer, esophagus cancer and skin cancer.

11. The compound for use according to claims 7, 9 or 10, wherein the cancer is skin cancer, preferably melanoma.

12. The compound for use according to claim 11, wherein the compound is selected from the group consisting of *2-(4-methylpiperazin-1-yl)-N-(4-(3-((4-pentylphenyl)sulfonamido)phenyl)thiazol-2 yl)acetamide, 2-(4-(2-hydroxyethyl)piperazin-1-yl)-N-(4-(3-((4-pentylphenyl)sulfonamido)phenyl)thiazol-2-yl)acetamide, N-(4-(4-methoxy-3-((4-pentylphenyl)sulfonamido)phenyl)thiazol-2-yl)-2-(4-methylpiperazin-1-yl)acetamide 2-(4-(2-hydroxyethyl)piperazin-1-yl)-N-(4-(4-methoxy-3-((4-pentylphenyl)sulfonamido) phenyl)thiazol-2-yl)acetamide, 2-((2-(dimethylamino)ethyl)amino)-N-(4-(3-((4-pentylphenyl)sulfonamido)phenyl)thiazol-2-yl)acetamide, 6-(4-methylpiperazin-1-yl)-N-(4-(3-((4-pentylphenyl)sulfonamido)phenyl)thiazol-2-yl)hexanamide, 6-(4-(2-hydroxyethyl)piperazin-1-yl)-N-(4-(3-((4-pentylphenyl)sulfonamido)phenyl)thiazol-2-yl)hexanamide, 2-(4-methylpiperazin-1-yl)-N-(4-(4-(4-methylpiperazin-1-yl)-3-((4-pentylphenyl)sulfonamido)phenyl)thiazol-2-yl)acetamide, N-(3-(2-((2-(4-methylpiperazin-1-yl)ethyl)amino)thiazol-4-yl)phenyl)-4-pentylbenzenesulfonamide,* and *N-(2-(4-methylpiperazin-1-yl)ethyl)-N-(4-(3-(N-((4-pentylphenyl)sulfonyl)acetamido)phenyl)thiazol-2-yl)acetamide.*

13. The compound for use according to claims 7, 9 or 10, wherein the cancer is stomach and gastric cancer or esophagus cancer.

14. The compound for use according to claim 13, wherein the compound is selected from the group consisting of *22-(4-methylpiperazin-1-yl)-N-(4-(3-((4-pentylphenyl)sulfonamido)phenyl)thiazol-2 yl)acetamide, 2-(4-(2-hydroxyethyl)piperazin-1-yl)-N-(4-(3-((4-pentylphenyl)sulfonamido)phenyl)thiazol-2-yl)acetamide, N-(4-(4-methoxy-3-((4-pentylphenyl)sulfonamido)phenyl)thiazol-2-yl)-2-(4-methylpiperazin-1-yl)acetamide, 2-(4-(2-hydroxyethyl)piperazin-1-yl)-N-(4-(4-methoxy-3-((4-pentylphenyl)sulfonamido) phenyl)thiazol-2-yl)acetamide, 2-((2-(dimethylamino)ethyl)amino)-N-(4-(3-((4-pentylphenyl)sulfonamido)phenyl)thiazol-2-yl)acetamide, 6-(4-methylpiperazin-1-yl)-N-(4-(3-((4-pentylphenyl)sulfonamido)phenyl)thiazol-2-yl)hexanamide, 6-(4-(2-hydroxyethyl)piperazin-1-yl)-N-(4-(3-((4-pentylphenyl)sulfonamido)phenyl)thiazol-2-yl)hexanamide, 2-(4-methylpiperazin-1-yl)-N-(4-(4-(4-methylpiperazin-1-yl)-3-((4-pentylphenyl)sulfonamido)phenyl)thiazol-2-yl)acetamide, N-(3-(2-((2-(4-methylpiperazin-1-yl)ethyl)amino)thiazol-4-yl)phenyl)-4-pentylbenzenesulfonamide,* and *N-(2-(4-methylpiperazin-1-yl)ethyl)-N-(4-(3-(N-((4-pentylphenyl)sulfonyl)acetamido)phenyl)thiazol-2-yl)acetamide.*
